(19) Europäisches Patentamt

European Patent Office

Office européen des brevets

(11) **EP 1 698 356 A1**

(12) **EUROPEAN PATENT APPLICATION**
published in accordance with Art. 158(3) EPC

(43) Date of publication:
06.09.2006 Bulletin 2006/36

(51) Int Cl.:
*A61L 27/00* (2006.01)    *A61K 35/12* (2006.01)

(21) Application number: 04807797.8

(22) Date of filing: 24.12.2004

(86) International application number:
PCT/JP2004/019441

(87) International publication number:
WO 2005/063314 (14.07.2005 Gazette 2005/28)

(84) Designated Contracting States:
AT BE BG CH CY CZ DE DK EE ES FI FR GB GR HU IE IS IT LI LT LU MC NL PL PT RO SE SI SK TR

(30) Priority: 26.12.2003 JP 2003435922

(71) Applicants:
• Cardio, Inc.
Osaka-shi,
Osaka 530-0043 (JP)
• National Institute of Advanced Industrial Science and Technology
Tokyo 100-8921 (JP)

(72) Inventors:
• MATSUDA, H.,
Osaka Univ. Graduate School of Med.
Suita-shi, Osaka 5650871 (JP)
• SAWA, Yoshiki,
Osaka Univ. Graduate School of Med.
Suita-shi, Osaka 5650871 (JP)
• TAKETANI, S.,
Osaka Univ. Graduate School of Med.
Suita-shi, Osaka 5650871 (JP)

• MIYAGAWA, S.,
Osaka Univ. Graduate School of Med.
Suita-shi, Osaka 5650871 (JP)
• IWAI, S.,
Osaka Univ. Graduate School of Med.
Suita-shi, Osaka 5650871 (JP)
• OTA, Takeyoshi,
Osaka Univ. Graduate School of Med
Suita-shi, Osaka 5650871 (JP)
• MIYAKE, Jun,
AIST Kansai, Amagasaki Site
Amagasaki-shi, Hyogo 6610974 (JP)
• HARA, Masayuki,
AIST Kansai, Amagasaki Site
Amagasaki-shi, Hyogo 6610974 (JP)
• FURUTA, Masakazu,
AIST Kansai, Amagasaki Site
Amagasaki-shi, Hyogo 6610974 (JP)
• UCHIMURA, Eiichiro,
AIST Kansai, Amagasaki Site
Amagasaki-shi, Hyogo 610974 (JP)

(74) Representative: Khoo, Chong-Yee
D Young & Co
120 Holborn
London EC1N 2DY (GB)

(54) **DECELLULARIZED TISSUE AND METHOD OF PREPARING THE SAME**

(57) Decellularization of tissue by means of an amphipathic solvent a well-established practice. However, situations exist where the provision of enhanced decellularization is preferred. There is a demand for treating methods for coping with such situations. Thus, it is intended to provide a method for enhancing decellularization. The method comprises not only the immersing of a tissue in a solution containing an amphiphilic molecule in non-micellar form (for example, 1,2-epoxide polymer) but also performing a radical reaction (for example, treatment selected from the group consisting of exposure to gamma-ray irradiation, ultraviolet irradiation, a free radical supply source, ultrasonication, electron beam irradiation, and X-ray irradiation).

EP 1 698 356 A1

**Description**

TECHNICAL FIELD

The present invention relates to a method and system for decellularizing tissue, tissue prepared by the decellularization method, and a pharmaceutical and therapeutic method utilizing a tissue graft or the like.

BACKGROUND ART

Implantation of organs (e.g., heart, blood vessel, etc.) derived from exogenous tissue is mainly hindered by immunologicalrejections. Changes occurring in allografts and xenografts were first described at least 90 years ago(Carrel A., 1907, J. Exp. Med. 9:226-8; Carrel A. , 1912., J. Exp. Med. 9:389-92; Guthrie C.C., 1908, J. Am. Med. Assoc; Calne R. Y., 1970, Transplant Proc. 2:550; and Auchincloss 1988, Transplantation 46:1). Rejection to artery grafts pathologically leads either to enlargement (up to rupture) or obstruction of the grafts. The former is caused by decomposition of extracellular matrices, while the latter is caused by the proliferation of cells in a blood vessel (Uretsky B. F., Mulari S., Reddy S., et al., 1987, Circulation 76:827-34).

Conventionally, two strategies have been used to alleviate rejection of these substances . One of the two strategies is to reduce the immune reaction of hosts (Schmitz-Rixen T., Megerman J., Colvin R. B., Williams A. M., Abbot W., 1988, J. Vasc. Surg. 7:82-92; and Plissonnier D., et al., 1993, Arteriosclerosis Thromb, 13:112-9). The other is to reduce the antigenicity of allografts or xenografts mainly by cross-linking (Rosenberg N., et al., 1956, Surg. Forum 6: 242-6; and Dumont C., Pissonnier D., Michel J. B., 1993, J. Surg. Res. 54:61-69). The cross-linking of extracellular matrices reduces the antigenicity of grafts, but changes bioengineering functions (Cosgrove D. M., Lytle B. W., Golding C. C., et al., 1983, J. Thorac. Cardiovasc. Surgery 64:172-176; and Broom N., Christie G. W. , 1982, In: Cohn L. H. , Gallucci V., editors. Cardiac bioprostheses: Proceedings of the Second International Symposium. New York: York Medical Books Pages 476-491), so that the grafts become susceptible to mineralization (Schoen F. J., Levy R. J., Piehler H. R., 1992, Cardiovasc. Pathology 1992; 1:29-52).

Cells in extracellular matrices have Class I and II histocompatibility antigens capable of eliciting rejection reactions. Also, the cells have glycoproteins recognized by the immune system of hosts, which elicit rejection reactions. Therefore, if these substances are eliminated from extracellular matrices, rejection reactions can be prevented. However, complete elimination of all antigens is considerably difficult to perform and verify. Malone et al. (Malone J. M., Brendel K. , Duhamel R. C. , Reinert R. L. , 1984, J. Vasc. Surg. 1:181-91) and Lalka et al. (Lalka S. G., Oelker L. M., and Malone J. M., et al., 1989, Ann Vasc. Surg., 3:108-17) reported that although immune reactions were stimulated in matrices to which "cell-free" artery allografts (graft to the same species animal) were implanted, proliferation in blood vessels and acceptance of endothelial cells were also observed. Most recently, O'Brian et al. reported that decellularized porcine tissue can be applied to implantation of cardiac blood vessels and that no hyperacute rejection was elicited when implanted to sheep (O'Brien M. F., et al., 1999 (October), Seminars in Thorac. and Cardiovasc. Surg.; 111(4), Suppl 1: 194-200).

Cardiovascular disease, including coronary artery and peripheral vascular disease, can be treated by surgical replacement. The number of cardiovascular disease cases has been recently increasing all over the world. In the case of small-diameter blood vessels, it is difficult to apply replacement therapy. In the case of small-diameter blood vessels, bypass operations are applied, using an autologous venous or arterial graft (Canver C. C., 1995, Chest 1995:108 1150-1155; Barner H. B., 1998, Ann. Thorac. Surg., 66 (Suppl 5) S2-5, discussion S25-28; and Bhan A., Gupta V., Choudhary S. K., et al., 1999, Ann Thorac. Surg., 1999:67 1631-1636). Although venous and arterial grafts currently yield the best results, disadvantages include the need for complicated operations and no suitable blood vessels available in patients with certain diseases. As a result, there is a demand for a vascular prosthesis which is suited to replace small-diameter blood vessels. In order to reduce the use of auto- or allo-grafts, efforts have been made to develop artificial materials. However, no artificial material is suitable for the construction of small-diameter arteries (<6 mm) required for extremity and coronary artery bypass grafting operations. On the other hand, in the case of cardiac blood vessels, the feasibility of native tissue grafts as biomaterials in clinical applications have been investigated. The use of xenograft and allograft tissues typically requires chemical or physical treatment (e.g., glutaraldehyde fixation). Cross-linking techniques have been investigated and found to be the ideal procedure for stabilizing the collagen-based structure of tissue (Hilbert S. L. , Ferrans V. J., Jone M. , 1988, Med Prog. Technol. 89:14, 115-163).

However, implantation has the problem of calcification in the long term, and this detrimental side effect in glutaraldehyde treatment is the main cause of failure of bioprosthetic heart valves (Rao K.P., Shanthi C., 1999, Biomaterials Appl., 13:238-268; and Grabenwoger M., Sider J., Fitzal F., et al., 1996, Ann. Thorac. Surg., 62:772-777). As an alternative approach to native tissue grafts, an attempt has been made to produce an acellular tissue matrix by specifically removing cellular components which were believed to promote calcification and elicit immune responses. The decellularization technique includes chemical, enzymatic, and mechanical means for removing cellular components, leaving a material composed essentially of extracellular matrix components. The resultant decellularized tissue retains native mechanical properties and promotes regeneration. Regeneration is caused by neovascularization and recellularization by the host. Surfactant treatment, which is a typical cell extraction method, has been carried out as a means for creating completely decellularized tissue for use as a biomaterial graft. This is because cellular components, lipids, and residual surfactant remaining within treated tissue may promote undesired effects, such as calcification (Valente M., Bortolotti U., Thiene G., 1985, Am. J. Pathol. 119, 12-21; Maranto A. R. , Shoen F. J., 1988, ASAIO Trans. 34, 827-830; Courtman D. W., Pereira C. A., Kashef V., McComb D., Lee J. M., WilsonG. L. , 1994, J. Biomed. Mater. Res. 28:655-666; and Levy R. J., Schoen F. J., Anderson H. C., et ai. , 1991, Biomaterials 12:'707-714). Removal of lipids from bovine pericardium treated by either chloroform/methanol or sodium dodecyl sulfate (SDS) decreased calcification of tissue in a rat model (Jorge-Herrero E., Fernandez P., Gutierrez M. P., Castillo-Olivares J.L., 1991, Biomaterials 12:683-689). Recently, Sunjay et al. showed that decellularized blood vessel grafts were coated with endothelial progenitor cells (EPCs). This is because these grafts have well preserved extracellular matrices and mechanical properties similar to those of native blood vessels including arteries (Sunjay Kaushal, Gilad E. Amiel, Kristine J., Guleserian, et al., 2001, Nature Medicine Vol. 9, 1035-1040). Sunjay et al. isolated endothelial progenitor cells (EPCs) from peripheral blood and disseminated EPCs in decellularized bovine ileac blood vessels. The EPC-disseminated decellularized graft developed new blood vessels *in vivo* by day 130. The new blood vessels underwent NO-mediated vascular relaxation.

The present inventors have discovered that use of amphipathic solution unexpectedly enhances decellularization efficiency, and in addition, biocompatibility and biological estabilishment property thereof are also good. However, there are some applications in which further decellularization is desired. Thus, further enhancement of decellularization is demanded in the art.

REFERENCES

[patent literature 1]
Japanese Laid-Open Publication No. 2002-543950
[patent literature 2]
Japanese Laid-Open Publication No. 2001-78'750
[patent literature 3]
WO89/05371
[non-patent literature 1]
Carrel A.,1907,J Exp Med 9:226-8
[non-patent literature 2]
Carrel A.,1912.,J Exp Med 9:389-92
[non-patent literature 3]
Toshiharu SHIN'OKA, Yasuharu IMAI, Kazuhiro SEO et al.,: Tissue engineering ni yoru shinkekkan zairyo no kaihatu, oyo [Development and application of cardiovascular material by means of tissue engineering] Japan Journal of Vascular Surgery 2000;29:38
[non-patent literature 4]
Calne RY.,1970,Transplant proc 2:550
[non-patent literature 5]
Auchincloss 1988,Transplantation 46:1
[non-patent literature 6]
Uretsky BF,Mulari S,Reddy S,et a1.,1987,Circulation 76:827-34
[non-patent literature 7]
Schmitz-Rixen T,Megerman J,Colvin RB,Williams AM,Abbot W.,1988,J Vasc Surg 7:82-92
[non-patent literature 8]
Plissonnier D, et al. ,1993, Arteriosclerosis Thromb 13:112-9
[non-patent literature 9]
Rosenberg N,et al.,1956,Surg Forum 6:242-6
[non-patent literature 10]
Dumont C,Pissonnier D,Michel JB.,1993,J Surg Res 54:61-69
[non-patent literature 11]
Cosgrove DM,Lytle BW,Golding CC,et al.,1983,J Thorac Cardiovasc Surgery 64:172-176
[non-patent literature 12]
Broom N, Christie GW.,1982,In:Cohn LH,Gallucci V,editors.Cardiac bioprostheses:Proceedings of the Second International Symposium. New York:York Medical Books Pages 476-491

[non-patent literature 13]

Schoen FJ,Levy RJ,Piehler HR.,Cardiovasc Pathology 1992;1:29-52

[non-patent literature 14]

J Thorac Cardiovasc Surg 1998;115;536-46

[non-patent literature 15]

Malone JM, Brendel K, Duhamel RC,Reinert RL., 1984, J Vasc Surg 1:181-91

[non-patent literature 16]

Lalka SG, Oelker LM, and Malone JM, et al.,1989,Ann Vasc Surg 3:108-17

[non-patent literature 17]

O'Brien MF,et al., 1999(October), Seminars in Thorac and Cardiovasc Surg;111(4),Suppl 1:194-200

[non-patent literature 18]

Canver CC.,1995,Chest 1995;108 1150-1155

[non-patent literature 19]

Earner HB.,1998,Ann Thorac Surg 66 (Suppl 5)S2-5;discussion S25-28

[non-patent literature 20]

Bhan A,Gupta V,Choudhary SK,eta1.,1999,Ann Thorac Surg 1999;67 1631-1636)

[non-patent literature 21]

Hilbert SL,Ferrans VJ,Jone M.,1988,Med Prog Technol 89;14,115-163

[non-patent literature 22]

Rao KP,Shanthi C.,1999,Biomatrials Appl 13:238-268

[non-patent literature 23]

Grabenwoger M,Sider J,Fitzal F,et al. ,1996,Ann Thorac Surg 62;772-777

[non-patent literature 24]

Valente M,Bortolotti U,Thiene G, ,1985,Am J Pathol 119,12-21

[non-patent literature 25]

Maranto AR,Shoen FJ,1988,ASAIO Trans 34,827-830

[non-patent literature 26]

Courtman DW,Pereira CA,Kashef V,McComb D,Lee JM,Wilson GL,1994,J Biomed Mater Res 28:655-666

[non-patent literature 27]

Levy RJ,Schoen FJ,Anderson HC,et al.,1991,Biomaterials 12:707-714

[non-patent literature 28]

Jorge-Herrero E , Fernandez P, Gutierrez MP, Castillo-Olivares JL,1991,Biomaterials 12:683-689

[non-patent literature 29]

Sunjay Kaushal, Gilad E.Amiel,Kristine J.Guleserian,et al.2001,Nature Medicine Vol.9,1035-1040

SUMMARY OF INVENTION

Problems to be solved by the invention:

Decellularization of tissue by amphipathic solvents are known in the art . However, there are certain circumstances where more decellularized tissues should preferably be provided, and thus there is a demand for a method for processing a tissue in order to meet such a requirement. As such, it is an objective of the present invention to provide a method for progressing decellularization and further reducing immunological rejection reactions.

Means for solving the problems:

(Summary of invention)

The present invention by the present inventors, solves the above-mentioned problems in discovering that radical reactions (in particular, treatment or exposure to a radical such as gamma-ray irradiation) in decellularization technology unexpectedly promotes decellularization. Accordingly, the present invention provides a novel method for attaining a decellularization ratio which has not been achieved by conventional treatment by only amphipathic substance.

The above-mentioned problems have been solved by immersing a provided tissue in a solution comprising an amphipatic molecule (for example, exposure treatment to a radical such as gamma-ray irradiation and the like). Accordingly, the present invention is directed to a novel method for decellularizing a tissue for use as a scaffold for cardiovascular blood vessels, biological/artificial organ constructs, ex vivo or in vivo use, novel tissue or tissue grafts, or methods using the same. The present decellularized tissue may be used for a variety of purposes with or without a cell (either autologous, allogenic or xenogenic cells).

The present inventors have established a strategy for applying a radical reaction to a method for decellularization using an amphipathic solvent. Firstly, cytosol and cell membrane are extracted using amphipathic molecules (e.g., 1,2-epoxide polymers such as polyethylene glycol (PEG)) which are not in the micelle form. 1,2-epoxide polymers such as polyethylene glycol (PEG) have the effect of destabilizing the cell membrane which separates the inside of a cell from the outside, so that most cell membrane components and cytosol (water soluble components) are removed in the first step. In combination with this first step, radical reactions (for example, gamma-ray irradiation, ultraviolet irradiation, exposure to a free radical source, exposure to ultrasonication, electron beam irradiation, and x-ray irradiation) and the like are conducted. Although not wishing to be bound by any theory, it is believed that the radical reaction allows progression of cell damaging action by means of radicals by the amphipathic solvent which acts as a scavenger therefor. In addition, nucleic acid components are enzymatically decomposed and deposited from the extracts .

Therefore, the present invention provides the following:

(1) A decellularized tissue which has been subjected to a radical reaction.

(2) A decellularized tissue according to Item 1, characterized in that said decellularized tissue is substantially free of solubilized protein.

(3) A decellularized tissue according to Item 1, characterized in that a extracellular matrix component is at least partially crosslinked by means of covalent bonding.

(4) A decellularized tissue according to Item 3, wherein the extracellular matrix component is selected from the group consisting of collagen, elastin, laminin, fibronectin, glycosaminoglycan, and proteoglycan.

(5) A decellularized tissue according to Item 3, wherein the crosslinking is formed between two or more amino acids having no free amino group or carboxyl group at the side chain thereof.

(6) A decellularized tissue according to Item 3, wherein the crosslinking is formed on groups other than carboxyl, hydroxyl, and amino groups.

(7) A decellularized tissue according to Item 6, wherein the group comprises a group selected from the group consisting of sulfhydryl group, aldehyde group, carbonyl group, sulfo group and nitro group, and the crosslinking is formed by means of a bonding selected from the group consisting of carbon-carbon bonding, ether bonding and ester bonding.

(8) A decellularized tissue according to Item 1, wherein

A) a cell residual rate of the tissue is less than a level at which an immune reaction is elicited in an organism; and
B) the tissue is not damaged to such an extent that hinders the tissue from exhibiting a function which was possessed by the tissue when the tissue was not decellularized.

(9) A decellularized tissue according to Item 1, wherein the radical reaction comprises exposure to a free radical.

(10) A decellularized tissue according to Item 1, wherein the tissue strength thereof is substantially the same as that of a decellularized tissue without exposure to a radical reaction, whereas the decellularization thereof is significantly progressed.

(11) A decellularized tissue according to Item 1, wherein the radical reaction comprises a treatment selected from the group consisting of gamma-ray irradiation, ultraviolet irradiation, exposure to a free radical source, exposure to ultrasonication, electron beam irradiation, and x-ray irradiation.

(12) A decellularized tissue according to Item 1, wherein the radical reaction is gamma-ray irradiation, and the dose of the gamma-ray irradiation is between 10 and 250 kGy.

(13) A decellularized tissue according to Item 1, wherein the radical reaction is gamma-ray irradiation conducted under an atmosphere selected from the group consisting of in vacuo, in oxygen, in nitrogen, in the atmosphere, in water, in an amphipathic molecule solution and a combination thereof.

(14) A decellularized tissue according to Item 1, wherein the decellularized tissue is subjected to DNase treatment.

(15) Decellularized tissue according to Item 1, wherein the tissue is treated in a solution containing a non-micellar amphipathic molecule.

(16) Decellularized tissue according to Item 15, wherein the solution containing the non-micellar amphipathic molecule comprises a 1,2-epoxide polymer.

(17) Decellularized tissue according to Item 15, wherein the solution containing the non-micellar amphipathic molecule comprises polyethylene glycol (PEG).

(18) Decellularized tissue according to Item 17, wherein an average molecular weight of the PEG is between 200 to 6000.

(19) Decellularized tissue according to Item 17, wherein an average molecular weight of the PEG is about 1000.

(20) Decellularized tissue according to Item 1, wherein the cell residual rate of the tissue is about 5 % or less.

(21) Decellularized tissue according to Item 1, wherein the cell residual rate of the tissue is about 4 % or less.

(22) Decellularized tissue according to Item 1, wherein the cell residual rate of the tissue is about 1 % or less.

(23) Decellularized tissue according to Item 1, wherein the tissue has substantially no cells remaining.

(24) Decellularized tissue according to item 1, wherein the tissue damage rate of the tissue is about 30% or less.

(25) Decellularized tissue according to item 1, wherein the tissue damage rate of the tissue is about 15% or less.

(26) Decellularized tissue according to item 1, wherein the tissue damage rate of the tissue is about 5% or less.

(27) Decellularized tissue according to item 1, wherein the tissue has a tissue strength which permits a clinical application.

(28) Decellularized tissue according to item 1, wherein the tissue has a tissue strength which is 80% or more of the tissue strength which was possessed by the tissue when the tissue was not decellularized.

(29) Decellularized tissue according to item 1 , wherein the tissue has a tissue strength having a β value which is 80% or more of the β value which was possessed by the tissue when the tissue was not decellularized.

(30) Decellularized tissue according to item 1, wherein the tissue has a tissue strength having a β value of 20 or more .

(31) Decellularized tissue according to item 1, wherein the tissue is luminal tissue.

(32) Decellularized tissue according to item 1, wherein the tissue is tissue selected from blood vessels, blood vessel-like tissue, cardiac valves, pericardia, dura mater, corneas, and bones.

(33) Decellularized tissue according to item 1, wherein a survival rate of naive extracellular matrix in the decellularized tissue is at least about 50%.

(34) Decellularized tissue according to item 33, wherein the survival rate of the extracellular matrix is substantially a hundred percent.

(35) Decellularized tissue according to item 1, wherein the tissue is derived from a mammal.

(36) Decellularized tissue according to item 1, wherein the tissue is derived from a human.

(37) Decellularized tissue according to item 1, wherein the tissue is derived from a swine.

(38) Decellularized tissue according to item 1, wherein viruses have been substantially removed from the tissue.

(39) Decellularized tissue according to item 38, wherein the viruses are selected from the group consisting of retroviruses and herpes viruses .

(40) Decellularized tissue according to item 38, wherein the viruses comprises porcine endogenous retrovirus (PERV).

(41) Decellularized tissue according to item 40, wherein the PERV is removed such that no significant infection to a human cell is observed.

(42) A tissue graft comprising decellularized tissue according to Item 1.

(43) A tissue graft according to Item 42 further comprising a cell.

(44) A tissue graft according to Item 42, wherein the tissue graft has a form selected from the group consisting of membrane-form, luminal form and valvular form.

(45) A method of producing decellularized tissue, comprising the steps of:

1) providing tissue;
2) immersing the tissue in a solution containing a non-micellar amphipathic molecule; and
3) subjecting the tissue to a radical reaction.

(46) A method according to Item 45, wherein the radical reaction comprises exposure to a free radical.

(47) A method according to Item 45, wherein the tissue strength thereof is substantially the same as that of a decellularized tissue without exposure to a radical reaction, whereas decellularization thereof is significantly progressed.

(48) A method according to Item 45, wherein the radical reaction comprises a treatment selected from the group consisting of gamma-ray irradiation, ultraviolet irradiation, exposure to a free radical source, exposure to ultrasonication, electron beam irradiation, and x-ray irradiation.

(49) A method according to Item 45, wherein the radical reaction is gamma-ray irradiation, and the dose of the gamma-ray irradiation is between 10 and 250 kGy.

(50) A method according to Item 45, wherein the radical reaction is gamma-ray irradiation, and the dose of the gamma-ray irradiation is between 40 and 100 kGy.

(51) A method according to Item 45, wherein the radical reaction is gamma-ray irradiation conducted under an atmosphere selected from the group consisting of in vacuo, in oxyten, in nitrogen, in the atmosphere, in water, in an amphipathic molecule solution and a combination thereof.

(52) A method according to Item 45, wherein the radical reaction is conducted in the atmosphere.

(53) A method according to Item 45, wherein the tissue is treated in a solution containing a non-micellar amphipathic molecule.

(54) A method according to Item 53, wherein the solution containing the non-micellar amphipathic molecule comprises a 1,2-epoxide polymer.

(55) A method according to Item 53, wherein the solution containing the non-micellar amphipathic molecule comprises polyethylene glycol (PEG).

(56) A method according to Item 55, wherein the average molecular weight of the PEG is between about 200 to about 2000.

(57) A method according to Item 55, wherein the average molecular weight of the PEG is between about 1000 to about 2000.

(58) A method according to Item 55, wherein the PEG solution is a solution of about 60% to about 100%.

(59) A method according to Item 45, wherein the tissue is a luminal tissue, a valvular tissue or a membrenous tissue.

(60) A method according to Item 45, wherein the tissue is tissue selected from blood vessels, blood vessel-like tissue, cardiac valves, pericardia, dura mater, corneas, and bones.

(61) A method according to Item 45, wherein the tissue is derived from a mammal.

(62) A method according to Item 45, wherein the tissue is derived from a human or a swine.

(63) A method according to Item 45, wherein the step of immersing is conducted for 30 minutes to ten days.

(64) A method according to Item 45, wherein the step of immersing further comprises a step of physical treatment.

(65) A method according to Item 45, further comprising the step of D) washing the tissue.

(66) A method according to Item 65, wherein the step of washing is conducted for half a day to five days.

(67) A method according to Item 45 , wherein the amphipathic molecule is biocompatible.

(68) A method according to Item 45, wherein the step of immersing comprises a step of agitation.

(69) A method according to Item 45, wherein the step of immersing comprises agitation for one week or longer.

(70) A method according to Item 45, wherein the decellularized tissue is subjected to a DNase treatment.

(71) A method according to item 45, further comprising:

    4) performing chemical treatment.

(72) A method according to item 68, wherein the chemical treatment is performed with DNase.

(73) A method according to item 68, wherein the chemical treatment is performed with DNaseI.

(74) A method according to Item 72, wherein the treatment by the DNase is conducted for 24 hours or longer.

(75) A method according to Item 45, wherein the radical reaction is gamma-ray irradiation and the period of irirradiation of the gamma-ray irradiation is a period to total a dose of about 10-300kGy.

(76) A method according to item 45, further comprising:

    disseminating a cell.

(77) Decellularized tissue obtainable by a method according to Item 45.

(78) Amethod for tissue regeneration, comprising the steps of:

    a) providing decellularized tissue subjected to a radical reaction to an organism; and
    b) incubating the tissue within the organism for a time sufficient for the tissue to regenerate.

(79) A method according to Item 78, wherein the radical reaction comprises exposure to a free radical.

(80) A method according to Item 78 further comprising the step of providing a cell to the decellularized tissue.

(81) A method according to Item 80, wherein the cell is derived from the organism.

(82) A method according to Item 80, wherein the cell is present within the organism.

(83) A method according to Item 80, wherein the cell is derived from a host homologous to the organism.

(84) A method according to Item 80, wherein the cell is derived from a host heterologous to the organism.

(85) A method according to Item 80, wherein the cell is previously isolated from the organism.

(86) A method according to Item 80, the cell is a blood vessel cell or a blood vessel-like cell.

(87) A method according to Item 78, further comprising providing a physiologically active substance capable of inducing cell differentiation to the organism.

(88) A method according to Item 87, wherein the physiologically active substance is derived from or foreign to the organism.

(89) A method according to Item 87, wherein the physiologically active substance is provided in a form of a nucleic acid or a polypeptide.

(90) A method according to Item 87, wherein the physiologically active substance is selected from the group consisting of HGF, VEGF, FGF, IGF, PDGF, and EGF.

(91) A method according to Item 78, wherein the tissue is tissue selected from the group consisting of blood vessels, blood vessel-like tissue, cardiac valves, pericardia, dura mater, corneas, and bones.

(92) A method of producing a tissue graft, comprising the steps of:

A) providing decellularized tissue subjected to a radical reaction to an organism;
B) allowing a self cell in the organism to infiltrate the decellularized tissue; and
C) incubating the tissue within the organism for a time sufficient for the cell to differentiate.

(93) A method according to Item 92, wherein the radical reaction comprises exposure to a free radical.

(94) A method according to Item 92, the cell is a blood vessel cell or a blood vessel-like cell.

(95) A method according to Item 92, wherein the tissue is tissue selected from the group consisting of blood vessels, blood vessel-like tissue, cardiac valves, pericardia, dura mater, corneas, and bones.

(96) A method according to Item 92, wherein the decellularized tissue further comprises a cell.

(97) A method according to Item 92, further comprising the step of D) providing a physiologically active substance capable of inducing differentiation of the cell.

(98) A method according to Item 97, wherein the physiologically active substance is a cytokine having hematopoiesis activity.

(99) A tissue graft, produced by a method according to item 92.

(100) A method for treating a subject requiring implantation of tissue or an organ or treating a subject at a risk of implantation of tissue or an organ for prophylaxis, the method comprising the steps of:

A) providing decellularized tissue subjected to a radical reaction, or a tissue graft comprising the decellularized tissue; and
B) implanting the decellularized tissue or tissue graft to the subject.

(101) A method according to Item 100, wherein the radical reaction comprises exposure to a free radical.

(102) A method according to Item 100, the tissue further comprises a cell.

(103) A method according to Item 100, wherein the tissue has no cells.

(104) A method according to Item 100, wherein the tissue is derived from the subject.

(105) A method according to Item 100, wherein the tissue is tissue selected from blood vessels, blood vessel-like tissue, cardiac valves, pericardia, dura mater, corneas, and bones.

(106) A method according to Item 100, wherein the tissue is derived from a mammal.

(107) A method according to Item 100, wherein the tissue is derived from a human.

(108) A pharmaceutical for organ transplantation comprising:

A) decellularized tissue subjected to a radical reaction, or a tissue graft comprising the decellularized tissue.

(109) A pharmaceutical according to Item 108, wherein the radical reaction comprises exposure to a free radical.

(110) A pharmaceutical according to Item 108, wherein the tissue is tissue selected from blood vessels, blood vessel-like tissue, cardiac valves, pericardia, dura mater, corneas, and bones.

(111) A pharmaceutical according to Item 108, wherein the tissue is derived from a mammal.

(112) A pharmaceutical according to Item 108, wherein the tissue is derived from a human or a swine.

(113) A pharmaceutical according to Item 108, wherein the tissue is derived from a subject in need of the transplantation.

(114) Use of decellularized tissue subjected to a radical reaction, or a tissue graft comprising the decellularized tissue for manufacture of a pharmaceutical for organ transplantation.

(115) Use according to Item 114, wherein the radical reaction comprises exposure to a free radical.

Accordingly, it is understood that these and other advantages of the present invention will be clear to those skilled in the ar uponreading and understanding the following detailed description in view of the drawings attached hereto.

EFFECTS OF THE INVENTION

The present invention is fundamentally different from conventional decellularization methods in that a radical reaction (in particular, exposure to a radical)is used in addition to a chemical treatment. In particular, it should be noted that use of a non-micellar molecule allows unexpected improvement in decellularization efficiency in decellularization technology in a manner similar to the machinery of cellular component extraction.

The decellularized tissue thus prpared are minimized with respect to the damage to extracellular matrices. The decellularized tissue of the present invention may be used as an artificial vessel which can be used in a permanent manner. The decellularized tissue of the present invention may be used as a transplant with or without use of cells, as such, the utility of the decellularized tissue of the present invention ranges to a wide extent. In particular, the efficiency of decellularization is improved to an extent which has not been conventionally achieved (i.e., cell residual rate is less than 5 %, preferably less than 1 %), and thus decellularized tissue substantially free of cells are provided. Therefore, the decellularization tissue of the present invention achieves unexpectedly superior effects in that adverse reactions such as calcification, immonological rejection reactions and the like, can be minimized. The present invention has also achieved the removal of the alpha-Gal epitope, which has been a problem in immunological rejection reaction. Furthermore, the present invention has made possible that infectious viruses such as porcine endogenous retrovirus (PERV), which has been a problem in immunological rejection reaction and infections are removed. Therefore, the present invention has solved a problem which has been problematic in conventional decellularized or artificial tissues.

Decellularized tissue and tissue grafts of the present invention may provide a base for cellular replacement by cells from a host in the tissue or tissue graft after transplantation to the host. Accordingly, decellularized tissue and tissue grafts of the present invention may be used forever. The efficiency of decellularization and the provision of decellularized tissue substantially free of cells has brought significant progress to transplantation medicine, and thus the importance thereof should be of note.

BRIEF DESCRIPTION OF THE DRAWINGS

Figure **1** depicts the test results of decellularization using a variety of PEG (average molecular weight 200, 600, 1000 and 2000).

Figure **2** depicts test results of decellularization with or without agitation.

Figure **3** depicts test results of decellularization in combination with ultrasonication.

Figure **4** depicts test results of decellularization with or without DNase. PEG used has average molecular weight of 2000.

Figure 5 depicts test results of decellularization of an example of control tissue performed in Example 1.

Figure 6 depicts an example of decellularization of the present invention conducted in Example 1.

Figure 7 depicts an example of decellularization of the present invention conducted in Example 1.

Figure 8 depicts an example of decellularization of the present invention conducted in Example 1.

Figure 9 depicts an example of decellularization of the present invention conducted in Example 1.

Figure 10 depicts an example of decellularization of the present invention conducted in Example 1.

Figure 11 depicts an example of decellularization of the present invention conducted in Example 1.

Figure 12 depicts an example of decellularization of the present invention conducted in Example 1.

Figure 13 depicts an example of decellularization of the present invention conducted in Example 1.

Figure 14 depicts an example of decellularization of the present invention conducted in Example 1.

Figure 15 depicts an example of decellularization of the present invention conducted in Example 1.

Figure 16 depicts an example of decellularization of the present invention conducted in Example 1.

Figure 17 depicts an example of decellularization of the present invention conducted in Example 1.

Figure 18 depicts an example of decellularization of the present invention conducted in Example 1.

Figure 19 depicts an example of decellularization of the present invention conducted in Example 1.

Figure 20 depicts an example of decellularization of the present invention conducted in Example 1.

Figure **21** depicts an example of decellularization of the present invention conducted in Example 1.

Figure **22** depicts an example of decellularization of the present invention conducted in Example 1.

Figure 23 depicts an example of decellularization of the present invention conducted in Example 1.

Figure 24 depicts an example of decellularization of the present invention conducted in Example 1.

Figure 25 depicts an example of decellularization of the present invention conducted in Example 1.

Figure **26** depicts an example of decellularization of the present invention conducted in Example 1.

Figure **27** depicts an example of decellularization of the present invention conducted in Example 1.

Figure **28** depicts an example of decellularization of the present invention conducted in Example 1.

Figure **29** depicts an example of decellularization of the present invention conducted in Example 1.

Figure **30A** depicts an example (valve) of decellularization of the present invention conducted in Example 1.

Figure **30B** depicts an example (valve cusp) of decellularization of the present invention conducted in Example 1.

Figure **31** depicts results of tensile test of the PEG valve of the present invention.

Figure **32** depicts results of tensile test of native canine aorta.

Figure **33** depicts results of tensile test of conventional artificial valves by means of decellularization cell methos by SDS, No. 3.

Figure **34** depicts results of subcutaneous transplantation of the decellularized tissue of the present invention.

Figure **35** depicts results (HE staining) of eleven days after the treatment of: treatment method: PEG+gamma ray (100kGy); transplantation target: HBD (HB dog) (20kg) (descending aorta). Cytoplasm has been stained by HE to find no cells, and thus no abnormality of fiber rupture, and therefore no rejection reaction has been recognized.

Figure **36** depicts results (vWF staining) of eleven days after the treatment of: treatment method: PEG+gamma ray (100kGy); transplantation target: HBD (HB dog) (20kg) (descending aorta). vWF antibody staining of vascular endogenous cells allows further recognition of vascular endogenous cells.

Figure **37** depicts results (HE staining) of eight days after the treatment of: treatment method: PEG+gamma ray (100kGy); transplantation target: HBD (HB dog) (20kg) (descending aorta) with respect to another subject.

Figure **38** depicts results (HE staining) of nine days after the treatment of: treatment method: PEG+gamma ray (100kGy); transplantation target: HBD (HB dog) (20kg) (descending aorta) with respect to a different subject.

Figure **39** depicts results (HE staining) of thirty days after the treatment of: treatment method: PEG+gamma ray (100kGy) ; transplantation target: HBD (HB dog) (20kg) (descending aorta) with respect to another subject. Cytoplasm has been stained with HE to find no cells, and thus no abnormality of fiber rupture, and therefore no rejection reaction has been recognized.

Figure **40** depicts results (vWF antibody staining) of thirty days after the treatment of: treatment method: PEG+gamma ray (100kGy); transplantation target: HBD (HB dog) (20kg) (descending aorta) with respect to another different subject. Uniform endogenous cells have been further recognized.

Figure **41** depicts results (reactivity of muscular specific actin antibody) of thirty days after the treatment of: treatment method: PEG+gamma ray (100kGy); transplantation target: HBD (HB dog) (20kg) (descending aorta) with respect to another different subject.

Figure **42** depicts results (vimentin staining) of thirty days after the treatment of: treatment method: PEG+gamma ray (100kGy); transplantation target: HBD (HB dog) (20kg) (descending aorta) with respect to another different subject. The numerals therein indicate the magnitude of amplification.

Figure **43** depicts the shaker used in Examples 8 or the like for rotation.

Figure **44** depicts results of investigation of remaining cells of the decellularized tissue of the present invention used in Example 8. Figure 44 depicts, from the left, the results of native (without treatment), DNase treatment after PEG treatment, an example of PEG and DNase treatments after exposure to gamma-ray irradiation, and an example of gamma-ray irradiation which was conducted after the PEG treatment but prior to the DNase treatment.

Figure **45A** depicts the results of investigation of remaining DNA of the decellularized tissue of the present invention used in Example 8. Figure **45A** depicts, from the left, the results of native (without treatment), DNase treatment after PEG treatment, an example of PEG and DNase treatments after exposure to gamma-ray irradiation, and an example of gamma-ray irradiation which was conducted after the PEG treatment but prior to the DNase treatment.

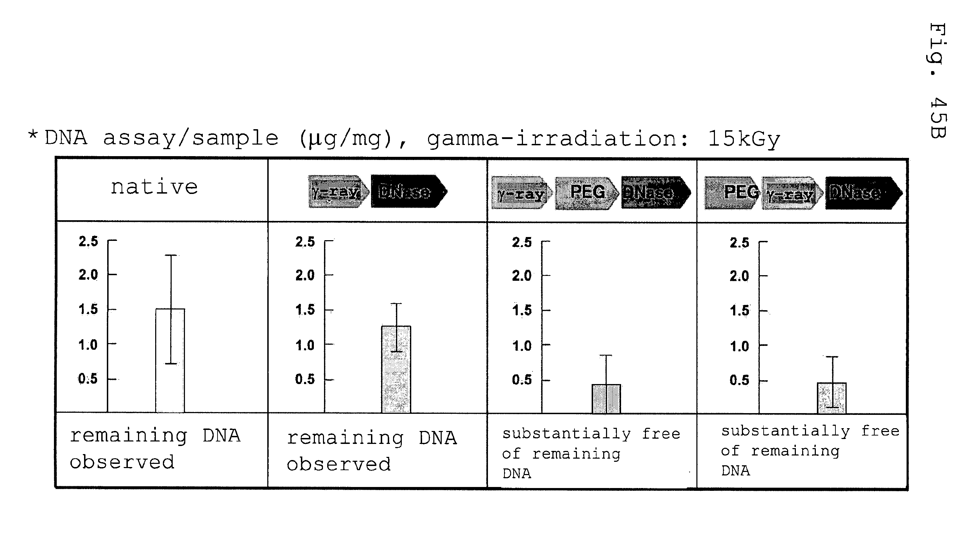

Figure **45B** depicts the results of investigation of remaining DNA of the decellularized tissue of the present invention used in Example 8. Figure **45B** depicts, from the left, the results of native (without treatment), DNase treatment after PEG treatment, an example of PEG and DNase treatments after exposure to gamma-ray irradiation, and an example of gamma-ray irradiation which was conducted after the PEG treatment but prior to the DNase treatment. It was observed that DNA remained in an example of gamma-ray irradiation + DNase treatment, but remaining DNA is significantly reduced in the example of PEG + DNase + gamma-ray treatment.

Figure **46** depicts the results of investigation of remaining protein of the decellularized tissue of the present invention used in Example 8. Figure **46** depicts, from the left, the results of native (without treatment), DNase treatment after PEG treatment, an example of PEG and DNase treatments after exposure to gamma-ray irradiation, and an example of gamma-ray irradiation which was conducted after the PEG treatment but prior to the DNase treatment.

Figure **47** depicts the results of investigation of the decellularized tissue of the present invention with TEM (transmission electron microscope) used in Example 8. Figure **47** depicts, from the left, the results of native (without treatment), DNase treatment after PEG treatment, an example of PEG and DNase treatments after exposure to gamma-ray irradiation, and an example of gamma-ray irradiation which was conducted after the PEG treatment but prior to the DNase treatment. Upper panel shows x3000 magnification, and the lower panel shows x 30,000 magnification.

Figure **48** depicts the results of investigation of the tissue strength of the decellularized tissue of the present invention by measuring tensile strength thereof used in Example 8. Figure **48** depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment.

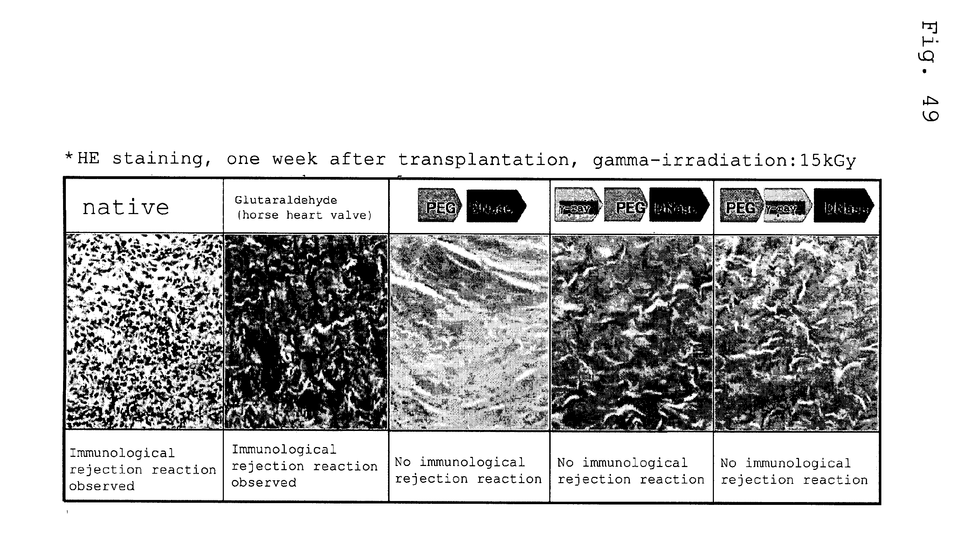

Figure **49** depicts the results of investigation of the immunological rejection reaction of the decellularized tissue of the present invention used in Example 8. Tissue with gamma ray irradiation of 15 kGy was investigated by means of HE staining one week after the transplantation. Figure **49** depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment.

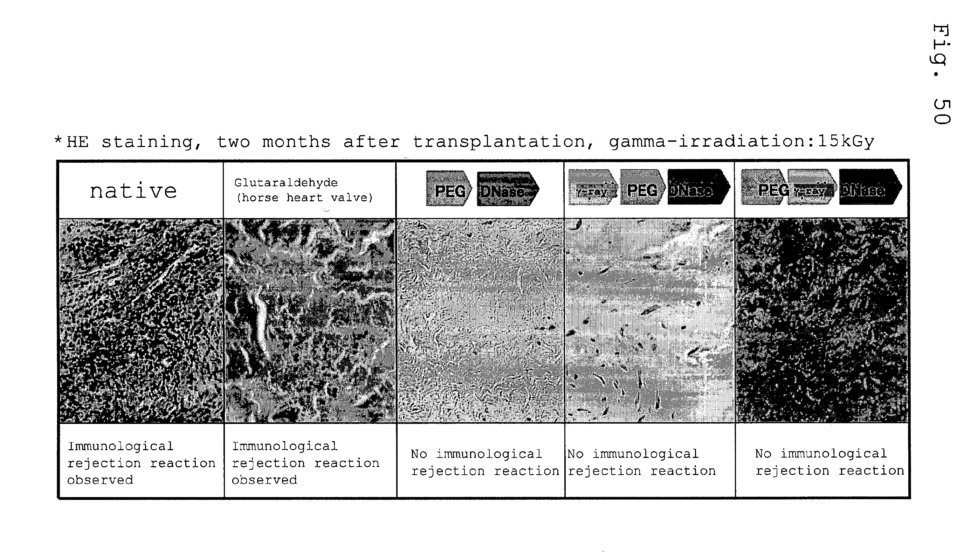

Figure **50** depicts the results of investigation of the immunological rejection reaction for long transplantation of the decellularized tissue of the present invention used in Example 8. Tissue with gamma ray irradiation of 15 kGywas investigated by means of HE staining two months after the transplantation. Figure 50 depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment.

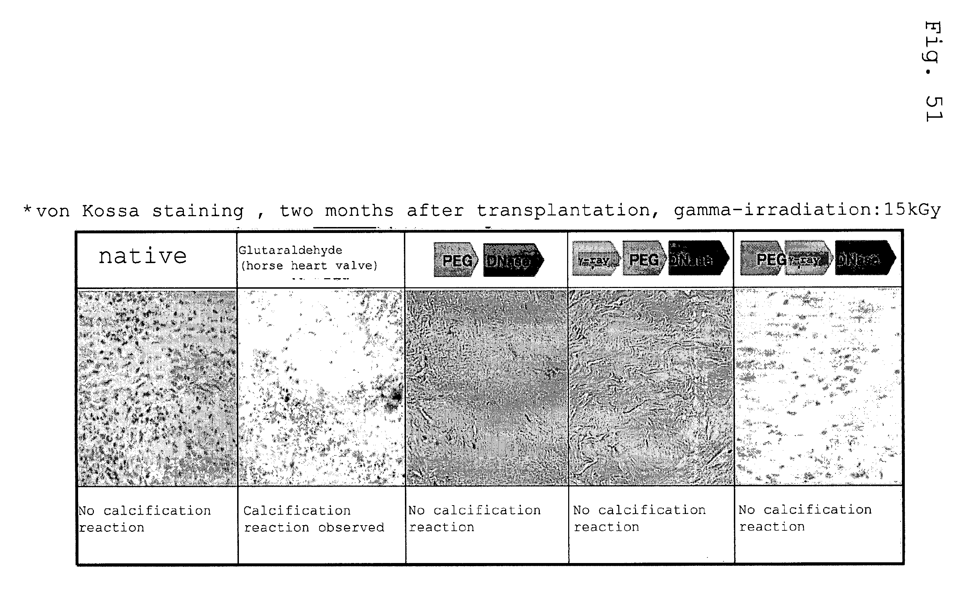

Figure **51** depicts the results of investigation of the calcification of the decellularized tissue of the present invention used in Example 8. Tissue with gamma-ray irradiation of 15 kGy was investigated by means of von Kossa staining two months after the transplantation. Figure **51** depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment.

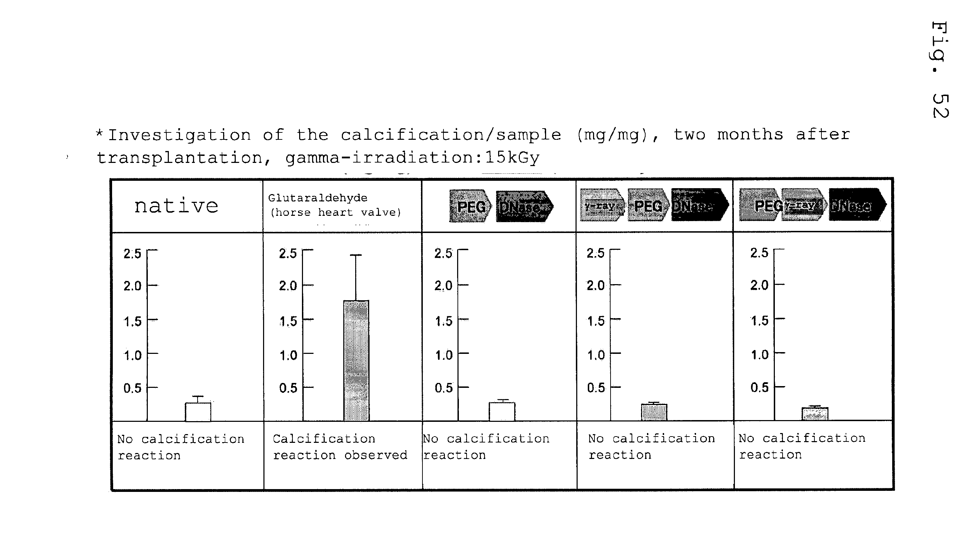

Figure **52** depicts the results of investigation of the calcification of the decellularized tissue of the present invention by means of calcium content measurement used in Example 8. Tissue with gamma ray irradiation of 15 kGy was investigated (calcium/sample <mg/mg>) two months after the transplantation. Figure **52** depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment.

Figure **53** depicts the results of investigation of whether alpha-Gal epitope has been removed by means of gamma-ray irradiation. Gamma-ray irradiation (100kGy) in addition to PEG+DNase treatment is conducted, and alpha-gal epitope has been removed. As a comparative control, an example of macaque is shown. Detection has been conducted by using lectin for GS1B4 (alpha 1,3 Gal) epitope.

Figure **54** depicts the results of investigation whether alpha-Gal epitope has been removed by means of gamma-ray irradiation. Gamma-ray irradiation (25kGy) in addition to PEG+DNase treatment is conducted, alpha-gal epitope has been removed. In the present experiment, observation was conducted by replacing the orde of gamma-ray irradiation. In any case, alpha-gal epitope has been significantly removed.

Figure **55** depicts the results of RT-PCR analysis relating to PERV for five examples of native porcine valves. As shown therein, PERV has been detected in the native porcine valves.

Figure **56** depicts results of RT-PCR analysis with respect to PERV for five cases of porcine valves treated with gamma-ray irradiation, and a positive control. As shown in the figure, no PERV has been detected in the decellularized porcine valve.

Figure **57** depicts the results of RT-PCR analysis relating to PERV for thirty days (3) after transplantation and fifty-six days (4) for porcine valves treated with gamma-ray irradiation, and a positive control (1; native porcine valves). As shown therein, no PERV has been detected in the decellularized porcine valves removed from the host thirty and fifty-six days after the transplantation.

BRIEF DESCRIPTION OF SEQUENCE LISTING

SEQ ID NO: 1 is a sequence of a forward primer sequence for detection of PERV.

SEQ ID NO: 2 is a sequence of a reverse primer sequence for detection of PERV.

SEQ ID NO: 3 is a sequence of a forward primer sequence for detection of control pig GAPDH.

SEQ ID NO: 4 is a sequence of a reverse primer sequence for detection of control pig GAPDH.

SEQ ID NO: 5 is a sequence of a forward primer sequence for detection of control dog GAPDH.

SEQ ID NO: 6 is a sequence of a reverse primer sequence for detection of control dog GAPDH.

DETAILED DESCRIPTION OF THE INVENTION

It should be understood throughout the present specification that expression of a singular form includes the concept of their plurality unless otherwise mentioned. Specifically, articles for a singular form (e.g. , "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include the concept of their plurality unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertain. If there is contradiction, the present specification (including the definition) precedes.

(Definition of Terms)

The definitions of terms used herein are described below.

(Regeneration Medicine)

As used herein, the term "regeneration" refers to a phenomenon in which when an individual organism loses a portion of tissue, the remaining tissue grows and recovers. The extent or manner of regeneration varies depending among animal species or among tissues in the same individual. Most human tissues have limited regeneration capability, and therefore, complete regeneration is not expected if a large portion of the tissue is lost. In the case of heavy damage, tissue having strong proliferation capability different from that of the lost tissue may grow, resulting in incomplete regeneration where the damaged tissue is incompletely regenerated and the function of the tissue cannot be recovered. In this case, a structure made of a bioabsorbable material is used to prevent tissue having a strong proliferation capability from infiltrating the defective portion of the tissue so as to secure space for proliferation of the damaged tissue. Further, by supplementation with a cell growth factor, the regeneration capability of the damaged tissue can be enhanced. For example, such regeneration techniques are applied to cartilages, bones, and peripheral nerves. It has been so far believed that nerve cells and cardiac muscles have no or poor regeneration capability. Recently, it was reported that there are tissue stem cells (somatic stem cells) which have both the capability of differentiating into these tissues and self-proliferation capability.

As used herein, the term "cell" is defined as having the widest meaning used in the art, referring to a structural unit of multicellular organisms, which has an enveloping membrane structure for separating the cell from the outside, has self-regeneration capability, and which is a living body having genetic information and an expression mechanism. In the method of the present invention, any cell can be used as a subject. The number of cells used in the present invention can be counted through an optical microscope. When counting using an optical microscope, the number of nuclei is counted. Tissues are sliced into tissue sections, which are then stained with hematoxylin-eosin (HE) to variegate nuclei derived from extracellular matrices (e.g., elastin or collagen) and cells. These tissue sections are observed under an optical microscope and the number of nuclei in a particular area (e.g., 200 $\mu$mm $\times$ 200 $\mu$m) can be estimated to be the number of cells.

Cells may elicit calcification and immune reactions. Therefore, non-self cells should be removed as much as possible for implantation of tissue or organs. In the case of self cells, decellularization is not required, since no immunological rejection problem is usually raised. However, since decellularization is sometimes preferable, cells should also be removed as much as possible. There is a strong desire for decellularized tissue.

As used herein, the term "cell replacement" indicates that cells originally existing are replaced with other infiltrating cells in decellularized tissue. This term is also referred to as "cellular infiltration". Preferably, in the present invention, cell replacement is carried out with cells of a host undergoing implantation.

As used herein, the term "tissue" refers to a group of cells having the same function and form in cellular organisms. In multicellular organisms, constituent cells are usually differentiated so that the cells have specialized functions, resulting in division of labor. Therefore, multicellular organisms are not simple cell aggregations, but constitute organic or social cell groups having a certain function and structure. Examples of tissue include, but are not limited to, integument tissue, connective tissue, muscular tissue, nervous tissue, and the like. Tissue targeted by the present invention may be derived from any organ or part of an organism. In a preferred embodiment of the present invention, tissue targeted by the present invention includes, but is not limited to, blood vessels, blood vessel-like tissue, cardiac valves, pericardia, dura mater, corneas, and bone tissue. Since non-micellar amphipathic molecules used in the present invention act in a mechanism like cell component extraction, tissue derived from any organ can be, in principle, treated with the method of the present invention.

As used herein, "membrane-like tissue" is also referred to as "planar tissue" and refers to a tissue having a membrane form. Membrane-like tissue includes tissue from organs, such as pericardia, dura mater, and corneas.

As used herein, "organ" or "part" is used interchangeably, referring to a structure which is a specific portion of an individual organism where a certain function of the individual organism is locally performed and which is morphologically independent. Generally, in multicellular organisms (e.g., animals and plants), organs are made of several tissues in specific spatial arrangement and tissue is made of a number of cells. Examples of organs or parts include organs or parts related to a blood vessel system. In one embodiment, examples of organs targeted by the present invention include ischemic organs (the heart undergoing cardiac infarction, skeletal muscle undergoing ischemia, and the like). In one preferred embodiment, organs targeted by the present invention are blood vessels, blood vessel-like tissue, cardiac valves, pericardia, dura mater, corneas, and bones. In another preferred embodiment, organs targeted by the present invention are cardiac valves, pericardia, and blood vessels.

In order to prepare grafts suitable for implantation, it may be optionally desirable that organs are cultured. Organ culture refers to in vitro culture of the whole or part of an embryonic or mature organ extracted from an organism where the structure, function and differentiation thereof are maintained. In contrast, general tissue culture mainly aims at cell proliferation, where dedifferentiation is likely to occur. Organ culture is conducted by methods, including, but not limited to, the watch glass method, the block Petri dish method, the support method, the sponge matrix method, the Trowell method, and the like. Also, large-scale cell culture (e.g., the hollow fiber method, the fixed bed method, the spin filter method, the precipitation tank method, perfusion culture, circulation type organ culture, etc.) as described in Tanpakushitsu Kakusan Koso [Protein Nucleic acid Enzyme] 45, 2188-2200(2000), may be used. An organ or tissue prepared by the decellularization method of the present invention is placed in a culture medium which is supplemented with various substances so as to regulate the structure, function, growth and differentiation, organ-to-organ interaction, and the like.

As used herein, the term "decellularization" and "decellularize" refers to removal of cells from tissue or organs. Preferably, decellularization may be carried out without damage to the structure and function of the original tissue or organs. Plasma components, cytosol components, cytoskeletons, and cell membrane components are removed from decellularized tissue. However, components required for maintaining the structure of tissue, such as extracellular matrix (ECM) components (e.g., elastin, collagen (Type I, IV), laminin, and the like), are maintained without degeneration. Therefore, decellularized tissue or organs are preferably substantially equivalent to untreated tissue or organs in terms of properties thereof, such as shape, physical strength, elasticity, flexibility, and the like. Since extracellular matrices are not degenerated after implantation, it is preferable to provide an environment suitable for the infiltration, adhesion, proliferation, and expression and maintenance of differentiation traits of the cells of recipients, so that the matrices are replaced with tissue made of self cells. The extent of decellularization can be measured using a cell residual rate as an index.

MHC class I and II proteins are preferably removed from decellularized tissue. MHC class I and II proteins can be confirmed by SDS PAGE and/or western blotting analysis. Other extracellular matrix proteins can be confirmed by SDS PAGE and/or western blotting analysis. Structural proteins in cells (collagen, elastin, and the like) can be evaluated by amino acid analysis (e.g., Edman degradation, automatic analysis using a peptide analyzer available from PE Biosystems, or the like). As a result, the influence of decellularized processes on ECM can be determined. Lipids and phospholipids contained in cell membrane and cells can be analyzed using thin layer chromatography and HPLC. Sugar chains (e.g., glycosaminoglycans or the like) can be analyzed by agarose gel electrophoresis or the like. This analysis can analyze the glycosaminoglycan composition of extracellular matrices as well as the presence of $\alpha$-Gal or the like.

As used herein, "cell residual rate" refers to a ratio of survival cells after decellularization of tissue by the method of the present invention to cells originally existing in the tissue. As used herein, the cell residual rate is typically measured by a counting method using a microscope after hematoxylin and eosin staining (H&E staining). This method comprises counting nuclei variegated by HE staining under an optical microscope. Therefore, for example, this method comprises the following steps: variegating samples by HE staining; counting the number of nuclei (the number of cells) present in an area of 100 mm x 100 mm in the sample; optionally repeating the counting step (e.g., a total of 8 times) and averaging the counts; and calculating a ratio to a control (e.g., untreated tissue is regarded as 100%). The ratio of the control (e.g., a ratio of survival nuclei to untreated tissue) can be regarded as a cell residual rate. Therefore, in this case, the cell residual rate (%) = (the number of nuclei in treated tissue)/(the number of nuclei in untreated tissue) x 100. The cell residual rate can also be determined by measuring the amount of survival DNA. This is because the total amount of DNA in cells of tissue is known to be generally proportional to the number of the cells of the tissue. Methods for measuring DNA are known in the art. For example, the amount of DNA extracted from tissue can be determined using a fluorescent reagent, such as Hoechst 33258 (from Molecular Probes) or the like. Specifically, tissue is isolated by ultrasonic pulverization treatment or the like; and the amount of DNA in extract supernatant can be determined by reacting with Hoechst 33258 (Ex (Excitation wavelength) 356, EM (Emission wavelength) 492), which is capable of specifically binding to DNA components in buffer solution and emitting fluorescence, and measuring the strength of fluorescence. The term "less than a level which can elicit an immune reaction in organisms" of the cell residual rate of certain tissue indicates that when the tissue is implanted into organisms, no immune reaction is elicited for a certain period of time (e.g., several months to several years, preferably the entirety of the rest of life).

As used herein, the term "immune reaction" refers to a reaction due to loss of coordination of immunologic tolerance between a graft and a host, including, for example, hyperacute rejection reactions (within several minutes after implantation; immune reactions due to an antibody, such as $\beta$-Gal or the like), acute rejection reactions (cell-mediated immune reactions about 7 to 21 days after implantation), chronic rejection reactions (rejection reactions due to cell-mediated immune response after three months), and the like.

As usedherein, elicitation of immunereactions can be determined by histological examination of the type, number, or the like, of cells (immune cells) infiltrating implanted tissue by observing under a microscope tissue sections stained by HE staining or immunological staining.

As usedherein, the term "calcification" refers to precipitation of calcareous substances in organisms.

As used herein, "calcification" *in vivo* can be determined by measuring calcium concentration. Specifically, implanted tissue is taken out; the tissue section is dissolved by acid treatment or the like; and the atomic absorption of the solution is measured by a trace element quantifying device.

As used herein, the term "within organism(s) (or in organism(s))" or "in vivo" refers to the inner part of organism (s). In a specific context, "within or ganism(s)" refers to the position at which a subject tissue or organ is placed.

As used herein, "in vitro" indicates that a portion of an organism is extracted or released outside the organism for various purposes of research (e.g. , in a test tube). The term *in vitro* is in contrast to the term *in vivo.*

As used herein, the term "*ex* vivo" refers to a series of operations where target cells into which a gene will be introduced are extracted from a subject; a therapeutic gene is introduced in vitro into the cells; and the cells are returned into the same subject.

As used herein, the term "a function in the untreated (naive) form" of certain tissue refers to a function possessed *in vivo* by the tissue in its normal state. Therefore, for example, in the case of cardiac valves, cardiac valves usually have a function of preventing a back flow of blood from a ventricle to an atrium or from the pulmonary artery and aorta to an atrium, a function in the untreated form of cardiac valves refers to the function of preventing a back flow of blood from a ventricle to an atrium or from the pulmonary artery and aorta to an atrium.

As used herein, the term "extracellular matrix" (ECM) refers to a substance existing between somatic cells no matter whether the cells are epithelial cells or non-epithelial cells. Extracellular matrices are involved in supporting of tissue as well as internal environmental structure essential for survival of all somatic cells . Extracellular matrices are generally produced from connective tissue cells. Some extracellular matrices are secreted from cells possessing basal membrane, such as epithelial cells or endothelial cells. Extracellular matrices are roughly divided into fibrous components and matrices filling there between. Fibrous components include collagen fibers and elastic fibers. A basic component of matrices is a glycosaminoglycan (acidic mucopolysaccharide), most of which is bound to non-collagenous protein to form a polymer of a proteoglycan (acidic mucopolysaccharide-protein complex). In addition, matrices include glycoproteins, such as laminin of basal membrane, microfibril around elastic fibers, fibers. fibronectins on cell surface, and the like. Particularly differentiated tissue has the same basic structure. For example, in hyaline cartilage, chondroblasts characteristically produce a large amount of cartilage matrices including proteoglycans. In bones, osteoblasts produce bone matrices which cause calcification. One embodiment of the present invention may be characterized in that extracellular matrices (e.g., elastin, collagen (e.g., Type I, IV, and the like), and the like) are not substantially changed from before the decellularization treatment of the present invention.

As used herein, the term "tissue damage rate" refers to a parameter indicating a function of tissue or an organ, which is an index of how much tissue or an organ is damaged and damaged after treatment or an index of how much an original function of the tissue or organ can be exhibited. As used herein, a method for measuring a tissue damage rate is well known in the art. For example, the rate can be determined by counting elastin rupture sites. In a method used herein, a visual field is divided into units of 100 $\mu$m x 100 $\mu$m and the number of units having an elastin rupture site is counted. There were 24 units per visual field. In HE-stained tissue sections, extracellular matrices were counted under a microscope. A damage rate is calculated as x/24, where untreated tissue is assumed to have a damage rate of 0%, i.e., x=0.

As used herein, the "tissue strength" refers to a parameter indicating a function of tissue or an organ, which is the physical strength of the tissue or organ that is generally determined by measuring tensile strength. Such a tensile test is well known in the art.

In the present specification, rupture load is employed as strength, which is measured as follows: a specimen is cut out into a strip of 5 mm in width and 30 mm in length (basically aortic base portion is cut such that the wall portion thereof is longer in the direction of the length); (2) the specimen is fixed for about 5 mm in the fixing member of tension testing machine (TENSILON RTC1150A: available from ORIENTEC) ; and (3) the specimen is stretched at a test speed of 1 cm/min by the tension testing machine to rupture point load. Herein, rupture load and elastic module can be measured.

The tissue strength of the decellularized tissue of the present invention in terms of the maximum load thereof may be usually at least about 8 N, preferably at least about 10 N, and more preferably at least about 11N. In the prior decellularized tissue or naturally-occurring tissue (for example, arteria), the strength thereof is usually about 7N. Further, conventional decellularization treatments have little effects on enhancement of the tissue strength. As such, the capability of tissue strength enhancement by the present invention is unexpectedly significant.

In a preferable embodiment, in terms of coefficient of elasticity, the decellularized tissue of the present invention usually has coefficient of elasticity of at least about 1.2 MPa or greater , preferably at least about 2 MPa. The decellularized tissue of the present invention may have a coefficient of elasticity less that that of a naturally occurring tissue (for example, 0.8MPa or greater, or 0.8-1.0MPa or the like), as long as the decellularized tissue can be used for the purpose of the present invention.

In measuring extension, tensile testing apparatus used in the strength measurement (TENSILLON ORIENTEC) may be used. Specifically, rectangular material having width of 5 mm and length of 30mm is loaded with weight at a speed of 10mm/min, and thus rupture load and elasticity efficiency may be measured. Specifically, measurement of extension may be obtained by measuring the length of each direction before and after the tension stimulus, dividing the length after the tension by that prior to the tension and multiplying the result. Usually, the extension is measured for both longitude and horizontal directions. The extension for both directions is preferably uniform but it is not limited thereto. Depending on the application thereof, the properties relating to the extension may be but is not limited to, e.g. at least 120%, preferably 150 %. With respect to the extension properties, the decellularized tissue of the present invention may have extension less than that possessed by a native tissue (for example at least 50 % or the like), as long as it can be used.

A general tensile testing method is well known and is not herein described in detail. By analyzing data obtained by a general tensile testing, various data such as breaking strength, stiffness, Young's modulus, and the like can be obtained and can be herein used as indexes of tissue strength. As used herein, in the case of luminal tissue, tissue strength can be represented by a stiffness parameter ($\beta$ value). A $\beta$ value is calculated by the following formula after the P-D (pressure-diameter) relationship is prepared:

$$Ln(P/Ps) = \beta(D/Ds-1) \qquad (1)$$

where Ps and Ds represent standard values at 100 mmHg and P and D represent pressure and diameter, respectively.

The opposite ends of luminal tissue, such as a blood vessel or the like, are fixed to pipe-like units or the like and the outside and inside of the luminal tissue are filled with physiological saline. In this situation, pressure is applied to the inside of the tissue by an external device, while monitoring the outer diameter of the tissue. The measured pressure and diameter are inserted into formula (1) to obtain a $\beta$ value (Sonoda H., Takamizawa K., et al., J. Biomed. Mater. Res. 2001:266-2'76).

As usedherein, the term "amphipathicmolecule" refers to a molecule having both a hydrophilic group (carboxyl group, sulfate group, quaternary ammonium group, hydroxyl group, etc.) and a hydrophobic group (also referred to as lipophilic group; including a long chain hydrocarbon group and the like). Amphipathic molecules have affinity for either polar solvents or non-polar solvents. This property is called amphiphilicity.

As used herein, the term "surfactant" refers to a substance which is dissolved in liquid and has an action to significantly reduce the surface tension of the liquid. There is a hydrophilic portion and a hydrophobic portion present within the molecule, so that the molecule is easily adsorbed onto a surface. Surfactant molecules form a molecular cluster, which is called micelle, at a certain concentration (critical micelle concentration) or more. Therefore, the term "surfactant" as used herein partially overlaps the term "amphipathic molecule". As used herein, the term "micelle" refers to a cluster of amphipathic molecules, such as a surfactant, formed when the molecules are dissolved in water at a certain concentration or more, in which the hydrophilic groups face the outside of the cluster while the lipophilic groups face the inside of the cluster. Formation of micelles abruptly occurs at a certain concentration called the "critical micelle concentration". The properties of an aqueous solution significantly changes at this concentration. Micelle-forming molecules are strongly absorbed onto a two-phase interface due to the hydrophilic-lipophilic balance of a surfactant. As a result, the free energy of the interface is significantly reduced so that cell components, such as proteins, lipids, and the like, are solubilized. Surfactants for decellularization are generally used at a critical micelle concentration or more (e.g., 1%). Therefore, decellularization treatment is achieved by a mechanism of decreasing free energy. The critical micelle concentration is determined depending on the substance, and is well known or can be determined by preparing its aqueous solution.

As used herein, the term "non-micelle forming" refers to a property of an amphipathic molecule which does not form micelles at a predetermined concentration. Preferably, the property of forming no micelles may be maintained at any concentration. It is desirable that substances used in the present invention (e.g. , polyethylene glycol) have no critical micelle concentration in an aqueous solution.

As used herein, the term "non-micelle forming amphipathicmolecule" refers to an amphipathic molecule which does not form micelles at a predetermined concentration. Preferably, such a molecule has no critical micelle concentration within a concentration range. Therefore, the molecule is not micellar at any concentration. When a non-micelle forming amphipathic molecule is used, decellularization treatment may be carried out by a mechanism similar to extraction of cell components using a chemical extraction technique. Therefore, decellularization treatment using a non-micelle forming amphipathic molecule solution is different from conventional decellularization treatment using a surfactant. As a result, a significant difference occurs in the degree of tissue damage. For decellularization treatment, any non-micelle forming amphipathic molecule, preferably 1,2-epoxide polymer, and more preferably polyethylene glycol, may be herein used.

As used herein, the term "non-micellar" refers to a state of not forming micelles when an amphipathic molecule is mentioned. Such a non-micellar state may be achieved by an amphipathic molecule at less than a critical micelle concentration or a non-micelle forming amphipathic molecule. Therefore, such a non-micellar state may be achieved with 1,2-epoxide polymer (particularly, polyethylene glycol). Alternatively, a surfactant, such as SDS, Triton X-100 (registered trademark), or the like, may be used to achieve a non-micellar state when the surfactant is present at less than a critical micelle concentration, and can be used in the present invention.

As used herein, the term "1,2-epoxide polymer" refers to a polymer formed by polymerization of 1,2-epoxide as a monomer. 1,2-epoxide polymers include, but are not limited to, ethylene oxide polymers or copolymers therewith, propylene oxide polymers or copolymers therewith, and higher 1,2-epoxide polymers or copolymers thexewith. 1, 2-epoxide is a compound having a structure in which oxygen atoms are bound to two carbon atoms which has been bound to each other within the molecule, i.e., an oxygen atom is bound to each of positions 1 and 2. Examples of 1, 2-epoxides include, but are not limited to, ethylene oxide, propylene oxide, butylene oxide, epichlorohydrin, and the like. Examples of 1,2-epoxide polymers include, but are not limited to, ethylene oxide polymers, propylene oxide polymers, copolymers therewith, polypropylene glycol, polyethylene glycol, and the like. Preferably, 1,2-epoxide polymers include, but are not limited to, polyethylene glycol, polypropylene glycol, and the like. A more preferable 1,2-epoxide polymer is polyethylene glycol. Preferably, 1,2-epoxide polymers have amphipathicity.

As used herein, the term "polyethylene glycol (PEG)" refers to a polymer of ethylene glycol, and is also referred to as polyethylene oxide (poly(ethylene oxide)) represented by $HO-(CH_2CH_2O)_n-H$. Polyethylene glycol is commercially available from various companies, such as, for example. Union Carbide (Carbowax), Dow (PolyglycolE), Texaco Chemical (Jeffox), Olin (PolyG), BASF Wyandotte (Pluracol E), Hodag, ICI Americas (ATEG), Nacalai tesque, NOF Corporation, and the like. Typically, PEG has an average molecular weight of between 200 and 6000. Such a PEG is, for example, polyethylene glycol ($H(OCH_2CH_2)_nOH$) having a # molecular weight of, for example, 200, 600, 1000, 2000 or 6000 from Nacalai tesque. Preferably, PEG has an average molecular weight of between 1000 and 2000. In another preferred embodiment, PEG may have an average molecular weight of 1000 or less. More preferably, PEG may have an average molecular weight of between 1500 and 2000. The above-described PEGs are commercially available and can be appropriately synthesized in order to achieve the desired characteristics. Such a synthesis method is well known in the art. Note that average molecular weights and molecular weights are herein represented by Daltons. Preferably, PEG used in the present invention has a uniform molecular weight. This is not essential. PEG having an averagemolecular weight within a certain range can be usually used. In the present invention, a substance (e.g., an enzyme (e.g., DNaseI or the like), an enzyme inhibitor, or the like) can be added in addition to PEG for the purpose of decellularization. When a chemical substance is added in addition to PEG, the amount or concentration of PEG to be added may vary depending on the amount of the existing chemical substance. A concentration used for cell fusion or the like is preferable. For example, a concentration of 1 g/ml or more (100 w/w or more) is preferable, but the present invention is not limited to this.

As used herein, the term "immerse" refers to placing a certain object in a certain fluid (e.g. , liquid). In the present invention, the term "immerse" indicates that tissue to be treated is placed in a solution containing a non-micellar amphipathic molecule (e.g., a 1,2-epoxide polymer, such as polyethylene glycol) of the present invention for treatment. Therefore, the immersing step is preferably carried out, preferably, in a manner that permits tissue to be treated to be completely immersed in a solution for treatment. Also, in the immersing step, tissue to be treated may be preferably subjected to physical treatment (e.g., rubbing or pressing with a glass rod) in order to more efficiently remove components.

As used herein, the term "washing" step indicates that after performing the step of immersing tissue to be treated in a solution of a non-micellar amphipathic molecule (e.g., a 1,2-epoxide polymer) of the present invention, the solution is removed from the tissue. Therefore, preferably, the washing step is performed using liquid. In the present invention, since treated tissue is intended to be used in an organism, the tissue is preferably washed with physiologically acceptable liquid. In one preferred embodiment, the washing step may be performed using PBS (phosphate buffered saline). A wash solution may optionally contain other pharmaceutical agents (e.g., a protease inhibitor). The other pharmaceutical agents are preferably not toxic and are biocompatible.

As used herein, the term "chemical treatment" refers to treating (e.g., immersing) a certain object with a chemical substance in a broad sense. Note that as used herein, the term "chemical treatment" refers to steps other than the step of immersing an object in a solution containing a 1,2-epoxide polymer (e.g., polyethylene glycol) as an essential step. Therefore, in a method of the present invention, for example, when chemical treatment is performed in addition to the step of immersing an object in a solution containing polyethylene glycol having an average molecular weight of 1000 to 2000, chemical treatment includes the step of immersing the object in a solution other than the solution containing polyethylene glycol having an average molecular weight of 1000 to 2000 (e.g., a DNaseI solution, a glutaraldehyde solution, a solution containing polyethylene glycol having another average molecular weight, other 1, 2-epoxide polymer solutions, and the like, but not limited to these solutions).

As used herein, the term "radical reaction" is also called group/radical leaving reaction and refers to a chemical reaction inwhich a free radical group is involved in the course of a reaction.

Radical reactions include, for example, as follows:

1) a reaction by cleavage of stable bonds in a molecule to result in a free radical.

e.g. $C_3H_8- >CH_3'+C_2H_5"$

2) a reaction between a free radical and a stable molecule to result in a different molecule and a different free radical.

e.g., $CH_3'+CH_3COCH_3->CH_4+CH_3COCH_3'$

3) a reaction between a free radical and a stable molecule to form a larger free radical

e.g. $CH_3'+C_2H_4->C_3H_7'$

4) a reaction of degradation of a free radical to result in a smaller free radical and a molecule

e.g. $n-C_3H_7'->C_2H_4+CH_3'$

5) a reaction between free radicals, which include:

recombination reaction $CH_3·+CH_3'->C_2H_6$

disproportionation $C_2H_5"+C_2H_5'->C_2H_4+C_2H_6$.

As used herein the term "radical", is also referred to as "free radical" , and refers to a chemical species having an unpaired electron. Those having two unpaired electrons are called biradicals. Radicals include, but are not limited to, e.g. , stable radicals, which can be handled as a normal substance, such as ozone, hydrogen peroxide, and 2,2-diphenyl-1-picrylhydradyl (DPPH), di-t-butyl aminoxyl and the like; radicals which can only exist in a solution such as triphenylmethyl free radical and the like.

As used herein, whether or not "radical reaction" has been undergone may be confirmed by observing dynamic changes in fragility; reduction of mechanical strength, such as reduction in rupture strength in a tensile test, and observing by means of a microscope, elastin in vessels or valves or the like, laminar structure of elastic plate comprising a number of extracellular matrices such as collagen, damage in fillet structure which is constituted thereby in terms of molecular structure.

As used herein, the term "free radical source" refers to an agent which can provide a radical, and includes, but is not limited to, a substance in a state of a radical as described above, and apparatuses which are capable of providing a radical (for example, X-ray irradiation apparatus, ultraviolet irradiation apparatus, electron beam irradiation apparatus, gamma-ray irradiation apparatus, and the like), and the like.

Generally, such a free radical is produced by cleaving chemical bonds by means of, e. g. , X-ray irradiation, ultraviolet irradiation, electron beam irradiation, gamma-ray irradiation, heat degradation, light degradation, and radioactive irradiation degradation of a molecule, and donation and reception of an electron and the like. Free radicals thus produced are extremely rich in chemical activity such as methyl free radical $CH_3$ methylene free radical $:CH_2$ and the like, and rapidly changes by reactions between radicals per se, or with a stable molecule (radical reaction). Such a free radical is an example of free radical source used in the present specification.

Search for such a product allows one to know whether or not and which free radical is produced and reacted. The presence of radicals may be confirmed by measuring methods such as chemical methods, electron spectrum or paramagnetic resonance absorption and the like. The presence of radicals having extremely short lifetime (for example, · OH) may be confirmed by using a flash photolysis method, rigid solvent method, and matrix isolation method and the like. When a metalatom of a transition metal complex has one or a plurality of unpaired electrons, it is not referred to as free radical.

As used herein the term "gamma-ray" refers to a electromagnetic wave having wavelength shorter than 0.001 nm. When expressed in terms of energy, it corresponds to a light quantum of several hundreds keV. Usually, gamma-ray irradiation is released upon electron transitions between energy levels of atomic nucleus. The source of gamma-ray irradiation includes, for example, any radioactive irradiation source, and preferably a irradiation source of $^{60}$Co(cobalt60) or $^{137}$Cs(cesium 137), as they are preferable for treating decellularization tissue.

Exposure or irradiation doses of gamma-ray irradiation is often expressed in kGy unit. Exposure dose may be measured by measuring absorbed dose using devices such as a cavity ionization chamber, a calorimeter, a film dosimeter, a PMMA dosimeter and the like.

As used herein the term "ultraviolet (ray or irradiation)" means an electromagnetic wave of wavelength within the range of about 360-400 nm as an upper limit for short wavelengths of visible light and about 1 nm as a lower limit. The lower limit is not clear, and overlaps with soft x-ray irradiation for the range of tens of nm, and thus as used herein it is understood that ultraviolet ray refers to the range overlapping with x-ray irradiation. Light sources used therefore include but are not limited to, for example, quartz lamp, carbon arc lamp, spark discharge, hydrogen or rare gas discharge, synchrotron irradiation and the like.

As used herein the term "x-ray", also refers to roentgen, and refers to an electromagnetic wave having wavelength of 0.001 nm or greater, preferably 0.01 nm to tens of nm. X-ray irradiation has greater permeability at shorter wavelengths and gradually transits to gamma-ray irradiation. At longer waverlengths x-ray irradiation has lower permeability and gradually transits to ultraviolet irradiation. Accordingly, the wavelengths of x-ray irradiation partially overlaps with those of gamma-ray and ultraviolet irradiation. Electrons emitted by heat from the negative pole of the X-ray tube is accelerated under high pressure and collapsed at the anti-negative pole (i.e. positive pole), which is cooled by water, to form x-ray irradiation. Regardless of the material of the anti-negative pole, the x-ray irradiation is classified into two types: continuous x-ray irradiation of continuous spectra caused by controlled irradiation and specific x-ray irradiation having line spectrum which is inherent to the material of the anti-negative pole. Any type of x-ray irradiation may be used in the present invention. Irradiation sources of x-rays include but are not limited to heat electron x-ray tube (Coolige tube), gas x-ray tube and the like. Electron beams emitted by heat from the negative pole is focussed on the positive pole surface by focussing by the Wehnelt electrode to produce x-ray irradiation. Materials from whichx-ray irradiation is extracted include berylliumplates and the like. Materials used for the positive pole include tungsten if continuous x-ray irradiation is desired, and if one wishes to obtain specific X-ray irradiation, like Ka line, include iron, copper, molybdenum, silver, and the like. Acceleration voltage is usually between the range of 30-100kV, but are not limited thereto. If one wished to obtain a x-ray irradiation having greater permeability, higher voltages are used. As used herein, it is understood that any acceleration voltage may be used.

As used herein the term "radical scavenger" refers to a molecule which reduces chemical activity of a radical by binding the free radical in a chemical or biochemical system. Although not wishing to be bound to any theories, the amphipathic molecule used in the present invention is expected to act as a radical scavenger.

As usedherein, radical reactions are usually achieved by exposure to a free radical but is not limited thereto. Radical reactions can be achieved by, for example, gamma-ray irradiation, ultraviolet irradiation, exposure to a free radical source, ultrasonication, x-ray irradiation and the like. Free radical sources include, for example, stable radicals as described above, radicals present in a solution and the like but are no limited thereto. Radical reactions by irradiation are preferable. This is because it can spread the radical reaction effects to the entire tissue.

As used herein, the term "electron beam" refers to a beam of electrons having some substantially stable kinetic energy and have wave properties expressed by a de Broglie wave. Usually, it can be produced by accelerating heated electrons in vacuo. Electron beam sources include, but are not limited to, for example, electron tubes, electron guns and the like. Electron guns are apparatuses for emitting an electron beam in a specific direction, and comprises a negative pole for emitting electrons and a positive pole for acceleration. Usually, it uses a heated negative pole and a number of circular plates or circular electrodes having a channel in the center on the way to the positive pole in an coaxial manner to produce a beam having a desired width and parallelism. It can also use a cold negative pole release or gas discharge.

For the above-described ultraviolet irradiation, measurement is made by actinometer. Ultrasonication exposure is measured by calorimeter or indicated by input of electric power or the amount of hydrogen peroxide produced. Electron beam is measured by an electric current measurement or in the same manner as the gamma-ray irradiation. X-ray is measured in the same manner as the gamma-ray irradiation, using a measuring decive such as a cavity ionization chamber , a calorimeter , a film dosimeter, a PMMA dosimeter and the like.

As used herein the term "vacuum" refers to a pressure of $1 \times 10^{-1}$Pa or less. As used herein the term "under reduced pressure" refers to a pressure of the range between $1 \times 10^{-1}$Pa to $1 \times 10^{5}$Pa.

As used herein the term "atmosphere" is used in the same manner as commonly used, and usually refers to what consists of 78% nitrogen, 21% oxygen, 1% argon, 0.03% carbon dioxide, about 0.3 % water vapor.

As used herein the term "in oxygen" refers to a circumstance at which the oxygen concentration thereof is higher than that of the atmosphere. Preferably, "in oxygen" refers to the existence of substantially oxygen only. As used herein the term "in water" refers to a reaction in a solvent substantially consisting only of $H_2O$. Water includes tap water, distilled water, ion exchanged water and the like.

As used herein the term "amphipathic molecule solution" in which a radical reaction is performed, may or may not be substantially the same amphipathic molecule solution used in the decellularization.

As used herein, the "physiologically active substance" refers to a substance which acts on cells or tissue. Physiologically active substances include cytokines and growth factors. Physiologically active substances may be naturally occurring or synthetic. Preferably, a physiologically active substance is one that is produced by cells or one having an action similar to that. As used herein, physiologically active substances may be in the form of a protein or nucleic acid or in other forms. When cytokines actually exert an action, the cytokines are usually in the form of proteins.

As used herein, the term "cytokine" is used in its broadest sense in the art, referring to a physiologically active substance which is produced by a cell and acts on the same or different cell. Cytokines are generally proteins or polypeptides, which have an action of controlling an immune response, an action of regulating an endocrine system, an action of regulating a nerve system, an anti-tumor action, an anti-virus action, an action of regulating proliferation, an action of regulating differentiation, and the like. As used herein, cytokines may be in the form of proteins or nucleic acids, or in other forms. When cytokines actually exert an action, the cytokines are usually in the form of proteins.

As used herein, the term "growth factor" or "cell growth factor" are used interchangeably, referring to a substance capable of promoting or controlling cell growth. Growth factors are also referred to as proliferation factors or development factors. Growth factors may be added to a medium in cell or tissue culture and may substitute for actions of serum macromolecule substances. It has been revealed that a number of growth factor s can function as factors controlling differentiation of cells in addition to cell growth.

Cytokines include, typically, interleukins, chemokines, hemopoietic factors (e.g., colony stimulating factors), tumor necrosis factors, and interferons. Growth factors include, typically, aplatelet-derivedgrowthfactor (PDGF), an epidermial growth factor (EGF), a fibroblast growth factor (FGF), a hepatocyte growth factor (HGF), and a vascular endothelial growth factor (VEGF), which have growth activity.

Physiologically active substances, such as cytokines and growth factors, generally have a redundancy of functions. Therefore, even a cytokine or growth factor known under a different name and function may be used in the present invention as long as it has the same function as that of a physiologically active substance of the present invention. If cytokines or growth factors have preferable activity as set forth herein, these substances can be used in a treatment method or medicament according to a preferred embodiment of the present invention.

As used herein, the term "differentiation" refers to a developmental process of a portion of an organism, such as a cell, tissue, or an organ, in which a characteristic tissue or organ is formed. The term "differentiation" is used mainly in embryology, developmental biology, and the like. An organism forms various tissue and organs from the division of a fertilized ovum to the formation of an adult. In an early stage of development of an organism before division or in inadequate division, individual cells or cell groups have no morphological or functional feature and it is difficult to separate the cells or cell groups. This state is called "undifferentiated" . "Differentiation" also takes place at the organ level. Cells constituting an organ develop various characteristic cells or cell groups. This is called "differentiation" in an organ during the formation of an organ. Therefore, regarding regeneration in the present invention, cell differentiation means that a cell acquires a certain morphological or functional feature which had not been possessed by the cell before treatment. For example, in the case of a cardiac valve, when a stem cell (e.g., an embryonic stem cell or a tissue stem cell) is provided, the stem cell is differentiated into a cell which is morphologically or functionally similar to a cell or tissue existing in the cardiac valve to some degree.

As used herein, the terms "graft" and "tissue graft" are used interchangeably, referring to homologous or heterologous tissue or cell group which is inserted into a particular site of a body and thereafter forms a portion of the body. Examples of grafts include, but are not limited to, organs or portions of organs, blood vessels, blood vessel-like tissue, skin segments, cardiac valves, pericardia, dura mater, corneas , bone segments, teeth, and the like. Therefore, grafts encompass any one of these which is inserted into a deficient portion so as to compensate for the deficiency. Grafts include, but are not limited to, autografts, allografts, and xenografts, which depend on the type of their donor.

As used herein, the term "autograft" refers to a graft which is implanted into the same individual from which the graft is derived. As used herein, the term "autograft" may encompass a graft from a genetically identical individual (e.g. an identical twin) in a broad sense.

As used herein, the term "allograft" refers to a graft which is implanted into an individual which is the same species but is genetically different from that from which the graft is derived. Since an allograft is genetically different from an individual (recipient) to which the graft is implanted, the graft may elicit an immune reaction. Such a graft includes, but is not limited to, for example, a graft derived from a parent.

As used herein, the term "xenograft" refers to a graft which is implanted from a different species. Therefore, for example, when a human is a recipient, a porcine-derived graft is called a xenograft.

As used herein, "recipient" (acceptor) refers to an individual which receives a graft or implanted matter and is also called a "host". In contrast, an individual providing a graft or implanted matter is called a "donor" (provider).

With the decellularization technique of the present invention, any graft can be used. This is because a graft (e.g., tissue, an organ, or the like) decellularized by a method of the present invention retains such a tissue damage rate that does not hinder therapy (i.e., a low tissue damage rate) and adverse effects, such as elicitation of an immune reaction, calcification, and the like, are significantly suppressed. Therefore, even in conventional situations where only an autograft is available, allografts or xenografts can be used. This is one of the significant effects of the present invention which cannot be achieved by conventional techniques.

As used herein, the term "subject" refers to an organism to which treatment of the present invention is applied and is also referred to as "patient". A patient or subject may be preferably a human.

Cells optionally used in the method or tissue graft of the present invention may be derived from a donor genetically identical to the recipient (autologous cells); a donor genetically different from, though of the same species as the recipient (homologous cells); or a donor of a species different from the recipient (heterologous cells). Considering rejection reactions, autologous cells are preferable. When rejection reactions raise no problem, homologous cells may be used. Further, if cells capable of causing rejection reactions are treated to overcome the rejection reaction, such cells can be used. A method for avoiding rejection reactions is known in the art and is described in, for example, "Shin-Geka-Taikei, Shinzo-Ishoku Hai-Ishoku Gijyutsuteki, Rinriteki-Seibi-kara-Jissi-nimukete [Heart Transplant Lung Transplant - From Technical and Ethical Development to Practice]" (3rd Version). Examples of such a method include use of an immunosuppressant or a steroid drug, and the like. Immunosuppressants for preventing rejection reactions currently include "cyclosporine" (SANDIMMUNE/NEORAL), "tacrolimus" (PROGRAF), "azathioprine" (IMURAN), "steroid hormone" (prednine, methylprednine), and "T-cell antibodies" (OKT3, ATG, etc.). A method which is used for preventive immunosuppressive therapy in a number of facilities in the world is three-drug therapy using "cyclosporine, azathioprine, and steroid hormone". An immunosuppressant is preferably, but not necessarily, administered concomitantly with a medicament of the present invention. Therefore, an immunosuppressant may be administered before or after a regeneration/therapeutic method of the present invention as long as an immunosuppression effect can be achieved.

Cells used in the present invention may be derived from any organism (e.g., vertebrates and invertebrates). Preferably, cells derlvedfromvertebrates are used. More preferably, cells derived from mammals (e.g., primates, rodents, etc.) are used. Even more preferably, cells derived from primates are used. Most preferably, cells derived from humans are used.

Combinations of a subject targeted by the present invention and decellularized tissue include, but not limited to, for example, implantation to a heart suffering from a heart disease (e.g., ischemic heart diseases); pericardia patch, implantation of dura mater in brain surgery: implantation of a blood vessel to treat cardiac infarction, a lower limb, an upper limb, or the like; implantation of a bone to a patient suffering from bone fracture or bone failure; implantation of cornea of the present invention to a patient having an injured cornea; and the like.

Tissue targeted by the present invention may be any organ or part of an organism or may be derived from any kind of organism. Organisms targeted by the present invention include vertebrates and invertebrates. Preferably, organisms targeted by the present invention are mammals (e.g., primates, rodents, etc.). More preferably, organisms targeted by the present invention are primates. Most preferably, organisms targeted by the present invention are humans, particularly autologous.

Combinations of a subject targeted by the present invention and decellularized tissue include, but not limited to, for example, implantation to the heart suffering from a heart disease (e.g., ischemic heart diseases); pericardia patch, implantation of dura mater in brain surgery; implantation of abloodvessel to treat cardiac infarction, a lower limb, an upper limb, or the like; implantation of a bone to a patient suffering from bone fracture or bone failure; implantation of cornea of the present invention to a patient having an injured cornea; and the like.

Tissue targeted by the present invention may be any organ or part of an organism or may be derived from any kind of organism. Organisms targeted by the present invention include vertebrates and invertebrates. Preferably, organisms targeted by the present invention are mammals (e.g., primates, xodents, etc.). More preferably, organisms targeted by the present invention are primates. Most preferably, organisms targeted by the present invention are humans.

Attention has been attracted by blood vessel regeneration therapy using self cells, and a method for implanting tissue can be carried out as well known in the art. In the cardiovascular surgery region, various prosthetic valves or vascular prostheses or the like are used, however, a number of problems arise, such as their durability or necessity of anticoagulant therapy, and associated hemorrhagic tendency and susceptibility to infection, and the like. Therefore, there is an expectation for regeneration medical engineering. Shin'oka et al. prepared regenerated blood vessels by culturing blood vessel smooth muscle cells from a vein of a foot of a 4-year old female with pulmonary artery deficiency on a bioabsorbable high molecular weight polymer and carried out implantation of the regenerated blood vessel (Toshiharu Shin' oka, Yasuharu Imai, Kazuhiro Seo, et al.; "Tissu-Enjinearinngu-niyoru-Shinkekkanzairyo-no-Kaihats u, Oyo [Development and Application of Cardiovascular Material in Tissue Engineering]", Nichi Shinzo Kekkan Gekaishi, 2000; 29: 38). In tissue engineering in the cardiovascular system, implanted cells or structures can make direct contact with blood in a blood vessel and are therefore supplied with oxygen and nutrients immediately after implantation. Thus, implanted cells or structures are considered to be under advantageous conditions. Particularly, use of self cells (cell replacement) has various advantages as follows, for example.

1. Removal of the possibility of a rejection reaction.
2. No necessity of considering a donor.
3. Expectation of long durability due to utilization of living tissue.
4. No residual foreign matter since a scaffold polymer is completely biodegraded when cellscomplete the extracellular stroma.
5. Excellent antithrombogenicity due to eventual coverage with endothelia and no necessity of anticoagulant therapy after implantation.
6. Expectation of growth due to autologous tissue.

At present, self cells are used in the right arterial system within a blood pressure range around a pulmonary artery pressure. However, self cells can be appropriately applied to use with an aorta pressure, artery grafts for valve tissue or AC bypass, tendinous cord tissue, or the like ("Junkanki-Shikkan-no-Saishin-Tiryo 2002-2003 [Up-to-date Therapy for Cardiovascular diseases 2002-2003]"; Nankodo, p. 29, published in 2002).

General use of prosthetic valves is well known in the art. The present invention is also carried out based on this well-known matter. For example, stentless heterologous tissue valves are known. In heterologous tissue valves, the presence of a stent reduces the effective area of the valve port, leading to calcification or degeneration of valve leaflets. Recently, by utilizing the morphology of the base portion of the porcine aorta, a stentless heterologous tissue valve stent has attracted attention as a prosthetic valve for the aortic valve (Gross C., et al., Ann. Thorac. Surg., 68:919, 1999). It is considered that the absence of a stent results in a small pressure difference across the valve even when a small-size valve is unavoidably used and is also effective for postoperative enlargement of the left ventricle. Further, the elasticity of the base portion of the aorta is maintained, stress to the cusp is small, and the durability can be expected to be improved as compared to a tissue valve with a stent. Further, stentless heterologous tissue valves can be used in the case of endocarditis due to infection or prosthetic valve infection. At present, substantially satisfactory intermediate-term postoperative results of stentless heterologous tissue valves have been reported in the USA and Europe, and long-term results can be expected to be satisfactory (Gross C. , et al., Ann. Thorac. Surg., 68: 919, 1999).

BEST MODE FOR CARRYING OUT THE INVENTION

(Preferable embodiments)

Hereinafter , preferred embodiments of the present invention will be described. The following embodiments are provided for a better understanding of the present invention and the scope of the present invention should not be limited to the following description. It will be clearly appreciated by those skilled in the art that variations and modifications can be made without departing from the scope of the present invention with reference to the specification.

In one aspect, the present invention provides a decellularized tissue which has been subjected to a radical reaction. The decellularized tissue is characterized in that the tissue strength thereof is substantially identical to decellularized tissue not subjected to radical reaction. To date, when promoting decellularization, the tissue strength thereof has been significantly reduced (i.e., the state of tissue being "frazzle"). The present invention achieved progression in decellularization while maintaining tissue strength thereof in an unexpected manner by means of radical reaction (e.g., gamma-ray irradiation). Conventionally, it has been difficult to maintain sufficient tissue strength with the cell residual rate being less than 30%, in particular less than 5%. However, the present invention has unexpectedly achieved such decellularization.

In one embodiment, whether or not radical reaction has been subjected to may be confirmed by the substantial absence of eluted proteins . Accordingly, in the present embodiment, the present invention provides a decellularized tissue wherein eluted proteins do not substantially exist . As used herein "eluted proteins do not substantially exist" refers to the state where the theoretical value (protein weight/decellularized cell weight) is 0.5mg/mg or less, preferably 0.2mg/mg or less, more preferably 0.1mg/mg or less, as determined by preparing a protein extract from the decellularized tissue by known methods, and detecting the protein by means of a general quantification method such as Bio-Rad Protein Assay and the like.

In another embodiment, whether or not a radical reaction is undergone may be confirmed by verifying whether or not at least a portion, preferably substantially entire portion of extracellular matrix components have been crosslinked. Accordingly, in the present embodiment, the present invention provides a decellularized tissue wherein extracellular matrices have been crosslinked for at least a portion by means of covalent bonding. Preferably, the extracellular matrices include but are not limited to, e.g. , collagen, elastin, laminin, fibronectin, tenascin, glycosaminoglycan, and proteoglycan and the like. More preferably, the crosslinkage is formed between two or more amino acids having no free amino or carboxyl groups on the side chain thereof. In this case, preferably, crosslinkage is formed by the groups other than carboxyl, hydroxyl and amino groups (for example, sulfhydryl group).

It is known that extracellular matrix-derived proteins such as collagen, elastin, laminin, fibronectin and tenascin and the like, and extracellular matrix-derived polysaccharide/glycoproteins such as glycosaminoglycan (hyalulonic acid, chondroitin sulfate and heparan sulfate and the like) and proteoglycan and the like are subjected to gamma-ray irradiation and then crosslinking reactions and degradation reactions occur in a mixed state.

In the present invention, when extracellular matrix-derived protein was subjected to gamma-ray irradiation in the presence of oxygen and water, it appeared that crosslinking reactions easily occurred. Furthermore, even if irradiated samples are subjected to polyacrylamide gel electrophoresis (SDS-PAGE), the samples do not enter the polyacrylamide gel as they would normally before irirradiation. Accordingly, it is revealed that eluted proteins did not sustantially exist.

Furthermore, there are a couple of reports in which amino acid residues are involved in the crosslinkage reaction of a protein by means of gamma-ray irradiation. It appears that the specificity is lower than in the case of chemical crosslinkage.

In the case of chemical crosslinking, for example, glutaraldehyde crosslinking crosslinks amino acids having a free amino group ($-NH_2$) in the side chain thereof, and thus lysine and arginine and the like may necessarily be the site of the crosslink. Amongst other chemical crosslinking, there are those which mainly target carboxyl groups. There are additionally other well known chemical crosslinking chemicals. For example, a variety of crosslinking patterns of collagen are reported in Nimni ME et al., J. Biomed. Matr. Res. 21, 741-771 (1987). and in addition, there are a number of other known crosslinking patterns.

On the other hand, in addition to reactions similar to the above, a variety of crosslinkage reactions caused by gamma-ray irradiation are known to occur. Further , it appears that a variety of amino acids are subjected to crosslinkage points. J.H.Bowesetal.,Irradiation Research 16,211-223(1962) has reported data wherein a sample of washed and removed aqueous components from bovine dermis was subjected to gamma irradiation in the presence of oxygen and nitrogen in both dry and wet states, and acid was used to quantify the change of amino acids after the degradation of the entire protein. According to the results, there are some amino acids such as glycine and valine, which do not substantially reduce, but there are a number of amino acids such as those other than those, which appear to slightly reduce, and the extent of reduction varies. This suggests that crosslinkage between proteins by means of gamma-ray irradiation have occurred to a variety of amino acids in a relatively random manner.

That is, in reference only to the extracellular matrix derived proteins, crosslinkage caused by gamma-ray irradiation and chemical crosslinkage can be distinguished by determining whether or not crosslinkage has occurred between two or more amino acids having free amino groups or carboxyl groups in their side chains thereof. That is, if the crosslinkage has occurred only between two or more amino acids having free amino group or carboxyl groups in their side chains thereof, then the crosslinkage is due to chemical crosslinkage, whereas if crosslinkage has occurred between amino acids other than those, then it can be said that the crosslinkage has occurred due to gamma-ray irradiation.

With respect to polysaccharides, a variety of chemical crosslinkage (bis-epoxide and divinyl sulfone crosslinkage, intramolecular esterification, glutaraldehyde crosslinkage, carbodiimide crosslinkage and hydrazide crosslinkage and the like) target carboxyl groups, hydroxyl groups or amino groups (Laurent,T.C.et al,,Acta Chem.Scand, 18, 274-275, (1964); Kuo,JW.et al., Bioconjug Chem. (4): 232-4L (1991); Luo Y. et al.,J Control Release, 69(1); 169-84. (2000)), therefore, the distinction can be made by observing whether or not crosslinkage has occurred between groups other than carboxyl groups, hydroxyl groups or amino groups (for example, the groups comprise groups selected from the group consisting of sulfhydryl group, aldehyde group, carbonyl group, sulfo group and nitro group), or whether or not the crosslinkage has occurred between a linkage selected from the group consisting of carbon-carbon linkage, ether linkage and ester linkage. In particular, intra-carbon linkage or ester linkage forming the backbone thereof and the like are cleaved to form a new linkage and thereby form a crosslinkage, which may be a characteristic of the present invention.

(Examples of groups)

| -SH group: | sulfhydryl group |
| -CHO: | aldehyde group |
| $C=O$: | carbonyl group |
| -SO$_3$H: | sulfo group |
| -NO$_2$: | nitro group |

(Examples of linkage)

| -C-C-: | CC linkage (carbon-carbon linkage) |
| -O-: | ether linkage |
| -(C=O)O-: | ester linkage |

Furthermore, the above description is not limited to gamma-ray irradiation, but can be extended to radical reactions in general.

In a preferable embodiment, the decellularized tissue of the present invention is characterized in that A) the cell residual rate of the tissue is less than the level at which an immune reaction is elicited in an organism; and B) the tissue is not damaged to such an extent that hinders the tissue from exhibiting a function which was possessed by the tissue when the tissue was not decellularized. Cells remaining in the tissue is a cause of calcification, and in addition, in the case of transplanting a tissue from other than self, there is a possiblity of raising undesired immunological reaction by the remaining cells. Therefore, in the decellularized tissue of the present invention, it is necessary to lower the level of remaining cells to lower than that which raises immunological reaction *in vivo.* Therefore, it is preferable to remove cells as much as possible. The treatment by radical reaction according to the present invention significantly promoted the decellularization ratio. When no radical reaction is conducted, the cell survival ratio has been reduced to as low as 5 %, whereas when a radical reaction is conducted, the cell residual rate is reduced to less than 5 %, preferably less than 4 %, still preferably less than 1 %, most preferably substantially no cells. Even though the cell residual rate is reduced, no significant adverse effects such as tissue strength reduction, reduction in biocompatibility and the like have been observed. Such effects would not have been expected from the prior art technology.

The decellularized tissue of the present invention is used in transplantation therapy, and thus it is preferable that the tissue or organ is not damaged such that the function possessed thereby prior to the treatment (decellularization). Such effects are not substantially damaged by the radical reaction according to the present invention. The presence or absence of such hindrances can be determined by confirming that the extracellular matrix of the tissue is not substantially degenerated. Therefore, the tissue of the present invention may be characterized in that the extracellular matrix remains substantially. The presence of extracellular matrices can be confirmed by staining using a marker specific thereto. As described above, the tissue of the present invention has excellent physical strength, pressure-resistant property, and the like and therefore is preferably used as a scaffold for recellularization (or cell replacement) in a recipient. Conventionally, there is a method for reducing a cell residual rate in tissue (Koide A., Hayashi T., eds (2000); "Basic and Clinical Studies of Extracellular Matrix (ECM); Aichi Shuppan Co., Ltd., Tokyo, Japan CAN:133.333569). However, when a conventional method is used to reduce a cell residual rate to 30% or less, particularly less than 5%, tissue is damaged to such an extent that the tissue cannot withstand organ implantation. The present invention achieves a significant effect in that cell residual rate can be reduced while tissue or an organ is not damaged to such an extent that hinders the tissue or organ from exhibiting a normal function which was possessed by the tissue or organ before treatment (decellularization). The present invention can be used with or without cells.

In order to avoid such toxic actions as described hereinabove, in an embodiment, the cell residual rate of the decellularized tissue of the present invention is usually about 30 % or less, typically about 20 % or less, preferably about 15 % or less, more preferably about 10 % or less, and still more preferably about 5 % or less, yet more preferably about 4 % or less, and still yet more preferably about 3 % or less, and most preferably about 1 % or less, and still more preferably there is substantially no cell survival. The decellularized tissue of the present invention is characterized in that no matter how low the cell residual rate, the tissue damage rate is low such that it is still clinically viable. No matter how the cell residual rate is reduced, if the functions originally possessed by the tissue (as in the native state; in particular function of retaining organs) are not maintained, clinical application is not possible. Accordingly, it is advantageous to retain the cell residual rate as described above, and have as low a tissue damage rate thereof as possible. That is, it is necessary to retain as low a tissue damage rate as possible such that clinical application is possible.

The smaller the tissue damage rate which permits tissue to be used in clinical applications, the more preferable the tissue of the present invention is. The tissue damage rate may be typically about 50% or less, representatively about 40% or less, preferably about 30% or less, more preferably about 25% or less, still more preferably about 20% or less, still even more preferably about 15% or less, still even more preferably about 10% or less, and most preferably about 5% or less. Even though the tissue damage rate is decreased in this manner, it is not possible to use tissue or an organ which is damaged to such an extent that the tissue or organ cannot exhibit an originally possessed function thereof. Therefore, in order to achieve an objective of the present invention, it is preferable that the tissue of the present invention is not damaged to such an extent that the tissue is hindered from exhibiting an originally possessed function thereof. It is possible to evaluate the tissue damage rate to determine whether or not the tissue can exhibit an originally possessed function thereof. The tissue damage rate can be evaluated by the above-described method.

Alternatively, the decellularized tissue of the present invention can be determined by evaluating the survival rate of extracellular matrices. The survival rate of extracellular matrices may be, for example, about 50% or more, preferably about 70% or more, more preferably about 80% or more, and even more preferably about 90% or more.

In one embodiment, the radical reaction used in the production of the decellularized tissue of the present invention includes exposure to a free radical. Exposure to a free radical may be achieved by using either, or both, physical means (e.g., gamma-ray irradiation, ultraviolet irradiation, ultrasonication exposure, electron beam irradiation and x-ray irradiation and the like), or chemical means (exposure to a free radical source (for example, active oxygen species such as hydrogen peroxydase, ozone and the like)). Exposure to a free radical facilitates a radical reaction. Such a radical reaction is preferably conducted to an extent such that extracellular matrices do not degrade.

In one embodiment, radical reaction used in the present invention is preferably gamma-ray irradiation. Although not wishing to be bound to any theories, gamma-ray irradiation is superior in permeability against matter or a substances, and thus it is appropriate to promote decellularization in an efficient manner and to depth while maintaining tissue strength.

In one embodiment, the exposure dose of gamma irradiation used in the present invention is usually between 10 kGy and 250 kGy. Although not wishing to be bound to any theories, this is because degradation may be caused to extracellular matrices which is desired to remain. However, exposure dosage which does not cause any substantial degradation of components necessary for maintaining tissue strength such as extracellular matrices and the like, irradiation exceeding the upper limit may be used. Although not wishing to be bound to any theories, exposure dose of 10 kGy or less may also may be used. Although it is believed that such exposure time may be too long, however, as long as promotion in decellularization is significantly recognized, exposure dose of 10 kGy or less may also be used, and thus it is understood by those skilled in the art that these are within the scope of the present invention.

Preferably, exposure dose of gamma-ray irradiation or the like is advantageously of 100kGy or less. Although not wishing to be bound to any theories, the range of 100kGy or less has observed almost no significant reduction in tissue strength. The preferable lower limit used is an exposure dose of 40kGy or more.

More preferably, exposure dosage is advantageously 50-80 kGy. Although not wishing to be bound to any theory, the combination of cleavage of a biomolecule such as genetic DNA or the like by means of gamma-ray irradiation, and extraction operation by means of PEG promotes decellularization, while not giving significant damages to extracellular matrix proteins which maintain mechanical strength of the tissue.

In one embodiment, a period of time for irradiation of gamma-ray irradiation used in the present invention varies depending on the total irradiation dose, and usually ranges from about 0.5 hour to about 10 days, and preferably about one hour to about two days for advantageous embodiments. More preferably, it is advantageous that the period is 3-8 hours. Although not wishing to be bound to any theories, the longer the irradiation period becomes, the more the decellularization promotes. However, conventional decellularization results in cleavage or crosslinkage of extracellular matrix proteins such as collagen, elastin or the like, which have rich properties in elasticity. In addition, they becomes weak in kinetic deformation such as bending or tension, and thus worsen the properties as transplantation tissue. Accordingly, the decellularization partially depends on the total irradiation dose, and thus a strong gamma-ray dose may be used to complete reactions in about several hours.

In a preferable embodiment, radical reactions used in the present invention (such as gamma-ray irradiation) are performed under an atmosphere selected from the group consisting of in vacuo, in oxygen, in nitrogen, in the atmosphere or air, in water, in an amphipathic molecule solution and a combination thereof.

Therefore, in a preferred embodiment, the decellularized tissue has 1) a cell residual rate of about 30% or less; and 2) a tissue damage rate of about 30% or less.

In a more preferred embodiment, the decellularized tissue has 1) a cell residual rate of about 5% or less. In a preferable embodiment, the tissue damage rate of the tissue is less than about 15%. In amore preferable embodiment, the tissue damage rate of the decellularized tissue is less than about 4 %, and in a most preferable embodiment, the tissue damage rate of the tissue is less than about 1%.

In another preferable embodiment, the tissue damage rate of the tissue of the present invention is less than about 15%. In a more preferable embodiment, the tissue damage rate of the decellularized tissue is less than about 5 %. These preferable embodiments are only exemplary embodiments of the present invention, and thus do not limit the scope of the claims of the present invention. Accordingly, it is understood that the decellularized tissue of the present invention encompass any tissue within the scope of the present invention as long as the tissue has a tissue strength which can be clinically applied.

In one embodiment, the decellularized tissue of the present invention has such a tissue strength that permits clinical applications. A sufficiently high level of tissue strength is an important characteristic, particularly when membrane-like tissue is used in clinical applications. Tissue strength can be generally determined by measuring tensile strength (e.g., breaking strength, stiffness, Young's modulus, etc.). In a certain embodiment, the decellularized tissue of the present invention may have a tissue strength which is at least about 75% or more of the strength which was possessed by the tissue before treatment, preferably about 80% or more, more preferably about 85% or more, still more preferably about 90% or more, or alternatively may have a tissue strength greater than or equal to that which was possessed by the untreated tissue (for example, tissue strength such as 110% or more, 120% or more, 150% or more, 200% or more). As used herein, the term "tissue strength of untreated tissue" refers to tissue strength which was possessed by tissue before treatment (e.g., treatment with a 1,2-epoxide polymer of the present invention such as PEG and a radical reaction; as in the native state). Sufficiently strong tissue strength is a preferable property for applying it to a tissue other than membranous tissue (for example, tubular tissue).

In the case of luminal tissue, tissue strength can be represented by a $\beta$ value. A method for calculating a $\beta$ value is heretofore described in detail and is also illustrated in examples below. In a certain embodiment, the decellularized tissue of the present invention has a tissue strength having a $\beta$ value of about 15 or more, preferably a $\beta$ value of about 18 or more, more preferably a $\beta$ value of about 20 or more, and still more preferably a $\beta$ value of about 22 or more. In another embodiment, the decellularized tissue of the present invention may have a $\beta$ value which is at least about 75% or more of that which was possessed by the tissue before treatment, preferably about 80% or more, more preferably about 85% or more, still more preferably about 90% or more, or alternatively may have a $\beta$ value greater than or equal to that which was possessed by the untreated tissue (originally possessed $\beta$ value). As used herein, the term "characteristic (or properties) of untreated tissue" refers to a characteristic which was possessed by tissue before treatment (e.g., treatment with a 1,2-epoxide polymer and a radical reaction of the present invention).

The decellularized tissue of the present invention may be tissue which is derived from any part of the body if the tissue is intended to be used in clinical applications. In a certain embodiment, the decellularized tissue of the present invention may require physical structure. In order to maintain physical structure, only structure, such as cytoskeleton and the like, is required. Intracellular components, such as plasma components and the like, are not necessarily required. Also, optionally required cells may be additionally provided to decellularized tissue or may be internally supplied from a host to which the tissue is implanted. In a certain embodiment, the decellularized tissue of the present invention may be derived from an organ selected from blood vessels, blood vessel-like tissue, cardiac valves, pericardia, dura mater, corneas, and bones. In another embodiment, the decellularized tissue of the present invention may be derived from an organ selected from cardiovascular tissue (e.g., blood vessels, blood vessel-like tissue, cardiac valves, and pericardia).

The decellularized tissue of the present invention may be tissue derived from any organism if the tissue is suitable for an intended clinical application. Therefore, the tissue of the present invention maybe derived from any organism (e.g., vertebrates and invertebrates). When intended to be applied to a human, vertebrate-derived tissue is preferablyused, and more preferably tissue derived from a mammal (e.g., a primate, a rodent, or the like) is used. When intended to be applied to a human, primate-derived tissue is more preferably used. When intended to be applied to a human, porcine-derived tissue is more preferably used. This is because porcine-derived tissue has a size similar to that of a human. When intended to be applied to a human, human-derived tissue is most preferably used. The decellularized tissue or tissue graft of the present invention preferably has a size similar to that of a human and a physical characteristic similar to that of a human, when the tissue or tissue graft is derived from organisms other than a human (e.g., swine).

In a preferable embodiment, the decellularized tissue of the present invention is substantially removed of viruses. In the present invention, viruses to be removed include, e.g., retroviruses and herpes viruses and the like. Preferably, viruses to be removed include porcine endogenous retrovirus (PERV). More preferably, the PERV is removed such that no significant infection is observed to a human cell. In particular, PERV has been of concern with respect to possible infection of a human, and as such, it is desirable that transplantation graft from a swine should be removed of the PERV. However, in the conventional technologies, it is difficult to remove the PERV. The present invention thus provides a decellularized tissue which could not be provided by the prior art technologies, in that viruses can be removed from the decellularized tissue.

In the present invention, the decellularized tissue of the present invention may be applied to any part of the body of an organism. Therefore, the decellularized tissue may be applied to a part of the body from which the decellularized tissue was derived or other parts of the body. As shown in the Examples, it was demonstrated that the desired effect (e.g., regeneration, self organization) of the present invention can be achieved for any part of the body to which the decellularized tissue of the present invention is applied, whether or not the part is a site from which the tissue was derived. Therefore, the present invention has a considerably advantageous utility such that the present invention can in principle be applied to any implantation operation and regeneration operation. Examples of parts to which the decellularized tissue of the present invention is applied, include, but are not limited to, skin, blood vessel, cornea, kidney, heart, liver, umbilical cord, intestine, nerve, lung, placenta, pancreas, brain, peripheral limb, retina, valve, epithelial tissue, connective tissue, muscular tissue, nervous tissue, and the like.

In another aspect, the present invention provides a tissue graft containing the decellularized tissue of the present invention. In this tissue graft, recipient-derived cells are disseminated and cultured in the above-described decellularized tissue so that desired tissue structure is formed. The tissue graft of the present invention may be intended to be implanted into any tissue in the body as long as the tissue graft is of tissue intended for a clinical application. In a certain embodiment, the tissue graft of the present invention may require physical structure. In order to maintain physical structure, recipient-derived cells may be provided to the above-described decellularized tissue before implantation. The recipient-derived cells may be internally supplied from a host to which the tissue graft is implanted. In a certain embodiment, the tissue graft of the present invention may be derived from an organ selected from blood vessels, blood vessel-like tissue, cardiac valves, pericardia, dura mater, corneas, and bones. In another embodiment, the tissue graft of the present invention may be derived from an organ selected from cardiovascular tissue (e.g., blood vessels, blood vessel-like tissue, cardiac valves, and pericardia).

The tissue graft of the present invention may contain tissue derived from any organism if the tissue is suitable for an intended clinical application. Therefore, the tissue graft of the present invention may contain tissue derived from any organism (e.g., vertebrates and invertebrates). When intended to be applied to a human, vertebrate-derived tissue is preferably used for the tissue graft of the present invention, and more preferably tissue derived from a mammal (e.g., a primate, a rodent, or the like) is used for the tissue graft of the present invention. When intended to be applied to a human, primate-derived tissue is more preferably used for the tissue graft of the present invention. When intended to be applied to a human, porcine-derived tissue is more preferably used for the tissue graft of the present invention. This is because porcine-derived tissue has a size similar to that of a human. When intended to be applied to a human, human-derived tissue is most preferably used.

Recipient-derived cells used in the tissue graft of the present invention may be any cells that are suitable for clinical applications . Therefore, the cell may be selected from the group consisting of vascular endothelial cells, smooth muscle cells, fibroblasts, blood cells, and precursor cells and somatic stem cells capable of differentiating into those cells. Preferably, the cell may be a cell capable of exhibiting a desired function at a site at which the cell is implanted.

In the present invention, the tissue graft of the present invention may be applied to any part of the body of an organism. Therefore, the tissue graft may be applied to a part of the body from which the tissue graft was derived or other parts of the body. As shown in the Examples, it was demonstrated that the desired ef f ect (e.g., regeneration, self organization) of the present invention can be achieved for any part of the body to which the tissue graft of the present invention is applied, no matter whether or not the part is a site from which the tissue was derived. Therefore, the present invention has a considerably advantageous utility such that the present invention can in principle be applied to any implantation operation and regeneration operation. Examples of parts to which the tissue graft of the present invention is applied, include, but are not limited to, skin, blood vessel, cornea, kidney, heart, liver, umbilical cord, intestine, nerve, lung, placenta, pancreas, brain, peripheral limb, retina, valve, epithelial tissue, connective tissue, muscular tissue, nervous tissue, and the like.

In another aspect, the present invention provides a membrane-like tissue graft. This membrane-like tissue graft comprises A) the decellularized tissue of the present invention. Here, recipient-derived cells are disseminated and cultured in the decellularized tissue so that desired tissue structure is formed. Cells are not necessarily included therein.

Preferably, it is advantageous not to have a cell, because no immunological rejection reaction will occur.

In another aspect, the present invention provides a method of producing decellularized tissue. This method comprises the steps of A) providing tissue; B) immersing the tissue in a solution containing a non-micellar amphipathic molecule (e.g., a 1,2-epoxide polymer); and C) subjecting the tissue to a radical reaction. Any amphipathic molecule which is non-micellar can be used. As the amphipathic molecule, any polymer which can remove plasma components can be used. The non-micellar amphipathic molecule used in the present invention is preferably a 1,2-epoxide polymer, and more preferably polyethylene glycol (PEG), though any non-micellar amphipathic molecule can be used. Amphipathic molecules, conventionally called surfactants, can be used. In these situations, it is preferable to confirm that an amphipathic molecule is non-micellar before using a solution containing the amphipathic molecule. The confirmation of the non-micellar state can be performed by using a well-known technique in the art. For example, such a technique includes, but is not limited to, visual inspection, absorbance measurement, and the like.

Radical reactions may be performed under any conditions as described hereinabove. Radical reactions may be simultaneously conducted with the step of immersing the tissue into a solution comprising an amphipathic molecule or thereafter. It appears preferable to conduct the radical reaction after the immersion step. Although not wishing to be bound to any theories, decellularization is first performed and then radical reaction is performed under conditions other than decellularization such as in the atmosphere which appears to result in enhancement of decellularization efficiency and/or retainment of tissue strength. Radical reactions are preferably performed so as to maintain the tissue strength of the decellularized tissue to the same level as in the decellularized tissue not subjected to radical reactions, while still significantly progressing the decellularization. Such conditions may be appropriately selected by those skilled in the art based on the description of the present specification.

Radical reactions are preferably performed in the atmosphere. Although not wishing to be bound to any theories, decellularization is first performed and then radical reaction is performed in the atmosphere which appears to result in enhancement of decellularization efficiency and/or retainment of tissue strength.

The present invention also provides treatment of a decellularized tissue subjected to decellularization treatment other than those with a solution comprising an amphipathic molecule which is not in the state of micelles, such as when treated with SDS. In such a case, the decellularization enhancement method of the present invention comprises a step of providing a decellularization tissue, and a step of performing a radical reaction to the tissue.

In the radical reaction of the present invention, PEGs having any molecular weight can be used to affect the decellularization enhancement effects. In one preferred embodiment, the average molecular weight of PEG is between 200 and 6000. PEG used in the present invention may have various average molecular weights. By changing treatment time (immersion time) depending on the average molecular weight, a desired effect (e.g., decellularization) can be achieved. In one preferred embodiment, the average molecular weight of PEG used in the present invention is between 1000 and 2000. In another preferred embodiment, the average molecular weight of PEG used in the present invention is between 1500 and 2000. In another embodiment, the average molecular weight of PEG used in the present invention is smaller than or equal to 1000. Alternatively, in one preferable embodiment, PEG having average molecular weight of 1000 is used.

Although not wishing to be bound to any theory, in general, as the average molecular weight of PEG used is increased, the treatment time has to be decreased. This is because as the average molecular weight of PEG used is increased, the effect of extracting intracellular components increases. Therefore, in one embodiment, tissue may be immersed in a 1,2-epoxide polymer-containing solution for 30 min to 60 min. A minimum time required in the immersing step varies depending on the amphipathic molecule used (e.g., a 1,2-epoxide polymer, such as PEG), but can be determined by those skilled in the art without undue experimentation. Therefore, the step of immersing tissue in a solution containing an amphipathic molecule, such as a 1,2-epoxide polymer, may be performed for less than 30 min. Alternatively, the immersing step using a solution containing an amphipathic molecule, such as a 1,2-epoxide polymer, may be performed for more than 60 min. A minimum time can be obtained as follows: various treatment times are provided in control experiments; at each time point, the state of treated tissue is determined by measuring the tissue damage rate, the cell residual rate, or the like of the tissue by methods as described herein; and based on the result, it is determined whether or not the treated tissue has an appropriate characteristic. In the method of the present invention, the step of immersing tissue in the solution may be performed under any condition and may be performed typically at a temperature of between 0°C and 42°C, at room temperature (between about 15°C and about 25°C), or at 37°C. The present step may be conducted at higher than 37°C.

In the method of the present invention, the 1, 2-epoxide polymer (e.g., PEG) may be present in the solution at any concentration and may be preferably present at a concentration of 1g/ml or more. Such a substance is expected to act as a radical scavenger for radical reactions (in particular, a physical radical source such as gamma-ray irradiation). Alternatively, when considering efficiency of radical reactions, concentrations of PEG solution may be at any concentration, but is preferably between about 60 % and about 100%, for example, those having about 80% may be used but are not limited thereto.

Note that when PEG (molecular weight: 1000) is used as a material reagent, it is solid at room temperature and therefore may be dissolved in water. In the method of the present invention, the 1,2-epoxide polymer (e.g., PEG) may be dissolved in any solvent and may be preferably dissolved in an aqueous medium, more preferably physiological saline, PBS (phosphate buffered saline), or other solutions containing salts.. Such a solution may be any solution in which an amphipathic molecule used in the present invention is non-micellar at a concentration which is usually used for the solution. The solution having a non-micellar amphipathic molecule (e.g., a 1,2-epoxide polymer) is preferably sterilized. Anon-micellaramphipathicmolecule (e.g., a 1,2-epoxide polymer) used in the present invention is preferably biocompatible. Such a biocompatible, non-micellar amphipathic molecule (e.g., a 1,2-epoxide polymer) includes, but is not limited to, polymers which are biocompatible and are of medicament grade, for example, PEG, segmented poly-urethane, silicone, MMA ($\alpha$-methylmethacrylate (contact lens material), etc.), and PTFE (polytetrafluoroethylene) (e.g., Teflon (registered trademark), Dacron, etc.). Such a biocompatible 1,2-epoxide polymer is described in, for example, Japanese Pharmacopoeia (or counterparts in other countries) or may beapolymer approved by the Health, Labor and Welfare Ministry (or counterparts in other countries).

Preferably, in the method of the present invention, the step of immersing tissue in a solution containing a 1,2-epoxide polymer may be performed concomitantly or before treatment with DNase (e.g., DNaseI).

Preferably, the method of the present invention further comprises washing the tissue immersed in the solution. The washing step can be performed using any liquid which is physiologically suitable (e.g., physiological saline, PBS (phosphate buffered saline), etc.). In a preferred embodiment, the washing step in the present invention is performed with PBS. The wash solution is preferably sterilized. The washing step may be performed under any condition and may be performed typically at a temperature of between 0°C and 42°C, at room temperature (about 15°C and about 25°C), or at 37°C. The washing step may be performed at a temperature of more than 37°C. In the method of the present invention, the washing step may be performed for any period of time as long as the solution containing an amphipathic molecule, such as a 1,2-epoxide polymer, can be sufficiently removed and may be usually performed for 0.5 days to 5 days. Preferably, in the washing step, a wash solution (e.g., PBS) may be changed several times.

Tissue used in the method of the present invention may be tissue derived from any organism if the tissue is suitable for an intended clinical application. Therefore, tissue used in the method of the present invention may be derived from any organism (e.g., vertebrates and invertebrates). When intended to be applied to a human, vertebrate-derived tissue is preferably used as tissue used in the method of the present invention, and more preferably tissue derived from a mammal (e.g., a primate, a rodent, or the like) is used as tissue used in the method of the present invention. When intended to be applied to a human, primate-derived tissue is more preferably used as tissue used in the method of the present invention. When intended to be applied to a human, porcine-derived tissue is more preferably used.. This is because porcine-derived tissue has a size similar to that of a human. When intended to be applied to a human, human-derived tissue is most preferably used.

Tissue used in the method of the present invention may be any tissue in the body as long as the tissue is intended to be used in clinical applications. In a certain embodiment, the tissue used in the method of the present invention may require physical structure or physical properties. In order to maintain physical structure or physical properties, only structure, such as extracellular matrices and the like, are required. While intracellular components, such as plasma components, cell membrane components, and the like, are not necessarily required. Also, optionally required cells may be additionally provided to decellularized tissue or may be internally supplied from a host to which the tissue is implanted. In a certain embodiment, tissue used in the method of the present invention may be derived from an organ selected from blood vessels, blood vessel-like tissue, cardiac valves, pericardia, dura mater, corneas, and bones. In another embodiment, the decellularized tissue of the present invention may be derived from an organ selected from cardiovascular tissue (e.g., blood vessels, blood vessel-like tissue, cardiac valves, and pericardia).

In a preferred embodiment, the method of the present invention may further comprise performing chemical treatment. Here, the chemical treatment may be one other than the step of immersing tissue in a solution containing a non-micellar amphipathic molecule (e.g., a 1,2-epoxide polymer, such as polyethylene glycol) in the method of the present invention. Alternatively, the chemical treatment may be the same as the step of immersing tissue in a solution containing a non-micellar amphipathic molecule (e.g., a 1,2-epoxide polymer, such as polyethylene glycol) in the method of the present invention (i.e., repeating the immersing step). Therefore, in the method of the present invention, for example, when the chemical treatment is performed in addition to the step of immersing tissue in a solution containing polyethylene glycol having an average molecular weight of 1000 to 2000, the chemical treatment comprises the step of immersing tissue in a solution other than the solution containing polyethylene glycol having an average molecular weight of 1000 to 2000 (including, but not limited to, for example, a solution containing DNase (e.g., DNaseI), a glutaraldehyde-containing solution, a solution containing polyethylene glycol having another average molecular weight, and a solution containing another non-micellar amphipathic molecule (e.g., a 1,2-epoxide polymer) in the present invention, and the like). Alternatively, for example, when the chemical treatment is performed in addition to the step of immersing tissue in the solution containing polyethylene glycol having an average molecular weight of 1000 to 2000, the chemical treatment may comprise repeating the step of immersing tissue in the solution containing polyethylene glycol having an average molecular weight of 1000 to 2000.

In one embodiment, the above-described chemical treatment may be treatment with a difunctional molecular crosslinking agent (e.g., glutaraldehyde or a derivative thereof). The treatment with a difunctional molecular crosslinking agent is intended to chemically crosslink a protein component contained in ECM or tissue cells to increase physical strength. Therefore, if this purpose can be achieved, any difunctional molecular crosslinking agent can be used. Among such difunctional molecular crosslinking agents, some agents which can be actually used for fixation of tissue (valve graft) include, but are not limited to, for example, cyanimide, 1-ethyl-3-(3-dimethylaminopropyl)carbodiimidehydrochloride (EDC), epoxy(poly(glycidylether)) or photo crosslinking agent PhotoMix™ (Sulzer Carbomedics Co., Ltd.) (see, Biomaterials (2000) 21:2215-2231).

In another embodiment, the chemical treatment may comprise treatment with DNase, such as DNaseI. DNA components undesirable for implantation can be removed by means of treatment with DNase. Therefore, treatment with DNase is preferable. Such DNase may be any DNase and is preferably DNaseI. With the DNase treatment, DNA, which is a charged polymer substance, can be removed. DNA has the possibility of eliciting an immune reaction. Therefore, by removing DNA, an advantage can be provided.

Treatment with a nuclease (e.g., DNase) may be conducted under many conditions, and may combine a number of conditions such as a pressurized condition, agitation condition and the like. Concentrations used are for example, 1U/ml-100U/ml and the like, and examples include, 50-75 U/ml but are not limited thereto. Nuclease treatment may be conducted for about 0.5 to five days for example, and amongst the first several hours (for example, 1-24 hours) may be conducted under a pressurized condition (for example, 100-1000kPa, for example, 500 kPa). Thereafter, agitation (for example, 100-500 RPM, preferably, 100-150RPM) may also be conducted. Before and after the nuclease treatment, washing solution as described above may preferably be used for preservaton.

In a preferable embodiment, the method for producing a decellularized tissue of the present invention includes a further step of seeding cells, but is not limited thereto. Cells to be seeded may be appropriately selected by those skilled in the art depending on the conditions as described hereinabove.

The present invention also provides decellularized tissue obtained by the decellularization method of the present invention. This decellularized tissue may preferably have the above-described cell residual rate and/or tissue damage rate and/or tissue strength. Before the decellularization method of the present invention is provided, it was not possible to provide decellularized tissue having the above described cell residual rate and/or tissue damage rate and/or tissue strength. Thus, the decellularization method of the present invention has an unexpected advantage of providing decellularized tissue having a characteristic which could not be provided by conventional methods.

In another aspect, the present invention provides a method of regenerating tissue. This method comprises the steps of A) providing decellularized tissue subjected to a radical reaction, into an organism; and B) incubating the tissue within the organism for a time sufficient for the tissue to regenerate. Optionally, the present inventive method may further comprises the step of providing a physiologically active subtance inducing differentiation of the cell and/or providing the decellularized tissue with a cell or it is not necessary to provide the decellularized tissue with a cell. Preferably, physiologically active substances necessary for maintenance or differentiation of a desired tissue are used. Physiologically active substance may be derived from the body or externally derived. In the case of those derived from the body, it is understood that no substantial operation is necessary for exposure of an appropriate physiologically active substance by lapsing a certain period of time after transplantation. Physiologically active substances include, but are not limited to, for example, HGF, VEGF, FGF, IGF, PDGF and EGF.

Preferably, the cell may be a blood vessel cell or a blood vessel-like cell. More preferably, the cell may be derived from a recipient . Preferably, the tissue may be selected from the group consisting of blood vessels, blood vessel-like tissue, cardiac valves, pericardia, dura mater, corneas, and bones. In a preferred embodiment, the tissue and the cell may be derived from the same host. In another embodiment, the tissue and the cell may be derived from homologous hosts. In another embodiment, the tissue and the cell may be derived from heterologous hosts. When the recipient and the cell are homologous or heterologous, a rejection reaction is expected. Therefore, the recipient and the cell are preferably derived from the same host. When a rejection reaction does raise a problem, the recipient and the cell may be homologous or heterologous. If the cell which elicits a rejection reaction is optionally treated to overcome the rejection reaction, the cell can be used. A method for overcoming a rejection reaction is known in the art and is described in, for example, "Shin-Geka-Taikei, Shinzo-Ishoku Hai-Ishoku Gijyutsuteki, Rinriteki-Seibi-kara-Jissi-nimukete [Heart Transplant Lung Transplant - From Technical and Ethical Development to Practice]" (3rd Version). Such a method includes, fox example, use of an immunosuppressant or a steroid, and the like. Immunosuppressants for preventing rejection reactions currently include "cyclosporine" (SANDIMMUNE/NEORAL), "tacrolimus" (PROGRAF), "azathioprine" (IMURAN), "steroid hormone" (prednine, methylprednine), "T-cell antibodies" (OKT3, ATG, etc.). A method which is used for preventive immunosuppressive therapy in a number of facilities in the world is three-drug therapy using "cyclosporine, azathioprine, and steroid hormone". An immunosuppressant is preferably, but not necessarily, administered concomitantly with a medicament of the present invention. Therefore, an immunosuppressant may be administered before or after a regeneration method of the present invention as long as an immunosuppression effect can be achieved.

In another aspect, the present invention provides a method of producing a tissue graft. This method comprises the steps of A) providing the decellularized tissue subjected to a radical reaction of the present invention into an organism; B) allowing self cells in the organism to infiltrate the decellularized tissue; and C) incubating the tissue for a sufficient time to differentiate the self cells. In this case, the decellularized tissue of the present invention may or may not have additional cells. The additional cells may be autologous, homologous, or heterologous. Preferably, the cell may be a blood vessel cell or a blood vessel-like cell. More preferably, the cell may be derived from the recipient . Preferably, the tissue may be selected from the group consisting of blood vessels, blood vessel-like tissue, cardiac valves, pericardia, dura mater, corneas, and bones. In a preferred embodiment, the tissue and the cell may be derived from the same host. In another embodiment, the tissue and the cell may be derived from homologous hosts. In another embodiment, the tissue and the cell may be derived from heterologous hosts. When the recipient and the cell are homologous or heterologous, a rejection reaction is expected. Therefore, the recipient and the cell are preferably derived from the same host. When a rejection reaction does not raise a problem, the recipient and the cell may be homologous or heterologous. If the cell which elicits a rejection reaction is optionally treated to overcome the rejection reaction, the cell can be used. A method for overcoming a rejection reaction is herein described.

In a preferred embodiment, the method of the tissue graft of the present invention may further comprise D) providing a physiologically active substance capable of inducing differentiation of the above-described cell. Preferably, the physiologically active substance may be a cytokine having hematopoiesis activity. In order to produce a tissue graft, the cytokine may be HGF, VEGF, FGF, or the like. Phtysiologically active substances may be derived from self or foreign source. Physiologically active substances may be derived from self or externally derived. It should be noted that when the physiologically active substance is derived from self, lapsing a certain period of time after transplantation is simply enough for attaining the purpose thereof.

In another aspect, the present invention provides a tissue graft produced by the method of the present invention. Such a tissue graft has a characteristic which is not conventionally obtained with respect to the tissue strength and the decellularization rate.

In another aspect, the present invention provides a method for treating a subject requiring implantation of tissue or an organ or treating a subject atarisk of implantation of tissue or an organ for prophylaxis. This method comprises A) providing the decellularized tissue or tissue graft sujected to a radical reaction of the present invention; and B) implanting the decellularized tissue to the subject. The decellularized tissue optionally contains additional cells. The cell may be autologous or homologous. Preferably, the tissue may be selected from the group consisting of blood vessels,blood vessel-like tissues, cardiac valves, pericardia, dura mater, corneas, and bones. In a preferred embodiment, the tissue may be derived from the subject. In another embodiment, the tissue maybe derived fromahost homologous to the subject. In another embodiment, the tissue may be derived from a host heterologous to the subject. The therapeutic/prophylactic method of the present invention employs tissue which is sufficiently decellularized that a tissue damage rate is reduced to such a level that permits implantation. Therefore, the tissue does not elicit a rejection reaction. In the case that a rejection reaction is elicited by the tissue or non-recipient-derived cells, treatment for overcoming a rejection reaction can be optionally performed. A method for overcoming a rejection reaction is herein described in detail. In one embodiment, tissue used in the treatment or prophylactic method of the present invention may be derived from the subject. In another embodiment, tissue used in the treatment or prophylactic method of the present invention may be derived from any organism (e.g., vertebrates and invertebrates). Preferably, when a human is treated for therapy or prophylaxis, vertebrate-derived tissue may be used. More preferably, when a human is treated for therapy or prophylaxis, tissue derived from a mammal (e.g., a primate, a rodent, etc.) may be used. Still more preferably, when a human is treated for therapy or prophylaxis, primate-derived tissue may be used. In another preferred embodiment, when a human is treated for therapy or prophylaxis, porcine-derived tissue may be used. This is because porcine-derived tissue has a size similar to that of a human. Most preferably, when a human is treated for therapy or prophylaxis, human-derived tissue may be used.

In another aspect, the present invention provides a medicament or pharmaceutical for organ implantation. This medicament or pharmaceutical comprises the decellularized tissue of the present invention or the tissue graft of the present invention. In a certain embodiment, the medicament or pharmaceutical of the present invention comprises tissue derived from an organ selected from blood vessels, blood vessel-like tissue, cardiac valves, pericardia, dura mater, corneas, and bones. In another embodiment, the medicament or pharmaceutical of the present invention may comprise cardiovascular tissue, for example, tissue derived from an organ selected from blood vessels, blood vessel-like tissue, cardiac valves, and pericardia.

The medicament or pharmaceutical of the present invention may comprise tissue derived from any organism as long as the tissue is suitable for an intended clinical application. Preferably, the medicament or pharmaceutical may comprise a material approved by an authority in a country in which the medicament or pharmaceutical is used. Therefore, the medicament or pharmaceutical of the present invention may comprise tissue derived from any organism (e.g., vertebrates and invertebrates). In the medicament or pharmaceutical of the present invention, when intended to apply the medicament or pharmaceutical to a human, preferably, vertebrate-derived tissue is used. More preferably, tissue from a mammal (e.g., a primate, a rodent, etc.) is used. In the medicament or pharmaceutical of the present invention, when intended to apply the medicament or pharmaceutical to a human, still more preferably, primate-derived tissue is used. When the medicament or pharmaceutical of the present invention is intended to be applied to a human, still even more preferably, porcine-derived tissue is used. This is because porcine-derived tissue has a size similar to that of a human. When the medicament or pharmaceutical of the present invention is intended to be applied to a human, most preferably, human-derived tissue is used. Note that when human-derived tissue is used, ethical regulations or problems have to be solved.

The pharmaceutical, medicament, tissue graft and decellularized tissue of the present invention may further comprise biocompatible material. For example, the biocompatible material may comprise at least one selected from the group consisting of silicone, collagen, gelatin, glycolic acid/lactic acid copolymers, ethylene/vinyl acetate copolymers, polyurethane, polyethylene, polytetrafluoroethylene, polypropylene, polyacrylate, and polymethacrylate. Silicone is preferable because of ease of molding. Examples of biodegradable polymers include collagen, gelatin, polymers or copolymers synthesized by non-catalytic dehydrocondensation of at least one selected from $\alpha$-hydroxycarboxylic acids (e.g., glycolic acid, lactic acid, hydroxybutyric acid, etc.), hydroxydicarboxylic acids (e.g., malic acid, etc.), and hydroxytricarboxylic acids (e.g., citric acid, etc.) or a mixture thereof, poly-$\alpha$-cyanoacrylic ester, polyamino acids (e.g., poly-$\gamma$-benzyl-L-glutamic acid, etc.), and polyanhydrides such as maleic anhydride copolymers (e.g., styrene-maleic acid copolymers, etc.), and the like. These copolymers may be any of random, block, and graft copolymers. When $\alpha$-hydroxycarboxylic acids, hydroxydicarboxylic acids, and hydroxytricaxboxylic acids have an optical activity center within the molecule, the molecule can be in any of the D-form, L-form, and DL-form. In a certain embodiment, a glycolic acid/lactic acid copolymer may be used.

The pharmaceutical, medicament, tissue graft, and decellularized tissue of the present invention may further comprise another pharmaceutical agent. Such a pharmaceutical agent may include any pharmaceutical agent known in the field of pharmaceuticals. The medicament, tissue graft, and decellularized tissue of the present invention may comprise two or more other pharmaceutical agents. Such pharmaceutical agents include, for example, those which are described in the up-to-date version of Japanese Pharmacopoeia, US Pharmacopoeia, pharmacopoeias in other countries, and the like. The pharmaceutical agent may preferably have an effect on an organ of an organism. The pharmaceutical agent includes, for example, thrombolytic agents, vasodepressors, and tissue activating agents. The amounts of a physiologically active substance and other pharmaceutical agents and cells contained in the medicament, tissue graft, and decellularized tissue of the present invention can be easily determined by those skilled in the art, considering purposes of use, target diseases (type, severity, etc.), patient's age, weight, sex, case history, and the like.

In another aspect, the present invention relates to use of the decellularized tissue of the present invention or the tissue graft of the present invention for organ implantation and medicament production. In a certain embodiment, regarding the use of the present invention, tissue derived from an organ selected from blood vessels, blood vessel-like tissue, cardiac valves, pericardia, dura mater , corneas, and bones, maybe used. In another embodiment, regarding the use of the present invention, tissue derived from an organ selected from cardiovascular tissue, such as blood vessels, blood vessel-like tissue, cardiac valves, and pericardia, may be used.

Regarding the use of the present invention, tissue derived from any organism may be used as long as the tissue is suitable for an intended clinical application. Preferably, a material approved by an authority in a country in which the medicament is used, is used. Therefore, regarding the use of the present invention, tissue derived from any organism (e.g., vertebrates and invertebrates) may be used. Regarding the use of the present invention, when intended to apply the present invention to a human, preferably, vertebrate-derived tissue is used. More preferably, tissue from a mammal (e.g., a primate, a rodent, etc.) is used. Regarding the use of the present invention, when intended to apply the present invention to a human, still more preferably, primate-derived tissue is used. When the present invention is intended to be applied to a human, still even more preferably, porcine-derived tissue is used. This is because porcine-derived tissue has a size similar to that of a human. When the present invention is intended to be applied to a human, most preferably, human-derived tissue isused. Note that when human-derived tissue is used, ethical regulations or problems have to be solved.

Tissues from swine has a problem of having the alpha-Gal epitope. It has turned out that anti-GAl antibody, a native antigen present in a human, recognizes and crossreacts with the alpha-gal antigen (alpha-gal epitope) largely expressed on the cellular surface of a porcine transplant, which greatly hinders xenograft transplantation adaptation in the body. This alpha-gal epitope is expressed in swine and other mammals, but not in a human. This is because alpha 1,3-galactose transferase gene, which biosynthesize this epitope, was inactivated in the evolutionary process of mammals (Trends in Glycoscience and Glycotechnology Vol. 11 No.62 (Nov. 1999) pp.317-327). The decellularization tissue according to the present invention attains effects of reducing the alpha-Gal epitope and is reduced to the extent that it does not raise an immunological rejection reaction. There has been no report to date with respect to the decellularization tissue which exhibits a reduction in the epitope so as not to raise any immunological rejection reaction. Accordingly, the present invention provides a novel decellularized tissue with particularly reduction in immunological rejection reaction.

Further, porcine tissues include porcine endogenous retrovirus (PERV), which has a high possiblity of infecting a human cell. This is one of the obstructions when trying to transplant a living material from swine into humans.

Porcine endogenous retrovirus (PERV) was incorporated into the porcine chromosome about several millions of years ago, and amongst them, there are some viruses which possess full viral genes which can thus release infectious viruses. Seven porcine endogenous retroviruses strains have been found to date, and classified into three types in terms of envelope gene structure.

To date, some PERV have been found to infect human cultured cells although the efficacy thereof is low. There is a report in which co-culture of porcine renal cancer cell strain and human lung or renal cell has produced PERV in a cell (Patience C et al., Nature Medicine 1997 Mar; 3(3): 275-6). Furthermore, there is also a report that when porcine pancreas Langerhans islet cell was co-cultured with human renal epidermal cell derived cell strain, PERV was produced (van der Laan LJ, et al. Nature. 2000 Sep 7; 407(6800): 27, 29-30). Accordingly, transplantation tissues from swine are always in the state of risk for infection.

Decellularized tissue of the present invention has surprisingly succeeded in reducing the PERV to the level such that no infection occurs. Such PERV removal technologies have not been known to date, and thus the present invention has achieved significant effects by providing decellularized tissue with PERV substantially removed. Furthermore, as it is understood by those skilled in the art that such technologies may be available in a similar manner for removal of other infectious viruses, the present invention provides a decellularized tissue with substantially all infectious viruses removed therefrom, and a method for preparing the same.

The use of the decellularized tissue, grafts, and medicament of the present invention is usually performed under supervision of a doctor, or without supervision of a doctor if approved by an authority and a law of a country in which the present invention is used.

The amounts of decellularized tissue, grafts, and medicament used in the treatment or prophylactic method of the present invention can be easily determined by those skilled in the art, considering purposes of use, target diseases (type, severity, etc.), patient's age, weight, sex, case history, the form or type of physiologically active substance, the form or type of the tissue, or the like.

The frequency of the method of the present invention being applied to a subject (or a patient) can be easily determined by those skilled in the art, considering the doses of decellularized tissue, a graft, and a medicament, purposes of use, targeted diseases (type, severity, etc.), patient's age, weight, sex, case history, the course of therapy, and the like. The frequency includes, for example, once per day to once per several months (e.g., once per week to once per month). Preferably, administration is carried out once per week to once per month while observing progress.

As used herein, molecular biological techniques, biochemical techniques, and microbiological techniques well known in the art are optionally used. These methods are described in, for example, Ausubel F.A. , et al., editors (1988), "Current Protocols in Molecular Biology", Wiley, New York, NY; Sambrook J., et al. (1987), "Molecular Cloning: A Laboratory Manual", 2nd Ed., Cold Spring Harbor Laboratory Press, Cold Spring Harbor, NY; Bessatsu Jikken Igaku, "Idenshi-Donyu & Hatsugen-Kaiseki-Jikkenho" [Experimental Medicine, Special Issue, "Experimental Methods for Gene Introduction & Expression Analysis", Yodo-sha, 1997; and the like.

In another aspect, the decellularized tissue, graft, and/or medicament or pharmaceutical of the present invention comprise instructions which provide guidelines for administration of the decellularized tissue , graft and/or medicament. Here, the instructions describe an appropriate method for administrating the decellularized tissue, graft and/or medicament. The instructions are prepared in a format defined by an authority in a country in which the present invention is used (e.g., the Health, Labor and Welfare Ministry in Japan, Food and Drug Administration (FDA) in the USA, etc.). The instructions explicitly describe approval by the authorities. The instructions are a so-called package insert and are usually provided in a paper format. The instructions are not limited to this. For example, the instructions maybe provided in the an electronic format (e.g. , a web site on the Internet, an electronic mail, etc.).

The decellularized tissue, tissue graft, and medicament of the present invention can be implanted using a technique well-known in the art (for surgery, see Hyojun-Geka-Kagaku [Standard Surgical Science], 9th ver. (Igakugakuin), Kihonteki-Geka-Shujutsu-Shugi [Basic Surgical Techniques] (pp. 41-66), Shinzo [Heart] (pp. 349-398), Kekkan [Blood Vessel] (pp. 399-428), and the like). The decellularized tissue of the present invention may be used in vascular anastomosis, cardiovascular reconstruction, vascular prosthesis replacement, prosthetic valve replacement, and the like. Therefore, those skilled in the art can apply the decellularized tissue, tissue graft, and medicament of the present invention in accordance with the disclosure of the present specification depending on circumstances where treatment is performed.

In the present invention, the pharmaceutical of the present invention may be applied to any part of the body of an organism. Therefore, the tissue graft may be applied to a part of the body from which the pharmaceutical was derived or other parts of the body. As shown in the Examples, it was demonstrated that the desired effect (e.g., regeneration, self organization) of the present invention can be achieved for any part of the body to which the pharmaceutical of the present invention is applied, whether or not the part is a site from which the tissue was derived. Therefore, the present invention has a considerably advantageous utility such that the present invention can in principle be applied to any implantation operation and regeneration operation. Examples of parts to which the pharmaceutical of the present invention is applied, include, but are not limited to, skin, blood vessel, cornea, kidney, heart, liver, umbilical cord, intestine, nerve, lung, placenta, pancreas, brain, peripheral limb, retina, valve, epithelial tissue, connective tissue, muscular tissue, nervous tissue, and the like.

Hereinafter, the present invention will be described by way of examples. The following examples are provided only for illustrative purposes. Therefore, the scope of the present invention is limited only by the claims.

EXAMPLES

Hereinafter, the present invention will be described in greater detail by way of examples. The present invention is not limited to the examples below. Reagents and the like used in the following examples are commercially available from Sigma (St. Louis, USA), Wako Pure Chemical Industries, Ltd. (Osaka, Japan), and the like, with exceptions. Animal experiments were conducted in accordance with the guidelines established by Osaka University.

(Example 1)

(Materials and Methods)

(Decellularization by PEG)

Porcine carotid arteries were prepared from Hybrid (Labo Products Co. Ltd., Osaka, Japan), and rat aortas were prepared from SD rats (male, 5 weeks old, Nippon Animal Co., Ltd., Tokyo, Japan) under sterile conditions. Animal experiments were conducted in accordance with the guidelines for ethics established by Osaka University.

Freshly collected porcine carotid arteries and rat aortas were placed in PBS (referred to as PBS (-) in this example; Gibco BRL, Life Technologies Inc. Rockville, MD, USA) containing antibiotics (Gibco BRL, Life Technologies Inc. Rockville, MD, USA) to wash out blood components. The blood vessels were then placed in a decellularizing aqueous solution containing polyethylene glycol (1 g/ml, Nacalai Tesque Inc., Kyoto, Japan) (average molecular weight: 1000), and allowed to stand for 0.5 h. Because of high viscosity of the solution, the blood vessels were gently pressed several times with a glass rod at room temperature. The blood vessels were placed in PBS (-) containing antibiotics (100 units of penicillin, 0.1 mg of streptomycin, 0.25 $\mu$g/ml amphotericin B; all of which are available from Gibco BRL, Life Technologies Inc. Rockville, MD, USA) on a rotor (Tube rotator TR-118: Iuchi Co. Ltd, Osaka, Japan) at room temperature. The wash solution was changed every 24 hours over 72hours. After rinsing, the blood vessels were immersed in PBS (+) (PBS (-) supplemented with 5 mM MgCl$_2$) containing DNaseI (Takara Shuzo Co., Ltd., Shiga, Japan) at 37°C for 1 h. The blood vessels were placed in the above-described PBS (-) containing antibiotics on a rotor at room temperature. The wash solution was changed every 24 hours over 72 hours. After rinsing, the blood vessels were preserved in PBS (-) containing antibiotics at 4°C.

(Collagen Structure)

It has been revealed that collagen structure is affected by pH, ionic strength, the polarity of a solvent, anionic surfactants, and the like (Ripamonti A., et al., 1980, Blopolymers 19:965-975: and Xiao W. H., Knight D. P. , Chapman J. A., 1997, Biochimica et Biophysica Acta. 1134:327-337). It is also known that a change in collagen structure results in an alteration in bioengineering characteristics. In order to avoid these problems, a decellularization procedure was designed in this example so that matrices are not affected. It was demonstrated by histological tests and tensile strength measurements of the collagen structure that matrices were absolutelynot impaired as follows.

(Histological examination)

Paraffin sections (thickness: 3 $\mu$m) of a blood vessel graft were prepared and stained with hematoxylin-eosin to identify extracellular matrices. To identify I/IV collagen which is a component of the basal membrane, immunohistochemical staining was used. Aortas of SD rats (male, 5 weeks old, Nippon Animal Co., Ltd., Tokyo, Japan) were fixed with 4% paraformaldehyde and cryoprotected. Frozen sections (thickness: 5 $\mu$m) were prepared, followed by permeabilization with PBS (-) for 3 h and then blocking with 1% BSA in PBS (-) for 1 h at room temperature. Subsequently, the sections were incubated with primary antibodies (anti-rat collagen antibodies, Cosmo Bio, Tokyo, Japan), and then with secondary antibodies conjugated with FITC (anti-sheep Ig antibodies; Cosmo Bio, Tokyo, Japan). Images were obtained with a Zeiss LSM 510 confocal microscope.

(RESULTS)

PEG having average molecular weight of 1, 000, 2,000, 200and 600 have been used for the present test to find out that PEG having any molecular weight may be used for decellularization. Decellularized treatment using PEG having average molecular weight of 2000 appeared to give better results, however, handling of decellularization treatment was easier when using PEG having average molecular weight of 1,000.

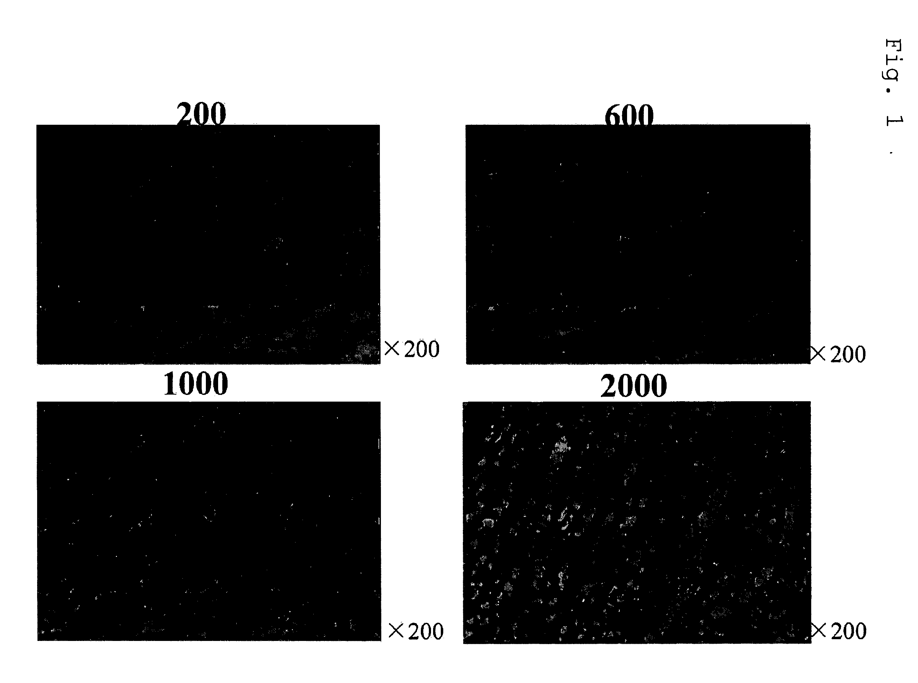

Decellularization process is typically depicted in Figure 1.

(Presence/Absence of agitation)

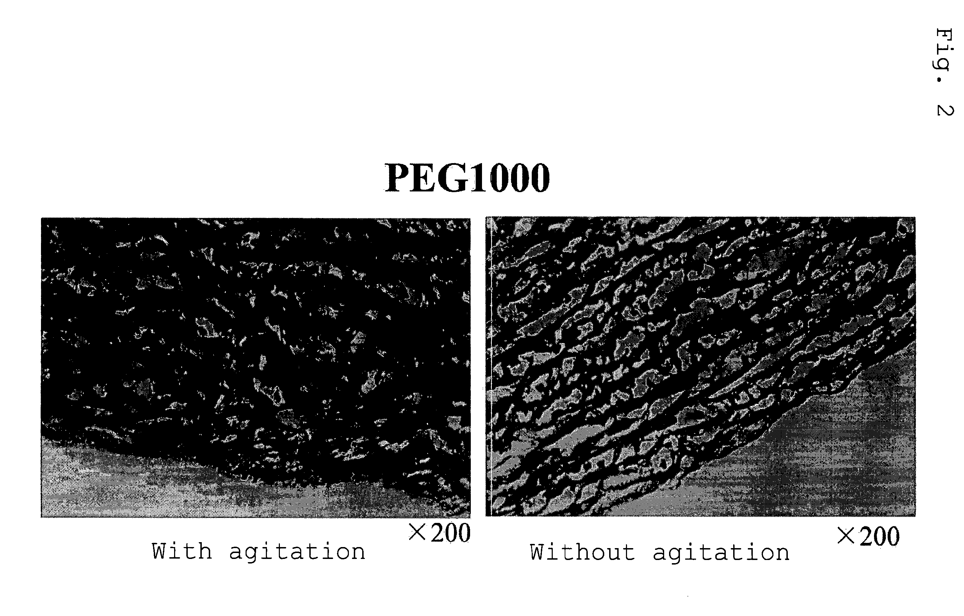

Next, effects of present or absence of agitation on decellularization efficiency is reviewed. In the present Example, PEG having average molecular weight of 1,000 was used and confirmed for the effects thereof. Three hundred RPM was employed as agitation condition, and the agitation was conducted for seven days. Decellularization without agitation was conducted using the same conditions except for agitation as control. Results are shown in Figure 2. As shown in Figure 2, presence or absence of agitation does not affect the efficiency of decellularization, nor does it affect the tissue strength.

(Combination of ultrasonication)

Next, PEG treatment is combined with ultrasonication treatment as an example of a radical reaction. As described above, PEG having average molecular weight of 1,000 was used, and BRANSONIC(R) mode13510; oscillation wavelength: 42kHz (see http://www.branson-jp.com/products/bransonic/3510.html; table washer). In the vessel used (in water), valve was placed in the same manner as the washing step, and sonication was conducted. Time period for treatment time was 24 hours

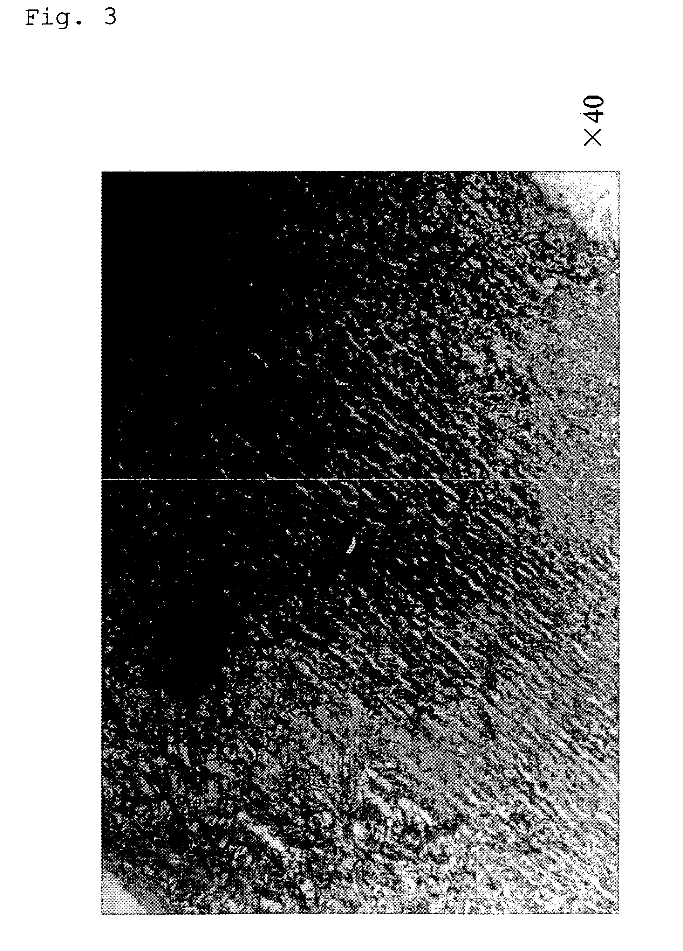

The results are shown in Figure 3. It is understood that combination with ultrasonication promoted decellularization in comparison with PEG treatment alone.

(Presence or absence of DNase treatment)

Next, under the above-mentioned experimental conditions, PEG (average molecular weight 2000) was used to study the enhancement of decellularization efficiency in the presence or absence of DNase. The results are shown in Figure 4. AS seen from Figure 4, it was revealed that treatment with DNase appears to accelerate decellularization.

Next, decellularization tissue was prepared based on a different decellularization process. The procedures therefor is described hereinbelow.

(Decellularization method by SDS, No. 1)

1) (Washing) Tissue was washed with physiological saline for one hour at 4°C.

2) (First surfactant treatment) The tissue was placed in physiological saline containing 1% SDS (sodium dodecyl sulfate, SIGMA L-4509) and allowed to stand at room temperature for 48 hours.

3) (Washing) The tissue was removed and was placed again in physiological saline and allowed to stand for 24 hours at room temperature.

4) (Second surfactant treatment) The tissue was placed in physiological saline containing 1% NP-40 (SIGMA 1-3021) and was allowed to stand at room temperature for 48 hours.

5) (Washing) The tissue was removed and was placed again in physiological saline and allowed to stand for 24 hours at room temperature.

6) (Sterilization) The tissue was placed in 20% isopropanol (SIGMA I-0398) and was aseptically preserved before use or experimentation.

(Decellularization method by SDS, No. 2)

1) (Washing) Tissue was placed in physiological saline containing a protease inhibitor (PROTEASE INHIBITOR COCKTAIL; SIGMA P2714) and 20 mM EDTA and washed for 24 hours at 37°C.

2) (First surfactant treatment) The tissue was placed in physiological saline containing 1% SDS (sodium dodecyl sulfate, SIGMA L-4509) and was allowed to stand at room temperature for 72 hours.

3) (Washing) The tissue was removed and was placed in physiological saline and allowed to stand for 48 hours or more at 37°C.

4) (Second surfactant treatment) The tissue was placed in physiological saline containing 1% NP-40 (SIGMA I-3021) and was allowed to stand at room temperature for 48 hours or more.

5) (Washing) The tissue was removed and was placed again in physiological saline for 48 hours at 37°C.

6) (Cryochemistry process) A cryochemistry process was performed in accordance with a commonly used method.

7) (Sterilization) The tissue was placed in 0.05% sodium azide and was aseptically preserved before use or experimentation.

The above-described procedure was performed in accordance with conventional methods.

(Triton treatment)

1) (Washing) Tissue was washed with physiological saline for 1 hour at 4°C.

2) (Surfactant treatment) The tissue (e.g., a blood vessel)was immersed in physiological saline containing 1% Triton (registered trademark) X-100 (ICN Biomedicals, Inc., CA, USA) +0.1% ammonium hydroxide (Wako Pure Chemical Industries Ltd., Osaka, Japan), i.e., a decellularization solution. The decellularization solution was agitated on a shaker at 4°C and changed every 24 hours..

3) (Washing) Thereafter, the tissue was washed by changing PBS every 24 hours a total of three times while agitated on a shaker at 4°C.

(Decellularization method by SDS, No. 3)

1) Tissue wash: 0.3% NaCl, protease inhibitor (PROTEASE INHIBITOR COCKTAIL; SIGMA P2714), 20 mM EDTA, 20°C, 24 hrs
2) Treatment with detergent: 0.5% SDS (sodium dodecyl sulfate, Sodium Laurel Sulfate, SIGMA L-4509)+ 0.5% Triton X-100(ICN Biomedicals Inc.CA.USA) in 0.3% NaCl + 0.05% $NaN_3$, room temperature
3) Wash: 0.3% physiological saline + 0.05% $NaN_3$, room temperature, at least 120 hours
4) Sterilization and preservation: cold chemical treatment: place and soak the sample for 3 hours in a saline solution containing 0.03% $NaN_3$.

Thus, in treatment of tissue using a surfactant, it is known that SDS and Triton are preferable for the purpose of obtaining desirable physical characteristics. When surfactants, such as SDS and Triton, are in the micellar state, they seem not to have the desired characteristics of strength and the like. Conversely, it was found that when the surfactants are not in the micellar state, these agents can achieve the object of the present invention. Whereas surfactants form micelles which in turn remove substances, such as proteins, lipids, and the like. An amphipathic molecule (e.g., PEG) as usedherein is used in the non-micellar state. Therefore, a solution containing a non-micellar amphipathic molecule is used to remove cell components in accordance with a method for extracting cell components. Thus, this example revealed that the non-micellar amphipathic molecule-containing solution performs decellularization treatment more satisfactorily than a solution containing a micellar molecule, such as a surfactant. Accordingly, it is considered that other non-micelle forming amphipathic molecules can be used for the decellularization treatment of the present invention.
Accordingly, decellularized tissue using such amphipathic solution may be subjected to any radical reaction as described below.

(Radical reactions)

In the present Example, a radical reaction was performed using gamma-ray irradiation. Those skilled in the art would have readily understood that the present invention can be performed in a similar manner by using a radical exposure other than gamma-ray irradiation.

Gamma-ray irradiation is conducted as follows: The procedures are briefly described.

Decellularized tissue after PEG treament (for example, decellularized heart valve or the like) was placed in a glass sampling vessel with plastic cap (size: diameter about 5cm x height about 8cm), and a cobalt 60 irradiation source was used to perform gamma-ray irirradiation with the cap closed (tissue and air existed inside). Temperature was ordinary temperature (room temperature) and the temperature rose as the irirradiation progressed by the heat produced thereby. The temperature during irirradiation was between 25° C and 40° C. Washing may or may not be performed after decellularization and before gamma-ray irradiation.

Although not wishing to be bound by any theories, effects of gamma-ray irradiation is believed to include, for example, both direct cell damaging action by means of the gamma-ray irradiation, and cell damaging action by free radicals caused by the employment of gamma-ray irradiation. In this case, PEG is believed to act as a scavenger therefor.

Next, a variety of conditions of gamma-ray irradiation have been studied.

(PEG)

As described above, 200, 600, 1000 or 2000 were used as molecular weight.

(Gamma-ray irradiation)

None, 20kGy, 40kGy, 50kGy, 80kGy, 100kGy, 130kGy and 250Gky were used as gamma-ray irradiation.

(Agitation treatment)

A variety of agitation as described above were combined.

(DNase treatment)

Presence or absence of DNase was combined with the conditions described above.

(Ambient conditions)

In addition to the atmosphere, those in water (similar to that of decellularization treatment solution, and PEG of average molecular weight of 1,000 were used).

(Control experiments)

As a control, irradiation results against a living valve was included.

(Results)

(Irradiation)

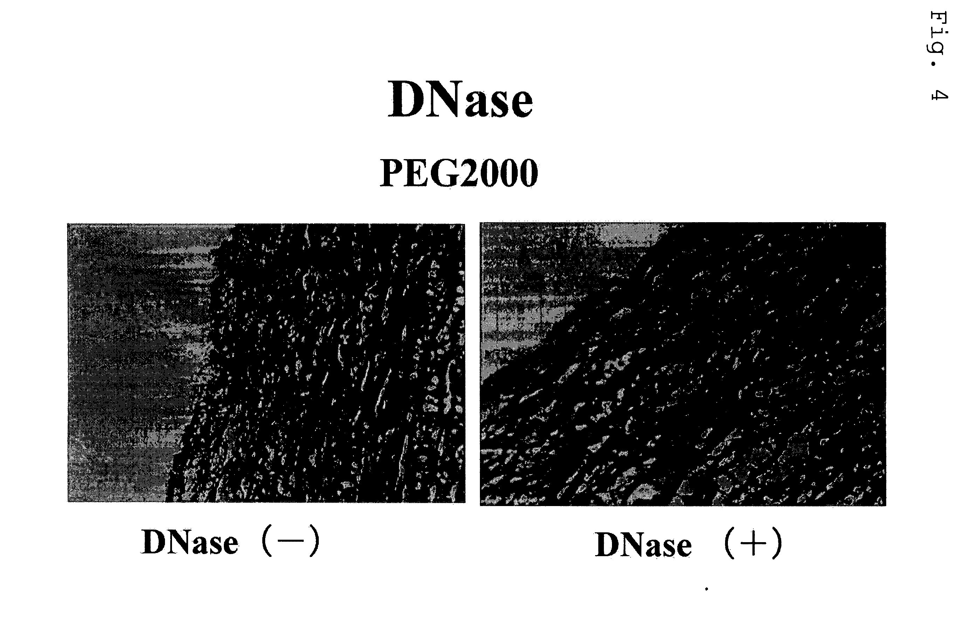

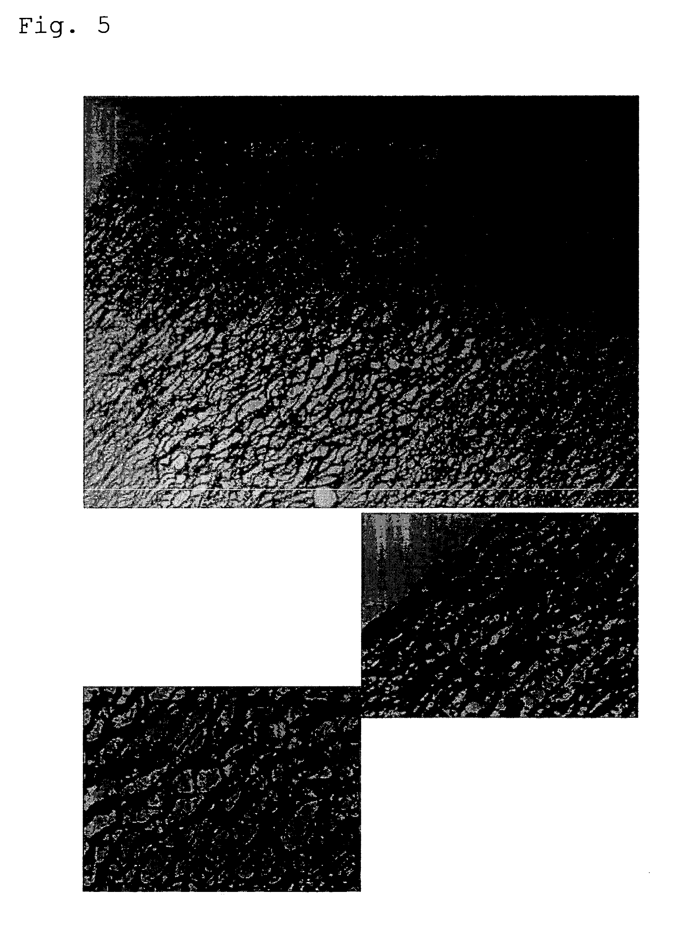

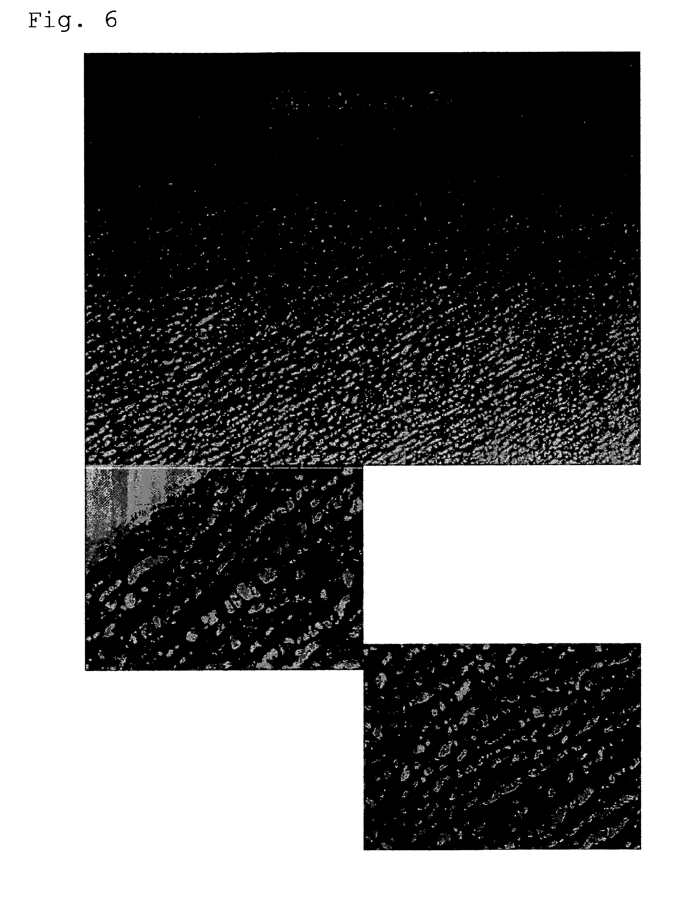

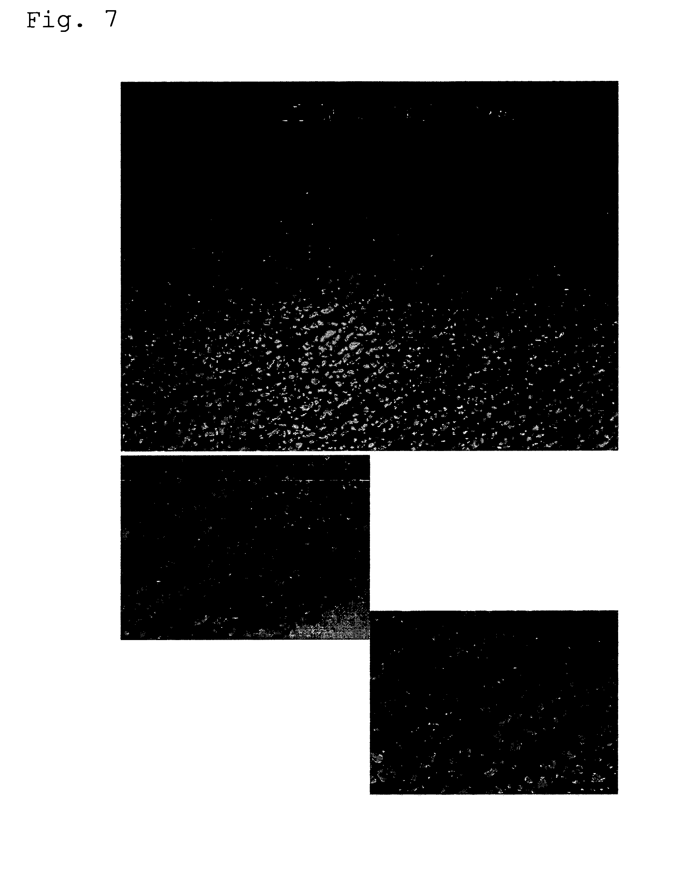

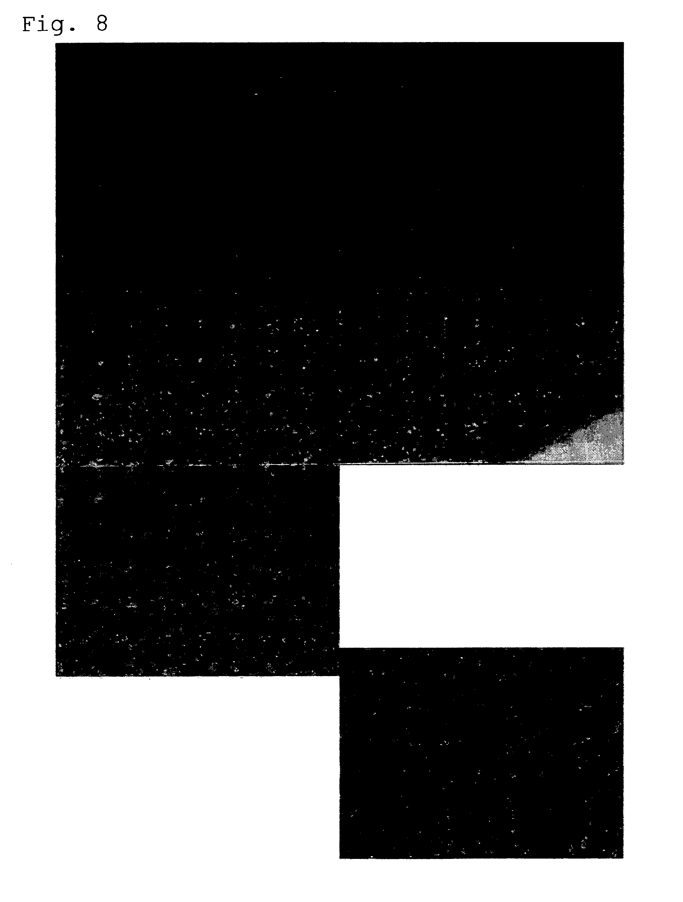

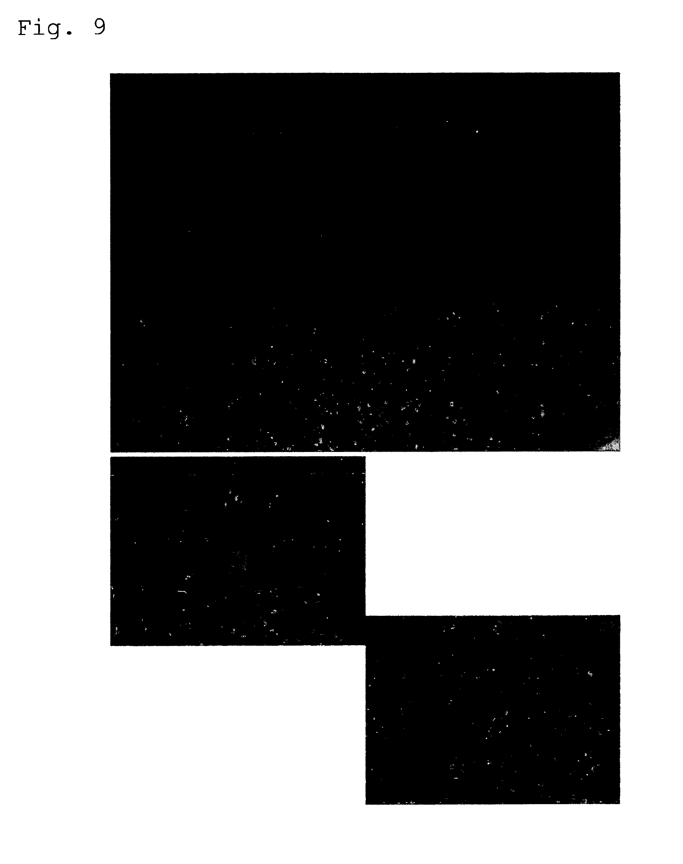

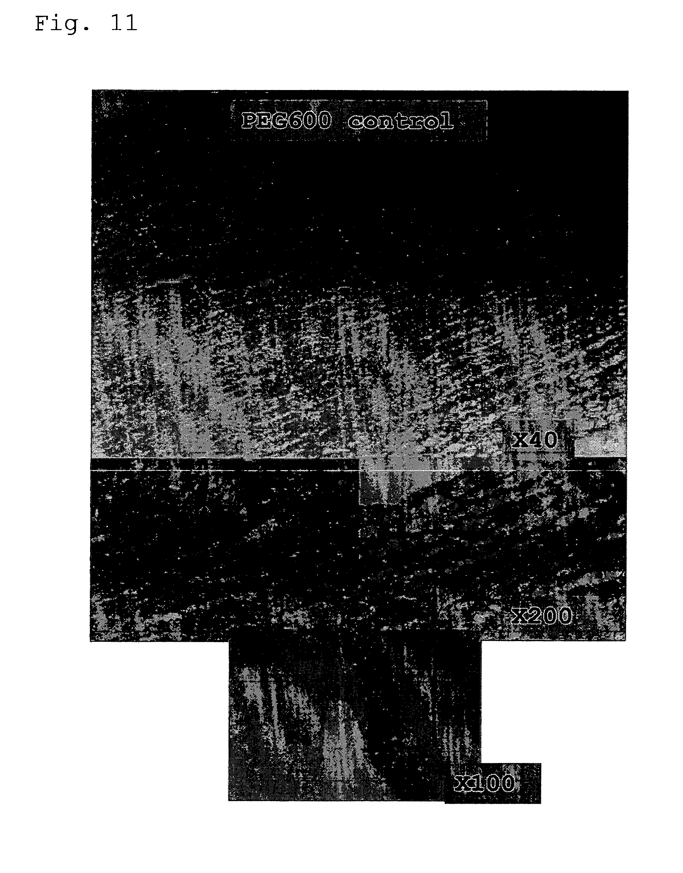

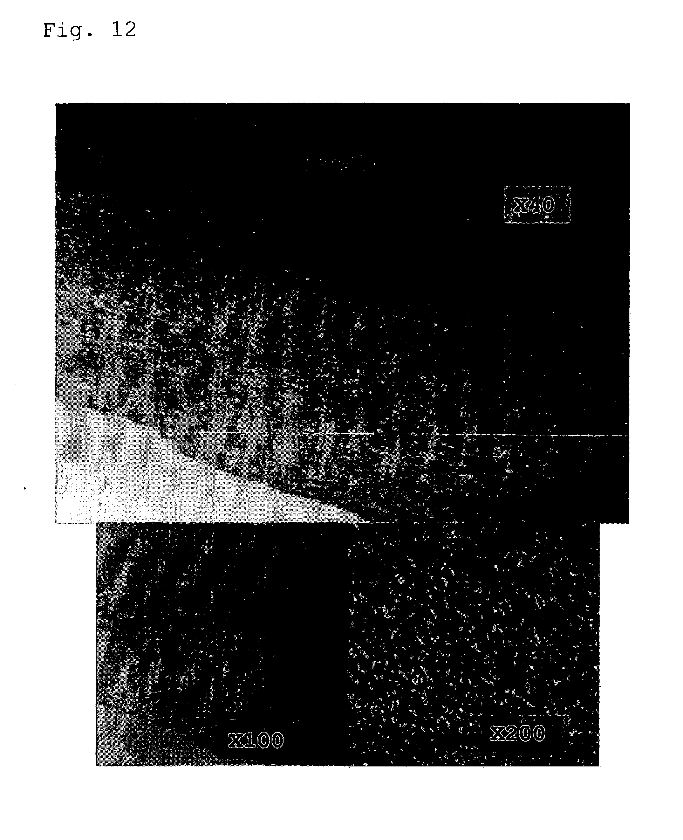

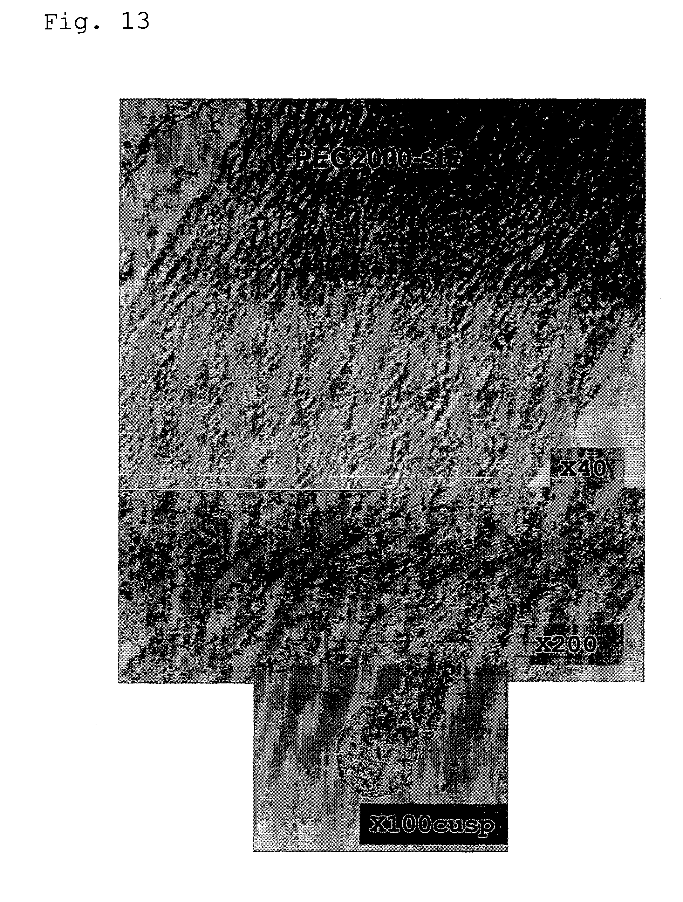

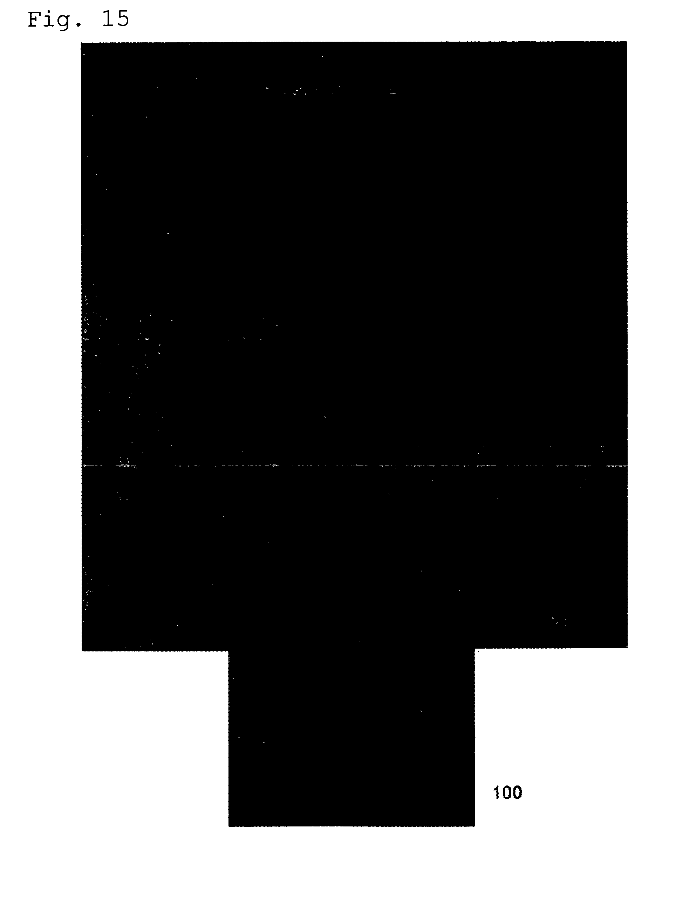

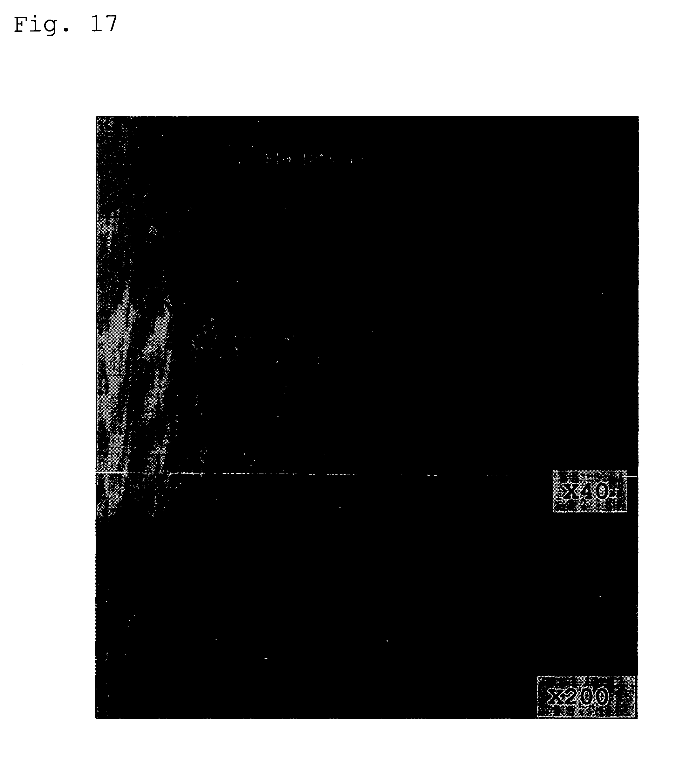

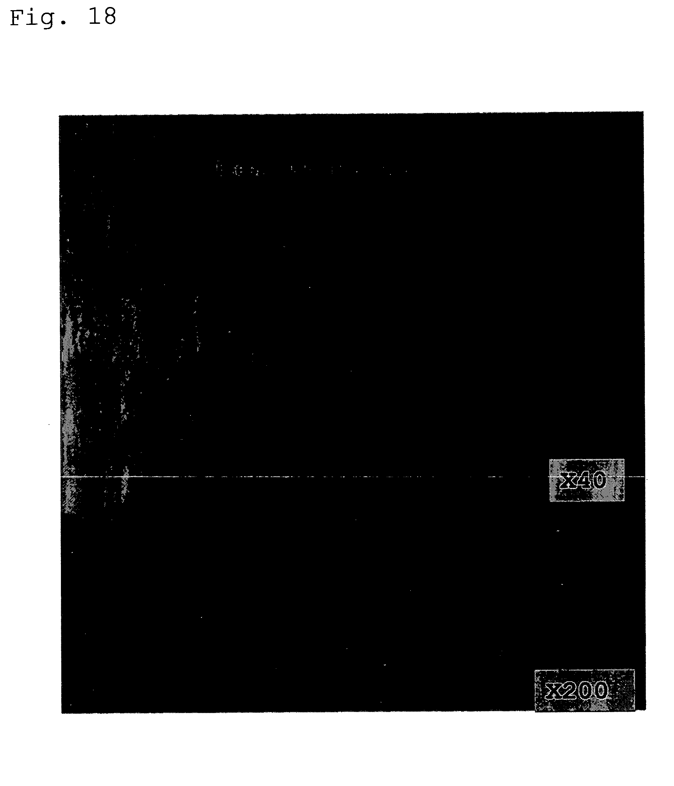

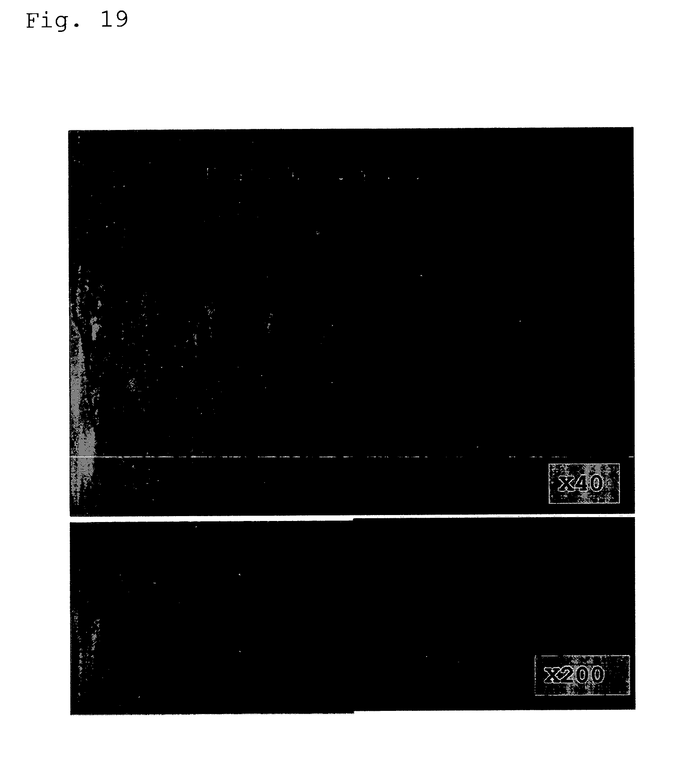

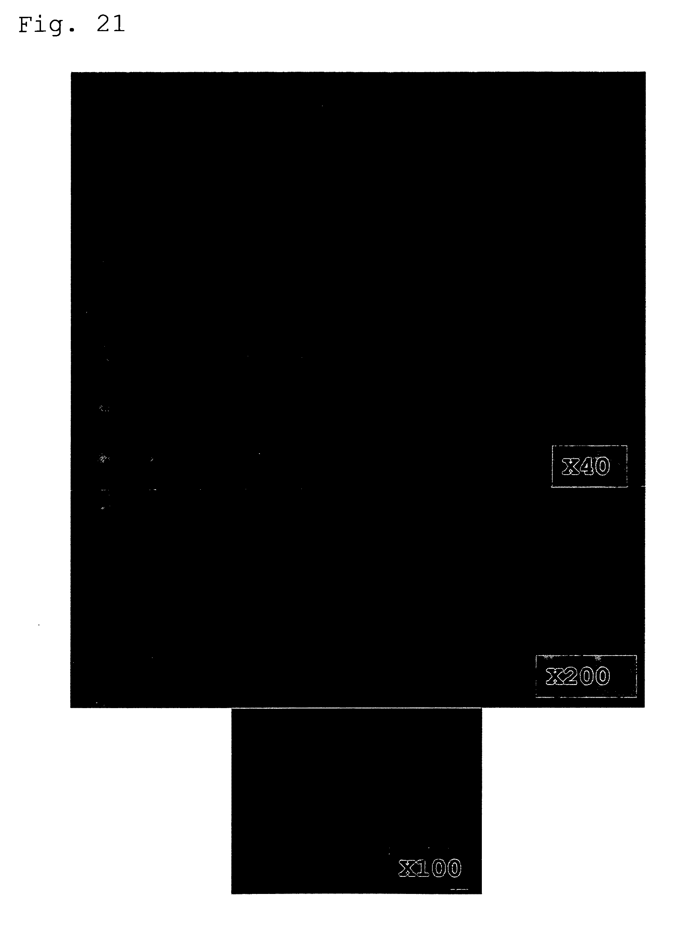

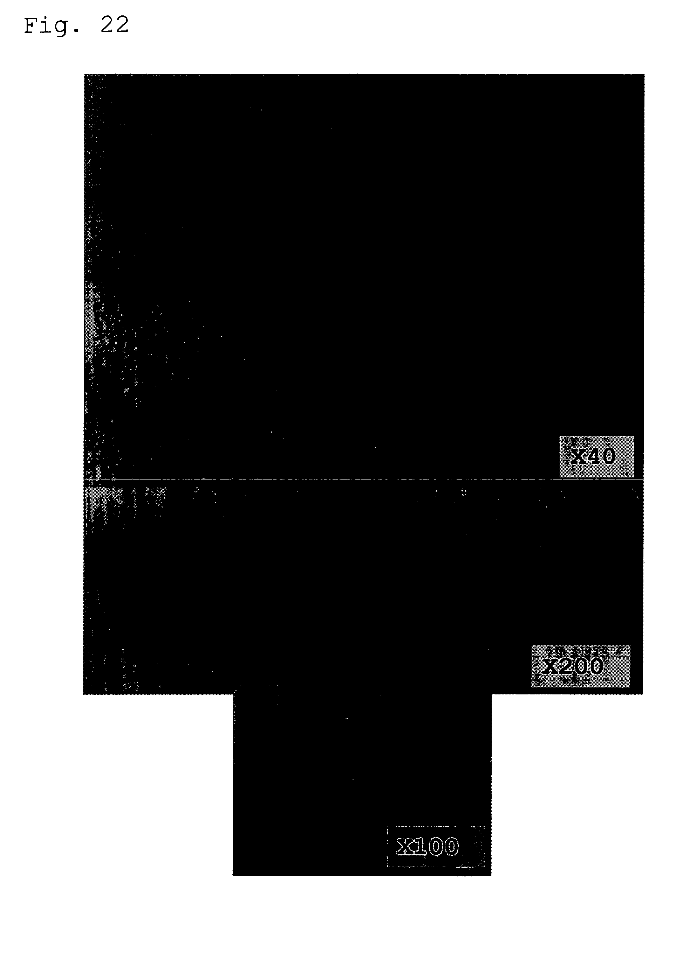

Differences of irradiation dosage are shown in Figures 5-22 for 20kGy, 50kGy, 100kGy and 130kGy. As shown in the photographs shown therein, the entire range of irradiation dosages were shown to decellularize in an efficient manner. In terms of cell strength, 40kGy-100kGy are preferable, and more preferably, 50kGy-80kGy appear to be advantageous, however, it was revealed that the entire range tested were shown to produce tissues with sufficient strength as transplantation tissue. Furthermore, the range of the presently preferable appears to recognize recellularization.

(Ambient conditions)

Comparative results in which ambient conditions were studied, are included in Figures 5-22. As seen from the figures, gamma-ray irradiation in the atmosphere appears to attain more efficient decellularization. However, it is also found that any ambient conditions promoted decellularization.

(Study of Preferable combinations)

Furthermore, a variety of conditions are studied as follows in the table.

TABLE 1

| Figu re | Site | PEG | DNase (70U/m 1, MW2000 37°C, 48 hours) | exposure dose (kGy)ex posure dose is five hours and 30 minutes | Ambient temperature | Agitat ion (seven days, room temper ature for each) |
|---|---|---|---|---|---|---|
| 5 | valve (cont rol) | 2000 | yes | none | none | yes |
| 6 | valve | 1000 | none | 20 | in water | yes |
| 7 | valve | 1000 | yes | 20 | in water | yes |
| 8 | valve | 1000 | none | 100 | in water | yes |
| 9 | valve | 1000 | yes | 100 | in water | yes |
| 10 | valve | 200 | none | none | none | yes |
| 11 | valve | 600 | none | none | none | yes |
| 12 | valve | 1000 | none | none | none | yes |
| 13 | valve | 2000 | none | none | none | yes |
| 14 | valve | none | none | 100 | atmosphere | none |
| 15 | valve | none | none | 100 | in water | none |
| 16 | valve | 1000 | yes | 100 | atmosphere | yes |
| 17 | valve | 1000 | yes | 100 | in water | yes |
| 18 | valve | none | yes | 100 | atmosphere | yes |
| 19 | valve | none | yes | 100 | in water | yes |
| 20 | valve | 1000 | none | 100 | atmosphere | yes |
| 21 | valve | 1000 | yes | 100 | atmosphere | yes |
| 22 | valve | 1000 | none | 130 | atmosphere | yes |
| 23 | valve | 1000 | yes | 130 | atmosphere | yes |
| 24 | valve | 1000 | none | 50 | atmosphere | yes |
| 25 | valve | 1000 | yes | 50 | atmosphere | yes |
| 26 | valve | 2000 | none | 50 | atmosphere | yes |
| 27 | valve | 2000 | none | 100 | atmosphere | yes |
| 28 | valve | 2000 | yes | 130 | atmosphere | yes |
| 29 | valve | 1000 | yes | 100 | atmosphere | yes |

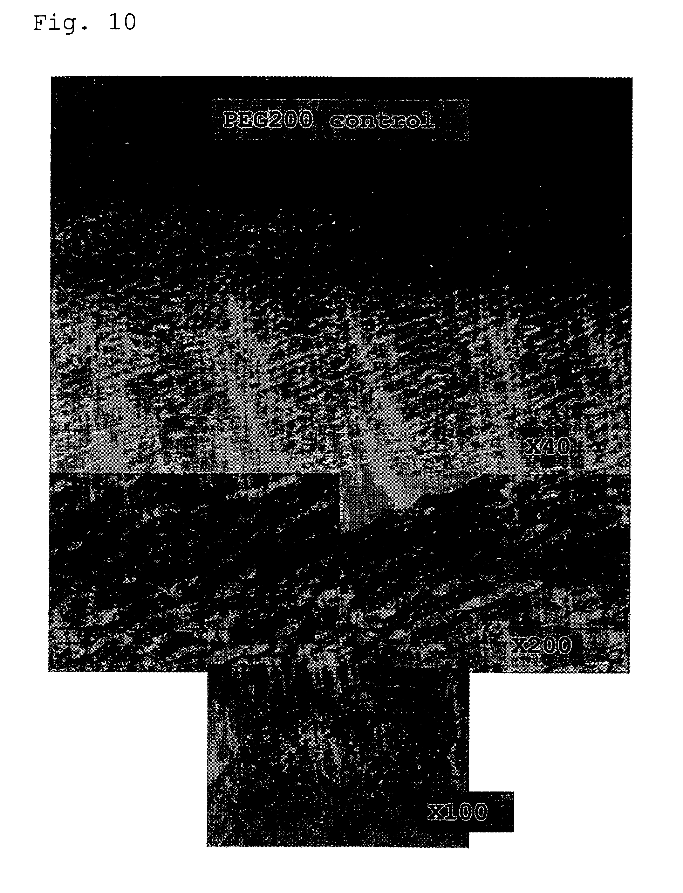

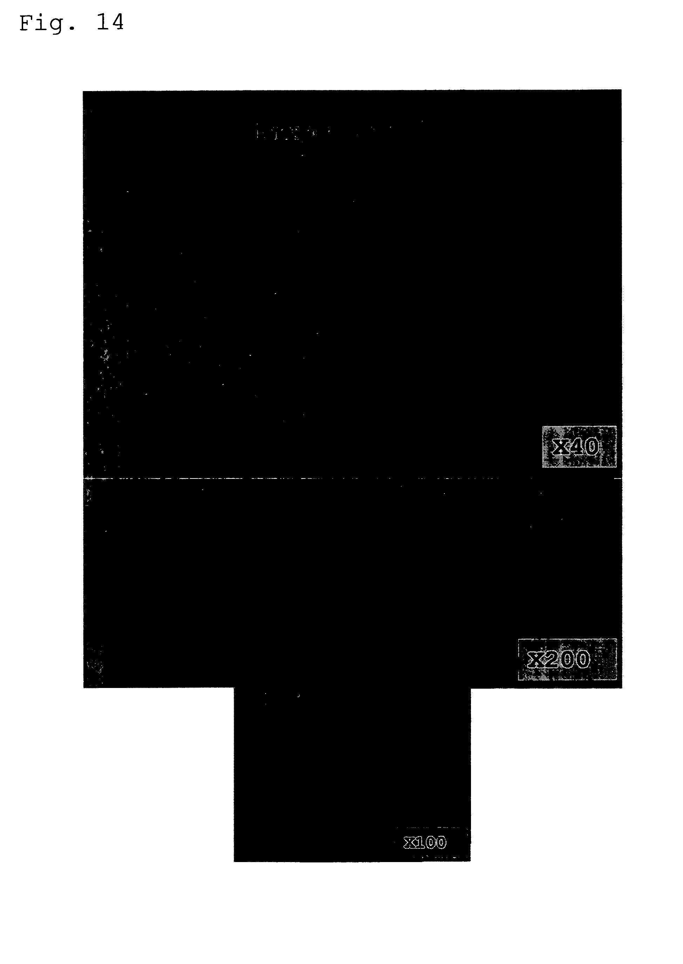

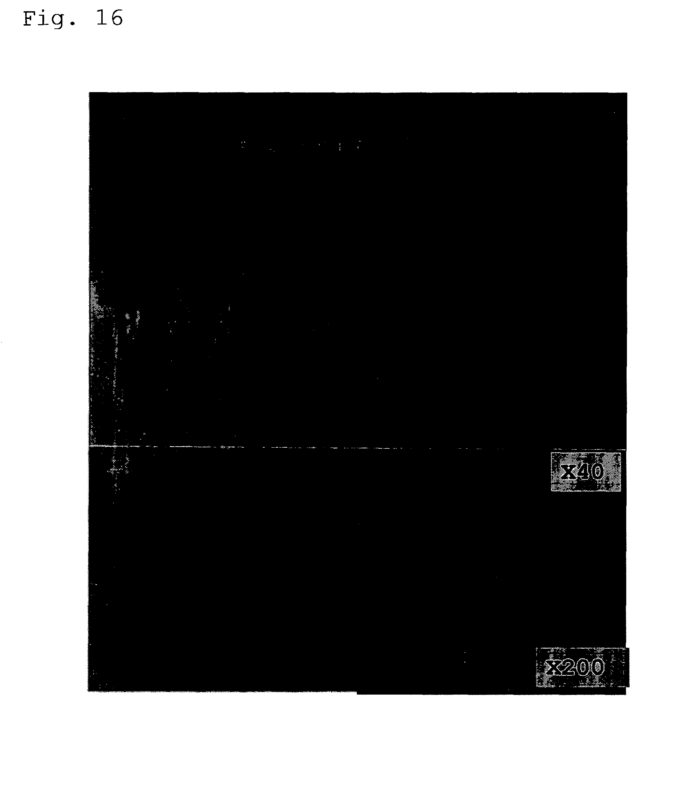

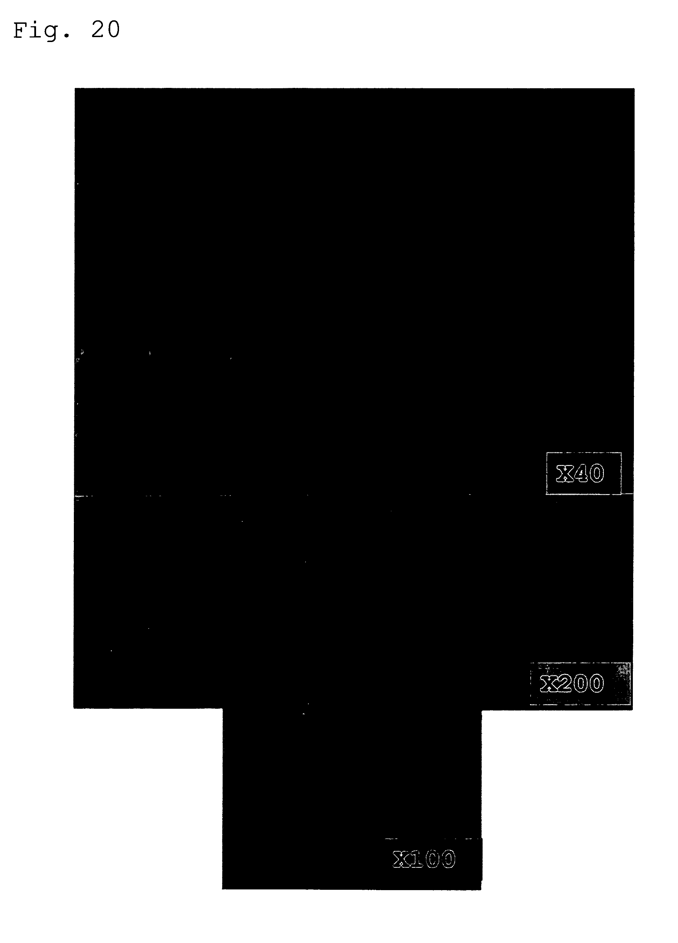

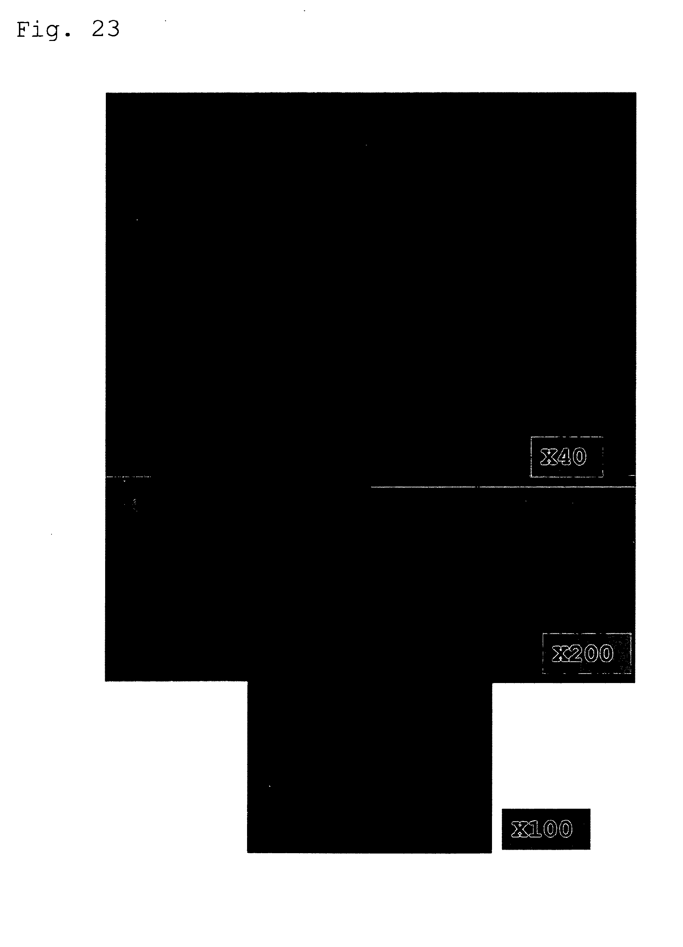

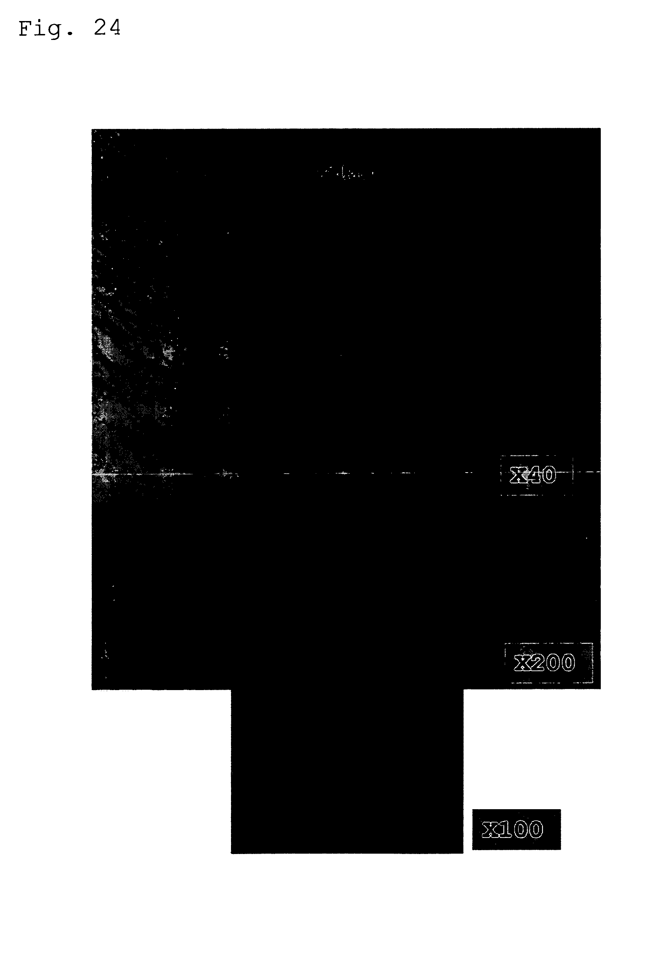

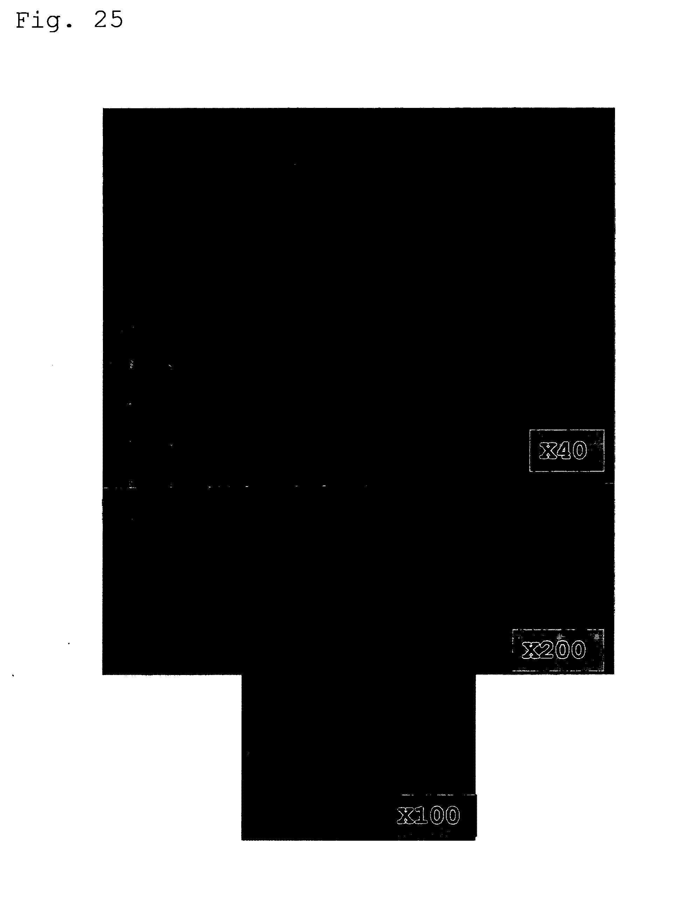

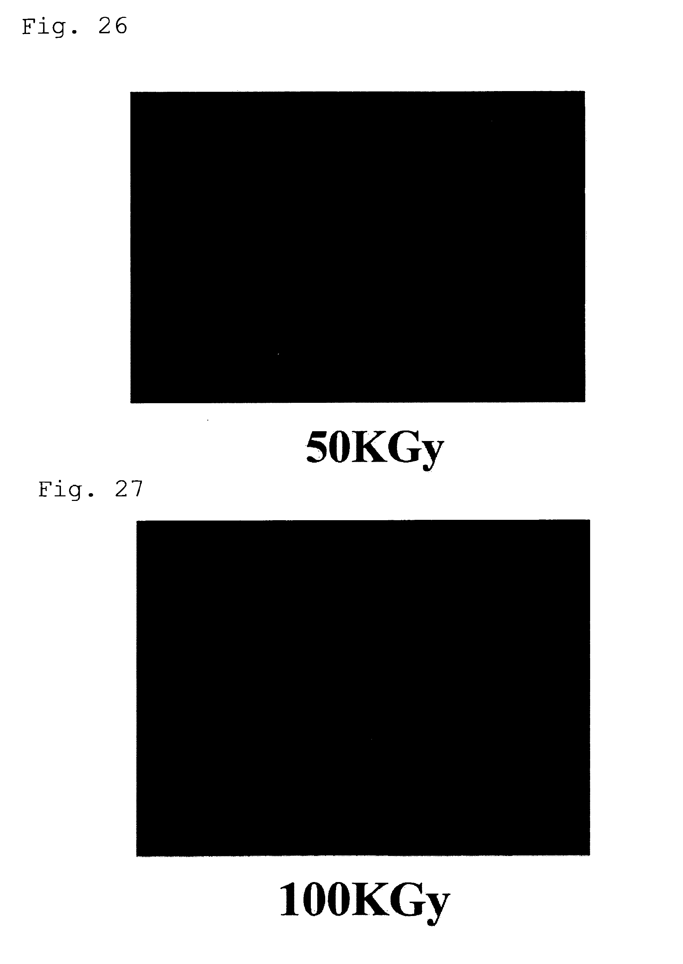

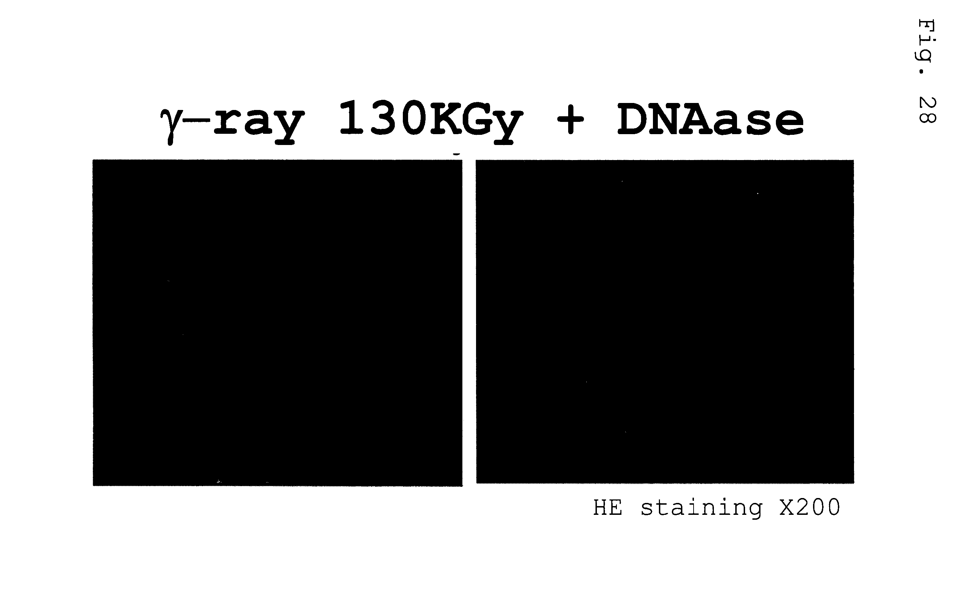

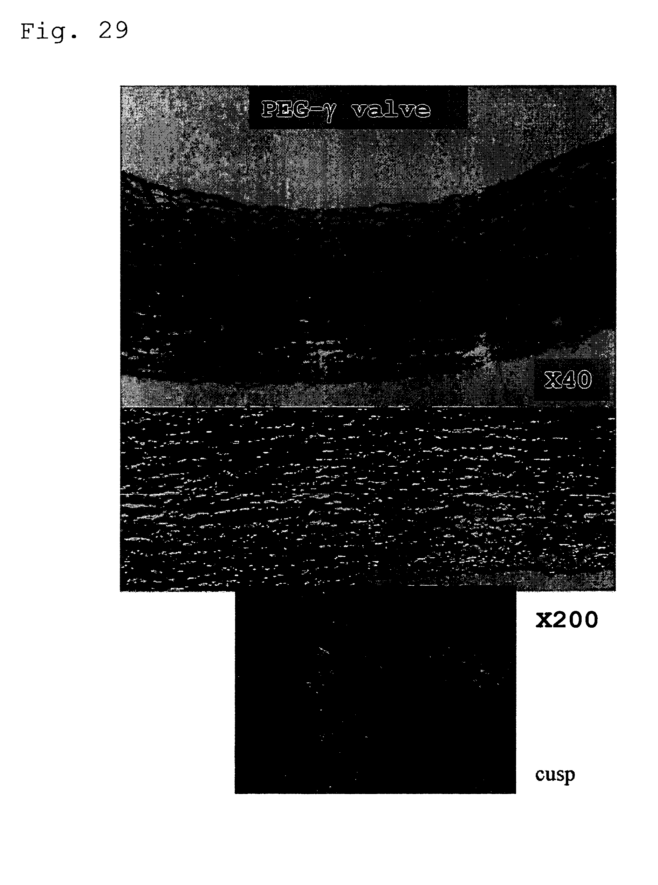

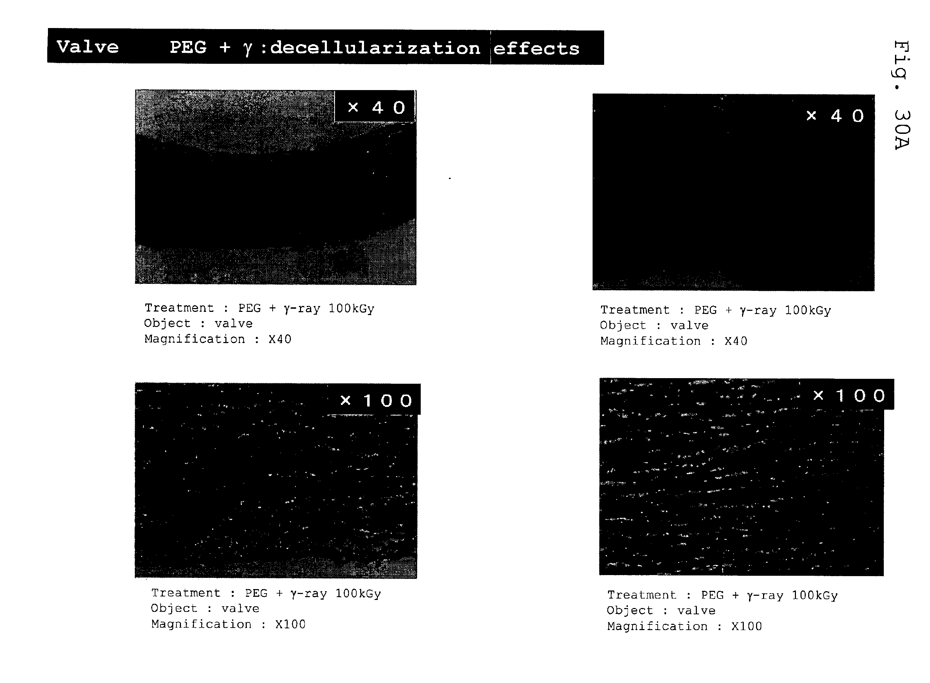

As shown in the above-mentioned Table, Figure 5 shows control (with DNase treatment) and Figures 6-9 show optimization in which water/PEG1000 were fixed and gamma-ray irradiation strength and presence/absence of DNase were varied. Figures 10-13 show the results in which PEG molecular weight optimization was conducted with gamma-ray irradiation and without DNase. Figures 14-15 show the results in which gamma-ray irradiation dosage was 100kGy, without PEG or DNase were fixed and the effects of only gamma-ray irradiation are shown (in water/atmosphere). Figures 16-19 show gamma-ray irradiation dosage fixed at 100kGy, and PEG1000 was fixed, and thereafter the final conditions (Figure 29 and the like) were performed in a reverse order, i.e., PEG treatment was performed after the irirradiation with gamma-rays. PEG treatment after gamma-ray irradiation was observed to see the effects of PEG treatment (in atmosphere/in water). Figure 20-25 show the effects of gamma-ray irradiation strength and the presence/absence of DNase in the atmosphere and PEG1000. Figures 26-28 show the effects of gamma-ray irradiation strength and the presence and absence of DNA in the atmosphere and PEG2000. Figure 29 show the final condition in the present Example.

(Comparative experiments by forms)

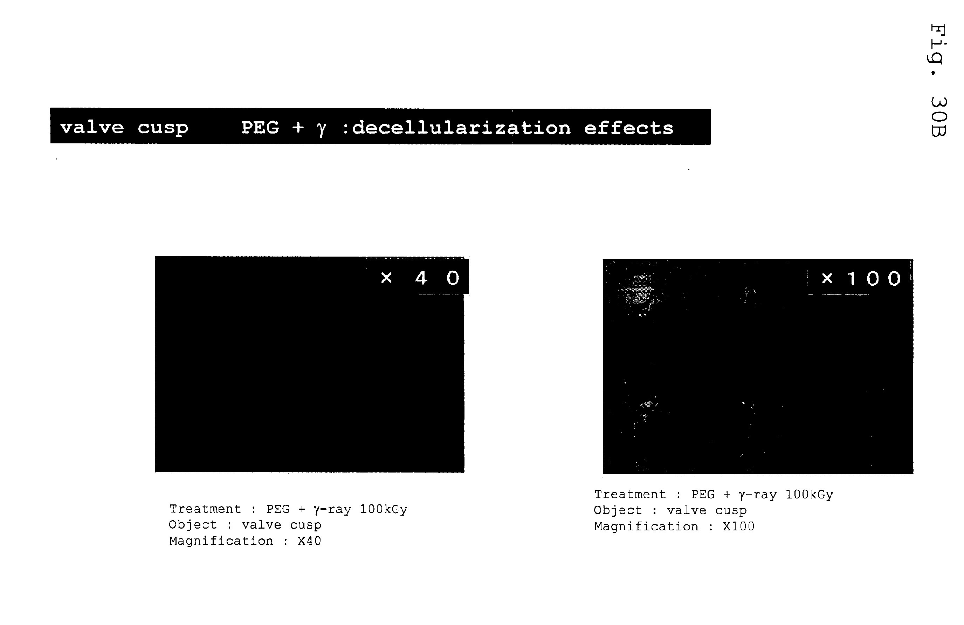

In order to demonstrate that decellularization effects are shown in a variety of forms, valves and valve cusps were used for confirming decellularization effects. Results are shown in Figure 30A (valve) and Figure 30B (valve cusp). Decellularization effects can be visually confirmed in a clear manner for valves and valve cusps.

(P-D relationship)

Measurement of pressure-diameter (P-D) relationship was carried out in accordance with the Hiromichi Sonoda and Keiichi Takamizawa method (Hiwomichi Sonoda et al., J. Biomed. Mater. Res., 2001, 55:266-276). One end of an artery segment was cannulated to a fixed stainless steel connector while the other end was cannulated to a sliding connector. The blood vessel segment was gradually inflated with a pressurized intraluminal Krebs-Ringer solution. The diameter of a middle portion of the blood vessel was monitored using a television system (C1000-16; Hamamatsu Photonics) and a width analyzer (HTV-C1170; Hamamatsu Photonics). The interluminal pressure of the vessel was simultaneously recorded using a pressure transducer (6M92; NEC Sanei, Tokyo, Japan).

(Results)

In the methods of the present Examples, cytoplasmic components in the native tissues have been removed by an aqueous solution of 80% PEG (average molecular weight: 200-2000). It is therefore apparent that 60-100 % of PEG is preferably used for better results. Fluidity of cellular membrane has been significantly removed by a PEG solution of high density. Cellular membrane and cellular components other than DNA components have been observed to be removed. DNA components were observed to sediment. Aggregation of these components are clarified by accessibility of PEG solution to DNA. This is a main cause for enabling extraction of cellular membrane and cytosol. Permanent degradation and solution of DNA components caused in the tissue transplantation may be easilyremovedbyDNaseI . This is clearly observed by HE staining images. Based on the removal of cellular components, cavity formation was observed between elastic plates. It appears that this cavity formation suppresses degradation of extracellular matrices . In particular, the present treatment is nominally lower in the effects against collagen components.

(Cell residual rate)

A cell residual rate was calculated by counting the number of nuclei in an area of 100 $\mu$m $\times$ 100 $\mu$m using a microscope. Specifically, the number of nuclei in the same sample was counted in the area before and after treatment. The number of nuclei in the sample after treatment was divided by the number of nuclei in the sample before treatment and the result was multiplied by 100 to obtain a cell residual rate (%). The results are shown in Table 2.

(Tissue damage rate)

A visual field was divided into units of 100 $\mu$m x 100 $\mu$m and the number of units having an elastin rupture site was counted. There were 24 units per visual field. Elastin rupture was represented by x/24. x was counted when rupture was found in a unit. For example, when no rupture was confirmed (e.g., untreated control), the damage rate was calculated as 0/24, i.e., 0%. The results are summarized in Table 2.

Table 2

|  | Cell residual rate[1] | Tissue damage rate[2] |
|---|---|---|
| No treatment | 100% | 0% |
| PEG treatment | 4.7% | 15% |
| TRITON treatment | 17.8% | 34% |
| SDS treatment | 3.0% | 30% |
| SDS + $\gamma$-ray treatment | 3.0% | 30% |

(continued)

|  | Cell residual rate[1] | Tissue damage rate[2] |
|---|---|---|
| PEG + γ-ray treatment | 3.0% | 10% |

1) calculated by an average of the number of nuclei in the unit area of 100 μm x 100 μm

2) One visual field is divided per 100μm x 100μm, and the number of units having an elastin rupture site was counted (24 units per visual field).

From the results above, gamma-ray irradiation at least attained less than 5% cell residual rate, and under preferable conditions (for example, 100kGy and the like), cell residual rate of less than 1% was observed. Cell residual rate was observed by counting method by microscope with hematoxylin and eosin staining (HE staining). Hematoxylin and eosin staining procedures are as follows: Optionally, conduct deparaffin treatment (for example, with pure ethanol), wash with water, and soak the sample in hematoxylin from Omni for ten minutes. Thereafter, wash in running water. Use ammonia water to develop color for thirty seconds. Thereafter , wash in running water for five minutes, stain for two minutes using eosin hydrochloride ten-fold diluted solution, dehydrate, perform lucidification and mount.

(Tissue strength)

Tension strength, elasticity and extension were observed by tension test. The general outline therefor is described hereinbelow:

The present Example measured the strength by tension testing device (TENSILLON available from ORIENTEC). Specifically, as used herein, (1) specimen is cut to a 5 x 30 mm rectangular form (basically, specimen is cut such that wall portion of aortic basal portion becomes longer in the longitude direction); (2) fixation portion of tension testing device is fixed for about 5 mm at both ends of the specimen (ORIENTEC, TENSILON using universal testing device RTC-1150A); and (3) start stretching and continue stretching at a rate of 1cm/min to the rupture points, which were used to measure rupture load and elasticity (Shinoka T,Mayer JE. Tissue engineering heart valve leaflets.Circulation.1997; 96 (suppl. II): II-102-II-107; Shum-Tim D, Mayer JE. Tissue engineering of autologus aorta using a new biodegradable polymer. Ann Thorac Surg. 1999; 68: 2298-2304).

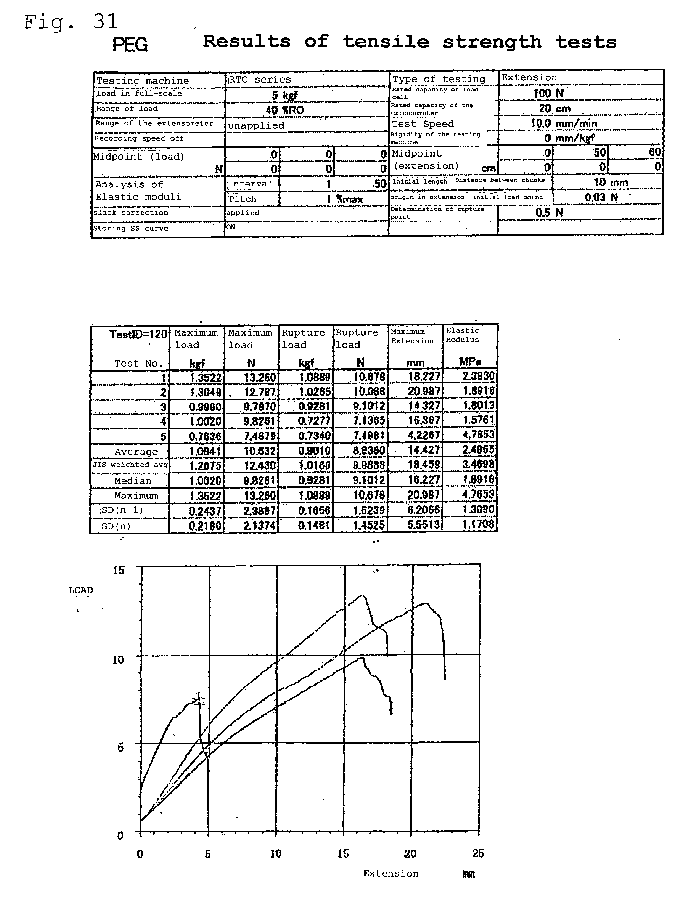

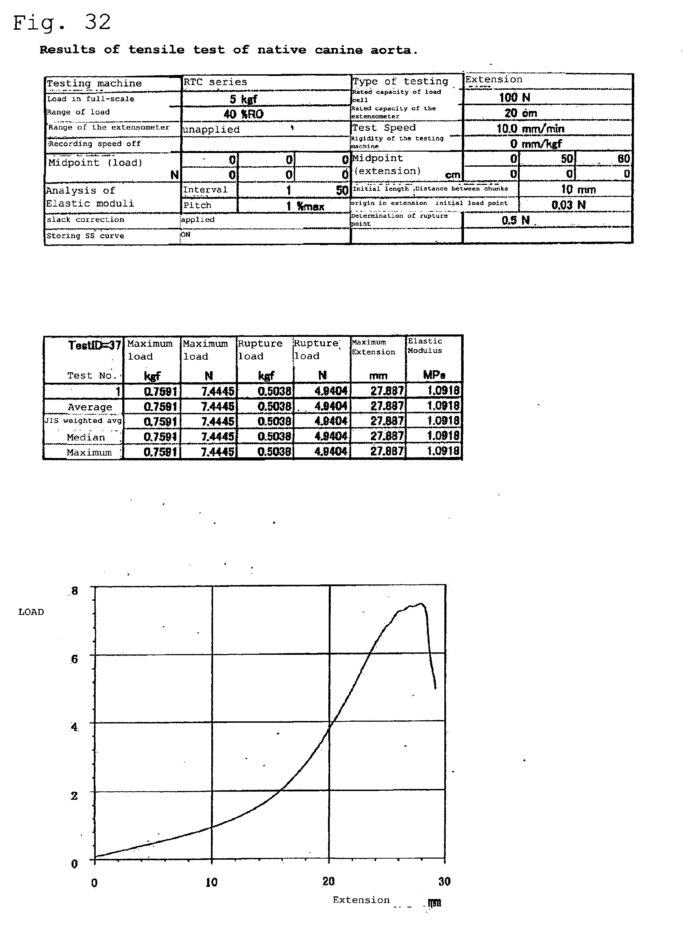

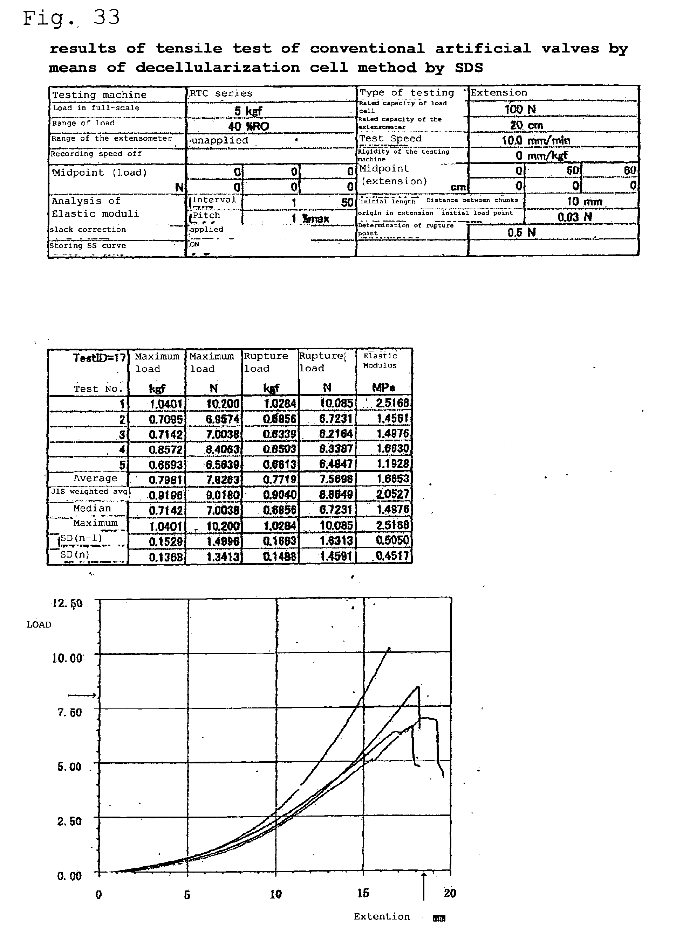

The results thereof are shown in Figures 31-33 and Table 3.

TABLE 3

| Elasticity (MPa) | Maximum point load (N) | Maximum point extension (mm) |
|---|---|---|
| Living Aorta (canine) 1.1 | 7.4 | 28 |
| SDS (Decellularization method by SDSNo. 3) 1.7 | 7.8 | 18 |
| Valve (PEG+ gamma-ray (100kGy) 2.4 | 11 | 17 |

By subjecting a decellularized tissue treated with PEG to gamma-rayirradiation, cytoplasmic components have been mostly removed. As described in Table 2, the cell residual rate may be lowered to as low as less than 1% by gamma-ray irradiation, and becomes substantially cell-free tissue.

Although reduction in mechanical properties had been expected by removal of cellular components, the decellularized tissue according to the present invention maintained extension properties (see Figures 31-33, and Table 3).

As described above, it was revealed that gamma-ray irirradiation treatment according to the present invention achieved tissue strength highter than that had been achieved by conventional decellularized tissues.

Based on the results above, typical PEG+gamma-ray irradiation protocols are given hereinbelow:

1) Porcine aortic basal portions are placed in PEG solution (concentration 80%, molecular weight 1000) and agitated (agitation condition: period of sevendays at 300RPM).

2) gamma ray irradiation (condition: in the atmosphere, room temperature, 100kGy)

3) Washing in physiological saline + antibiotics (24 hours)

4) Treatment with DNase (condition: concentration 70units/ml, room temperature, for 48 hours amongst which first 12 hours are under pressue of 500kPa, and the latter 36 hours at 150RPM) .

5) Wash and agitation in physiological saline + antibiotics.

(Example 2: Comparison of reactions within biological tissue)

(Method)

(Immunological response)

Aorta wall portions (1×1 cm) of a gamma-ray irradiated and decellularization treated valve according to Example 1 and an SDS-treated decellularization tissue (the artificial valve prepared by SDS decellularization III) according to Example 1 were implanted under the skins of the dorsal portions of SD rats. After one week and two mothds, the animals were sacrificed. The degree of inflammatory cellular infiltration was scored for evaluation. In this example, porcine native valves was used as controls for comparison.

(Calcification)

The specimens were collected one week and two months after subcutaneous implantation, followed by von Kossa staining for evaluation of calcification. Also, Ca concentration within the tissue was measured with an atomic absorption spectrometry. The Ca concentration was measured and quantified as follows. The tissue was placed in concentrated hydrochloric acid (or concentrated acid) and was dissolved while heating. Thereafter, atomic absorption spectrometry was performed. The solution was diluted and sprayed into high temperature plasma. Element-specific absorption wavelengths of spectra generated in combustion were measured (Ca: 393.366 nm).
von Kossa staining is conducted as follows: optionally conduct deparaffin treatment (for example, with pure ethanol), wash in water (distilled water) and immerse the sample in 25% silver nitrate (under indirect light) for two hours. Thereafter, wash with distilled water, and immerse the sample in 42% sodium thiosulfate (hypo) for five minutes. Thereafter, wash in running water for five minutes, and then nucleus fast Red for five minutes. The sample is washed with running water for 5 min, followed by dehydration, clearing, and mounting.
Aorta wall portions (10×10 mm) of decellularization treated porcine aortic valves were implanted under the skins of the dorsal portions of SD rats (250 g). After two months, the specimens were collected. Ca concentration (Ca content per dry weight) within the tissue was measured with an atomic absorption spectrometry (SPS7800: Seiko Instrument Inc.) (see, Ozaki S., "Pathophysiology of Calcification of Bioprosthetic Heart Valves", Acta Biomedical Lovaniensia, 2001).

(Implantation)

A portion of aorta wall tissue treated by the first generation decellularization process was implanted to dog descending aortas.

(Results)

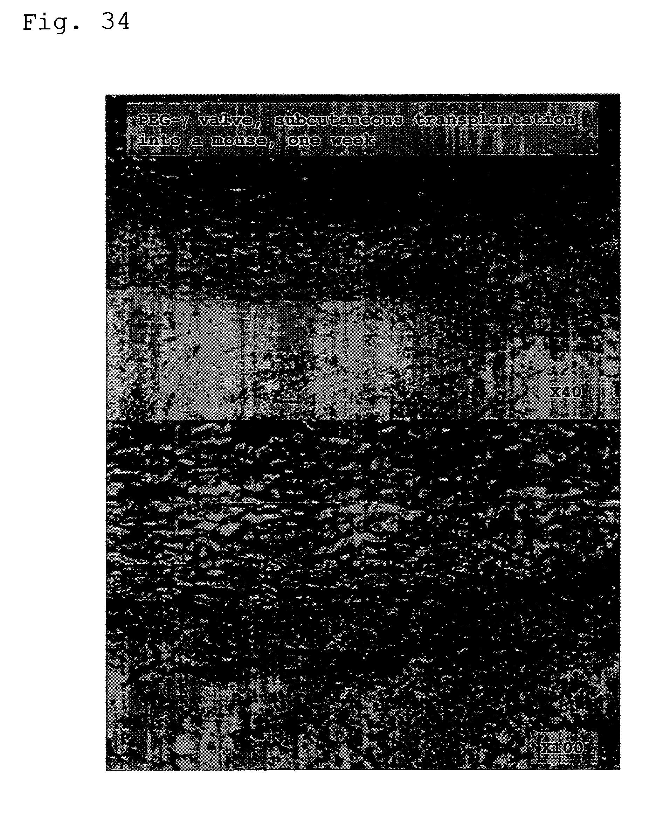

Results of one week after the subcutaneous implantation are shown in Figure 34. As seen from Figure 34, it was found that: no inflammatory cellular infiltration was observed. On the other hand, inflammatory cellular infiltration was observed in a plurality of layers in the porcine native valve; and in the SDS-treated valve, substantially no cellular infiltration was observed, only a slight inflammatory reaction was observed, and the whole tissue structure was not impaired.
Next, the results of evaluation of calcification can be performed for the decellularization tissue of the present invention.
Ca concentration within the tissue was measured with an atomic absorption spectrometry.
In the experiment in which a portion of aorta wall tissue of the above-described first generation (SDS, or NP-40 treatment) valve was implanted into dog descending aortas, one dog died of graft rupture two weeks after implantation. Another dog survived until autopsy. All dogs implanted with a valve treated with PEG/DNaseI subjected to gamma-ray irradiation according to the present invention survived for more than one month. A similar experiment may be conducted and valve samples may be excised two weeks after implantation.

(Example 3: Confirmation of cell replacement)

Comparison of Reactions in Biological Tissue between each Valve
Decellularization treated porcine forearm arteries according to Example 1 are implanted into dog femoral aortas. The animals are sacrificed after 10 days. The degree of inflammatory cellular infiltration is compared and studied.

(Results)

It is found that there is substantially no rejection reaction in the decellularized tissue of the present invention, and thus it is understood that global structure is not impaired. Further, by observing the implanted tissue, it is also possible to confirm that the decellularized tissue is replaced with self cells.

(Example 4: Decellularization of pericardia)

Next, pericardia are removed from swine and treated with a surfactant solution or a polyethylene glycol solution as described in Example 1. The cell residual rate and tissue damage rate of the treated pericardia are measured. As a result, decellularized pericardia having a tissue damage rate of less than 30% and a cell residual rate of less than 5% are obtained.

The pericardia are implanted to SD rats as described in Example 2 and were examined for inflammatory cellular infiltration and calcification. Decellularized tissue and control tissue are implanted to 3-week old rats, followed by monitoring for 60 days. After excision of the tissue, samples are collected for histological evaluation. The samples are stained by hematoxylin-eosin (H&E) staining for evaluation of the whole structure.

Next, this decellularized pericardium was implanted into rat cardiac infarct.

(Cardiac infarct rat model)

Lewis male rats are used in this example. The animals are cared for in compliance with the spirit of animal protection in accordance with "Principles of Laboratory Animal Care" prepared by National Society for Medical Research and "Guide for the Care and Use of Laboratory Animals" (NIH Publication No. 86-23, 1985 revised) prepared by Institute of Laboratory Animal Resource and published by National Intitute of Health.

Acute cardiac infarction is induced as described in Weisman H. F., Bush D. E., Mannisi J. A., et al. "Cellular mechanism of myocardial infarct expansion", Circulation, 1988;78:186-201. Briefly, rats (300 g, 8 weeks old) are anesthetized with pentobarbitol, and ventilated by positive pressure breathing. In order to produce rat cardiac infarction models, the chest is opened via the left 4th intercostal and the left coronary artery is ligated at a distance of 3 mm from the root thereof with 8-0 polypropylene thread.

(Implantation)

Recipient rats are anesthetized. The chest is opened via the left 5th intercostals to expose the heart. The rats are divided into two groups, depending on the substance which was administered into a cardiac infarct region of the rat, i.e, group C (no treatment, n=5) and group S (implanted with decellularized pericardium tissue, n=5). The decellularized pericardium is directly implanted into the infarct site two weeks after ligation of the left anterior descending branch.

(Measurement of function of rat heart)

Heart functions are measured by echocardiography (SONOS 5500, produced by Agilent Technologies) 2 weeks, 4 weeks, and 8 weeks after production of the infarction model. Minor axis images are drawn at positions where the left ventricle has the greatest diameter when viewed from the left, using a 12-MHz transducer. In the B-mode, the end systolic area of the left ventricle is measured. In the M-mode, the end diastolic diameter of the left ventricle (LVDd), the end systolic diameter of the left ventricle (LVDs), and the anterior wall thickness of the left ventricle (LVAWTh) are measured to obtain LVEF and LVFS.

(Histological analysis)

Eight weeks after implantation, the heart is removed. The heart is cut along a minor axis. The heart is immersed in 10% formaldehyde solution, followed by paraffin fixation. Sections are prepared, followed by hematoxylin-eosin staining and Masson trichrome staining. At the same time, the sections are frozen, followed by factor VIII immunological staining.

Thus, it is demonstrated that the decellularized tissue of the present invention can be well applicable to sites other than a site from which tissue is derived before treatment.

(Example 5: Decellularization of Dura mater)

Next, dura maters are removed from swine and were treated with a surfactant or a polyethylene glycol solution as described in Example 1. The cell residual rate and tissue damage rate of the treated duramater are measured. As a result, decellularized dura maters having a tissue damage rate of less than 30% and a cell residual rate of less than 5% are obtained.

The dura maters are implanted to Lewis rats as described in Example 2 and are examined for inflammatory cellular infiltration and calcification.

Thus, according to the method of the present invention, any tissue can be treated into decellularized tissue having similar excellent characteristics.

(Example 6)

Next, whether or not similar effects can be expressed in the decellularized tissue of blood vessel, intestinal tract, pericardium, cornea, retina, bone, and ureter. Similar decellularization experiments are conducted as in Example 1. For the enhanced decellularized tissues, similar experiments are conducted as in Examples 2-5, which allows confirmation of enhancement effects of decellularized tissues.

(Example 7: further analysis using porcine valve)

Next, a variety of protocols are applied to the preparation of porcine valves, to confirm that a variety of conditions are used to prepare decellularized tissues of the present invention and the effects attained thereby may also be attained.

Porcine aortic valve basal portion was used to conduct decellularization treatment in combination with decellularization with polyethylene glycol (PEG), an amphipathic compound, and gamma-ray irradiation (100kGy). Transplantation method was conducted for HBD dog (20kg) under general anesthesia and the breast was opened and transplantation performed to the descending aorta.

In all the nine examples in total, recovery soon after the operation was observed. Five amongst thereof, histological study was performed for each on Days 8, 9, 11, 11 and 30.

(Tissue staining method)

A. HE staining

Optionally, conduct deparaffin treatment (e.g. with pure ethanol), wash with water and soak the sample in hematoxylin from Omni therein for ten minutes. Thereafter, wash in running water. Use ammonia water to develop color for thirty seconds. Thereafter, wash in running water for five minutes, stain for two minutes using eosin hydrochloride ten-fold diluted solution, dehydrate, perform lucidification and mount.

B von Willebrand factor antibody staining Antibodies: polyclonal antibodies (anti-von Willebrand factor) (factor VIII-related antigen; rabbit, N1505) (DAKO(Kyoto, Japan))
Methods: based on the protocol for DAKO LSAB2/HRP kit (DAKO(Kyoto, Japan))

    (1) blocking reaction using endogenous peroxydase;
    (2) reaction with a primary antibody;
    (3) reaction with biotin-labeled secondary antibody;
    (4) reaction with peroxidase labeled streptoavidin;
    (5) color development of DAB; and
    (6) nucleus staining.

C. Muscle specific actin antibody staining Antibodies: Muscle actin, HHF35 (M0635) (DAKO(Kyoto, Japan) Methods: based on the protocol for DAKO LSAB2/HRP kit (DAKO(Kyoto, Japan)) (the same as above).

D. Vimentin antibody staining
Antibodies: vimentin, V9 (N1521) (DAKO(Kyoto, Japan)
Methods: based on the protocol for DAKO ENVISION/HRP kit (DAKO(Kyoto, Japan)).

    (1) blocking reaction using endogenous peroxydase;
    (2) reaction with a primary antibody;
    (3) reaction with ENVISION polymer
    (4) color development of DAB; and
    (5) nucleus staining.

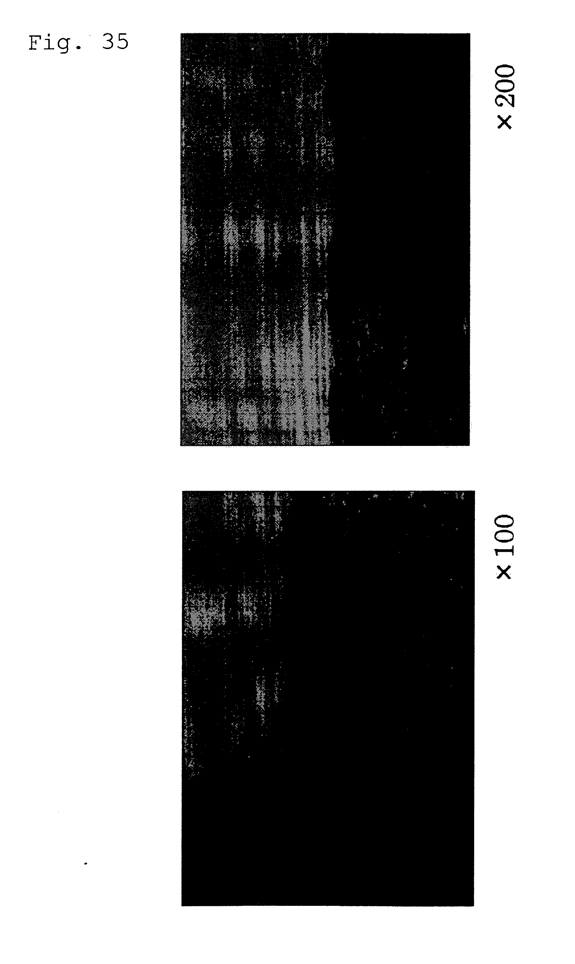

The results are shown in Figures 35 and the following figures. Figures show, after transplantation, histostaining data over long period of time (8, 9, 11 and 30 days) (HE staining, vWF antibody staining, muscle specific actin antibody staining and vimentin antibody staining).

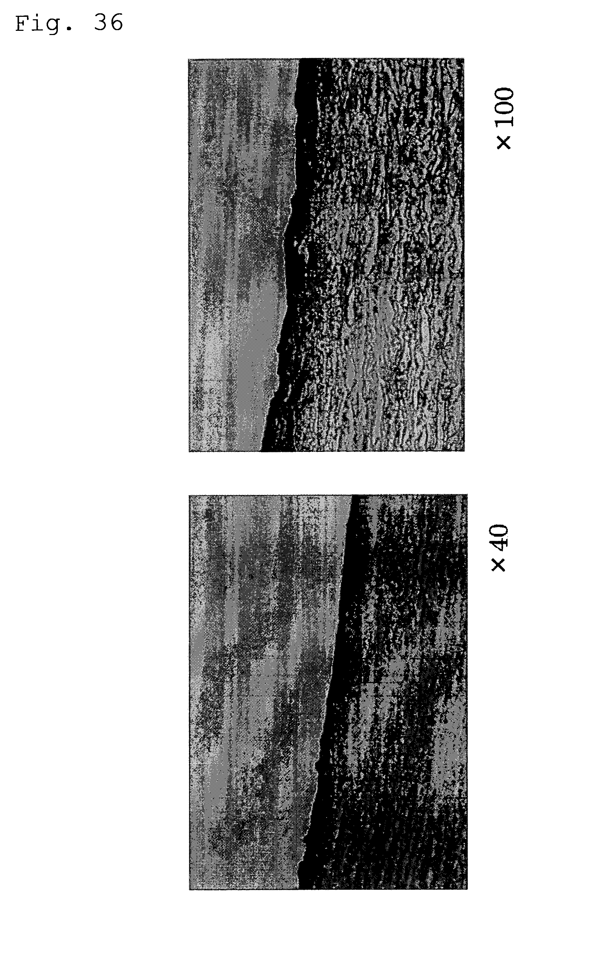

Figure 35 depicts results (HE staining) of eleven days after the treatment of: treatment method: PEG+gamma ray (100kGy); transplantation target: HBD (HB dog) (20kg) (descending aorta). Cytoplasm has been stained by HE to find no cells, and thus no abnormality of fiber rupture, and therefore no rejection reaction has been recognized. Accordingly, it can conclude that no early rejection reaction was raised. With respect to the same subject, as shown in Figure 36, vascular endogenous cells were stained by vWF antibody staining, vascular endogenous cells were further observed. Accordingly, endogenation accompanied by recellularization has been confirmed.

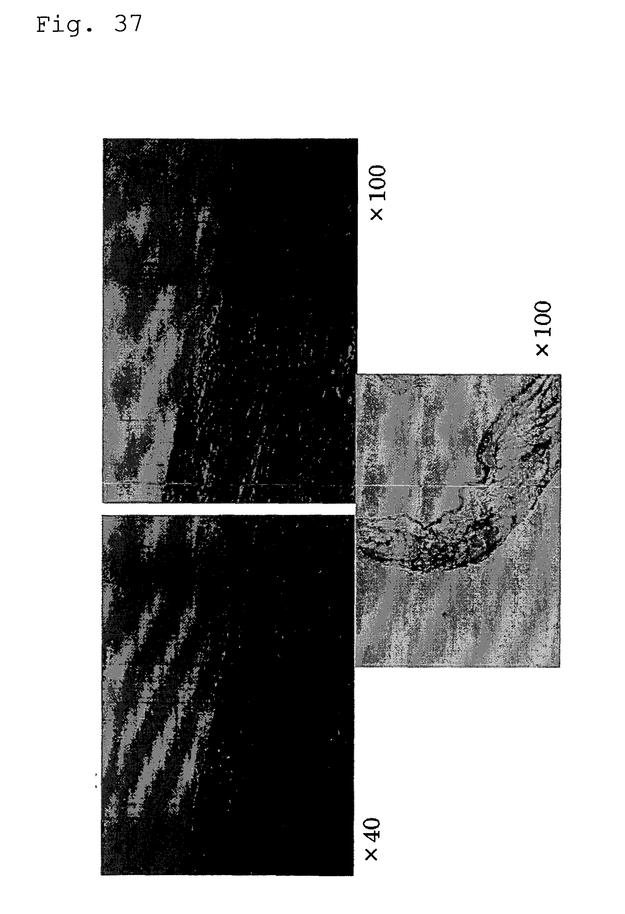

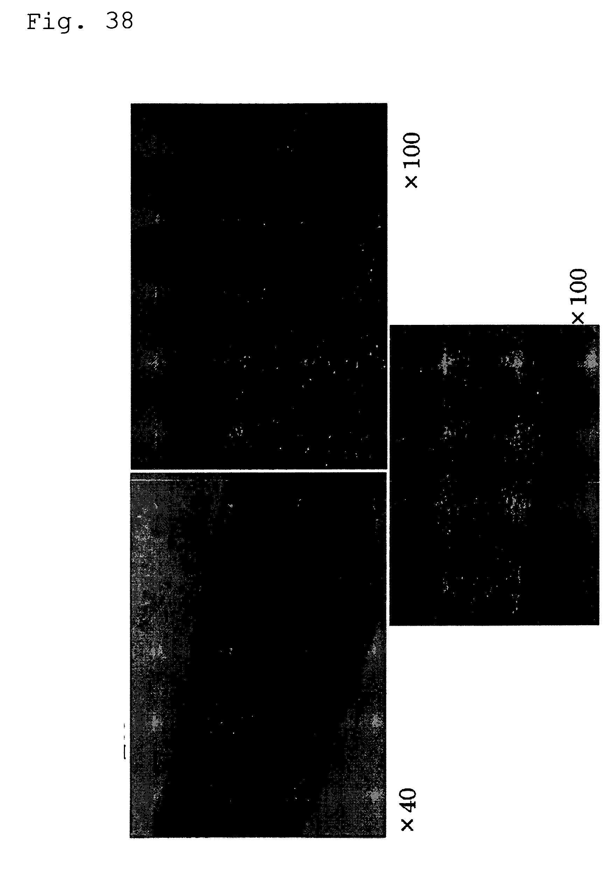

Figure 37 depicts results (HE staining) of eight days after the treatment of: treatment method: PEG+gamma ray (100kGy); transplantation target: HBD (HB dog) (20kg) (descending aorta) with respect to another subject. Figure 38 depicts results (HE staining) of nine days after the treatment of: treatment method: PEG+gamma ray (100kGy); transplantation target: HBD (HB dog) (20kg) (descending aorta) with respect to a different subject. Each cytoplasm was stained with HE staining, no cell was observed and thus no abnormality of fiber rupture, and therefore no rejection reaction has been recognized. Accordingly, it can br concluded that no early rejection reaction was raised.

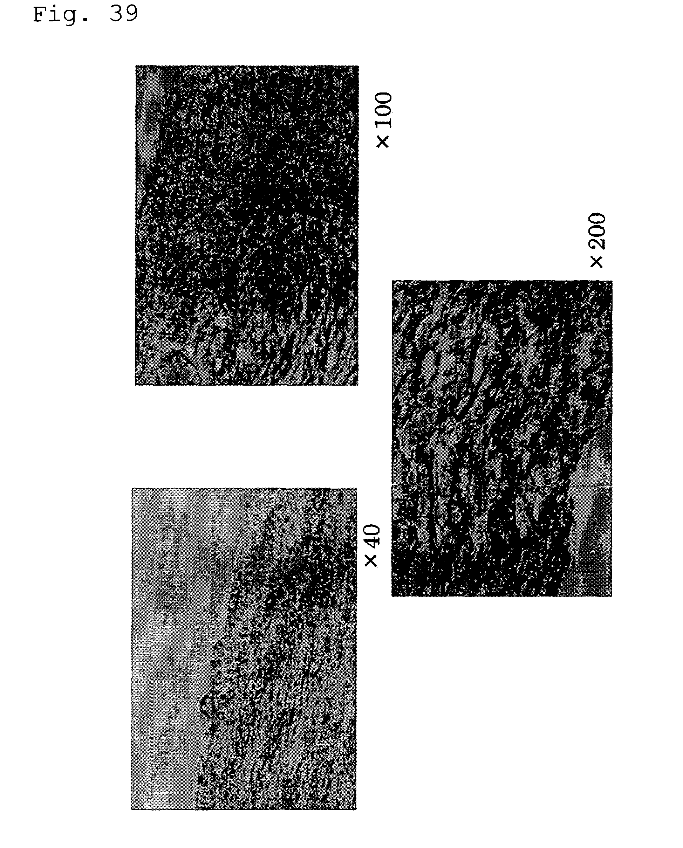

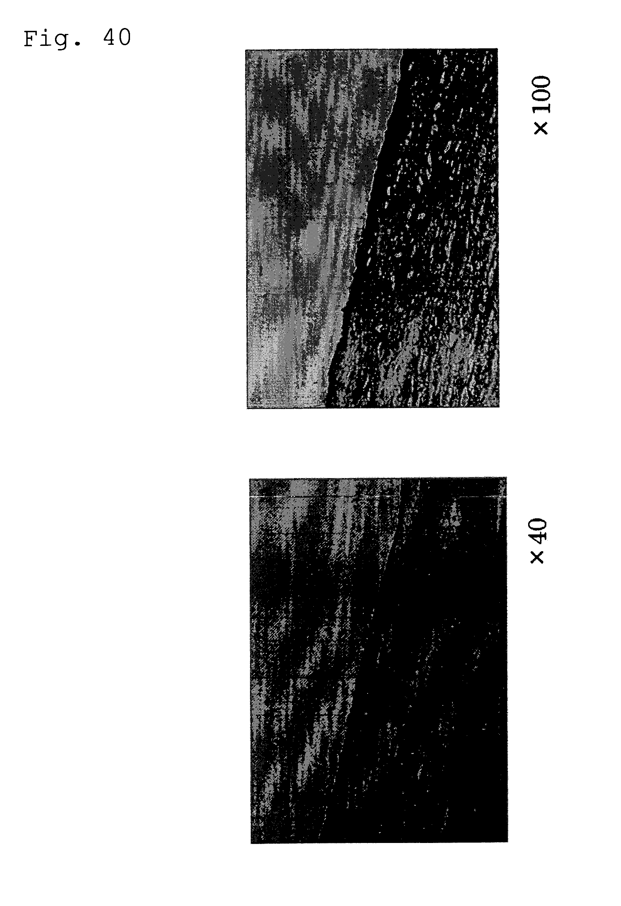

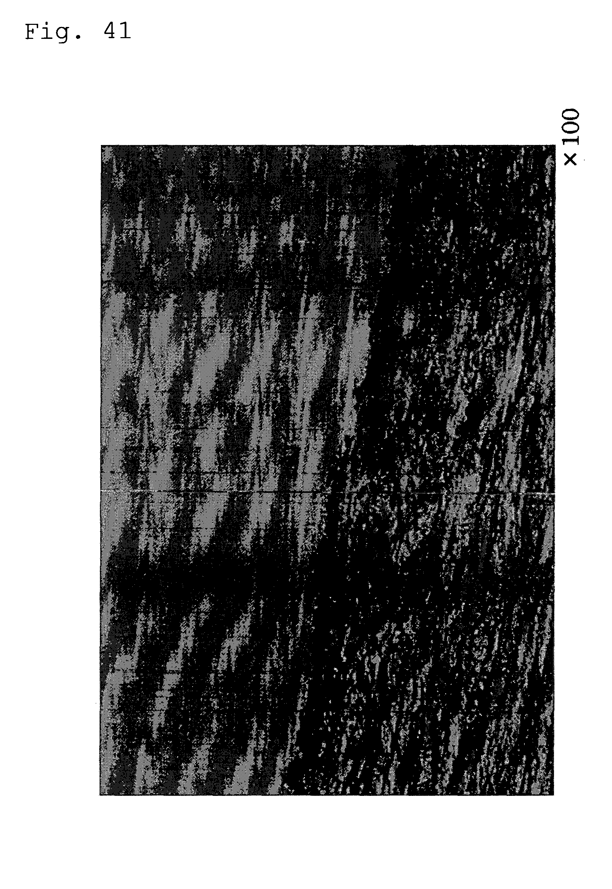

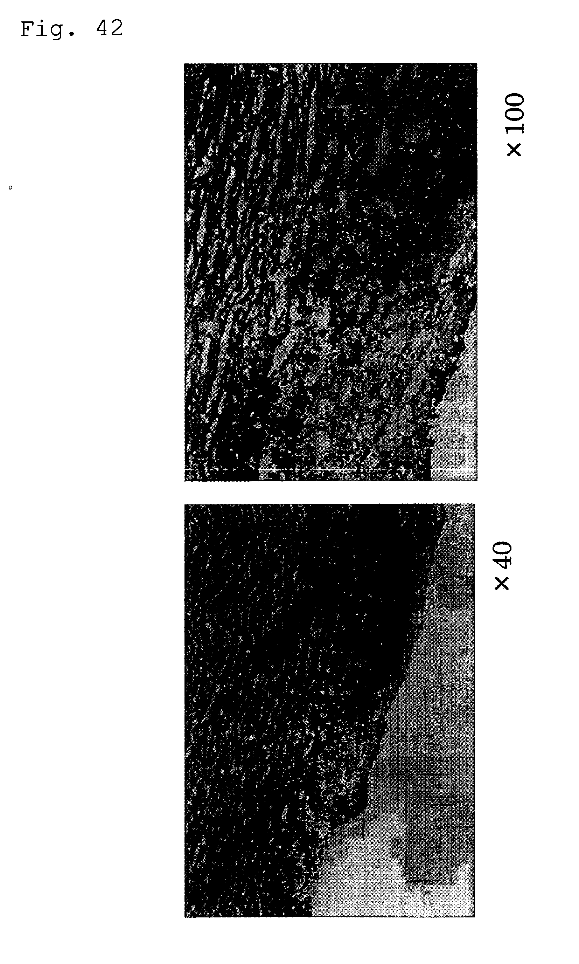

Figure 39 depicts results (HE staining) of thirty days after the treatment of: treatment method: PEG+gamma ray (100kGy); transplantation target: HBD (HB dog) (20kg) (descending aorta) with respect to another subject. Cytoplasm has been stained with HE to find no cells, and thus no abnormality of fiber rupture, and therefore no rejection reaction has been recognized. A portion of the tissue was recognized to suggest recellularization. With respect to the present subject, as shown in Figure 40, when the vascular endogenous cell was stained with vWF antibody staining, more uniform endogenous cells hae been further observed. Accordingly, recellularization is observed accompanied by endogenation. With respect to the present subject, as described in Figure 41, when observing reaction with muscle-specific actin antibody, it was found negative. Further, the present subject was stained for vimentin (Figure 42), the sample was positive for vimentin antibody. Accordingly, it is believed that recellularization occurred due to mesenchymal undifferentiated cells (cells capable of becoming smooth muscle or fibroblast cells in future) rather than highly differentiated cells which have been matured such as smooth muscle cells.

As described in the figures, at Day 30 after the transplantation, the decellularized tissue according to the present invention has (1) no recognition of fiber rupture, (2) no recognition of inflammatory reaction, and (3) recellularization has been partially recognized.

As a form of recellularization, (A) endogenation of external layer by vascular endogenous cells; and (B) recellularization by means of mesenchymal undifferentiated cells (cells capable of becoming smooth muscle or fibroblast cells in future) rather than differentiated cells which have been matured such as smooth muscle cells.

(Example 8: Further analysis using bovine pericardium)

Next, the present Example demonstrated that the present invention can be generally used using bovine pericaridium as a different tissue. The treatment procedures therefor are described hereinbelow:

A. Decellularization treatment

(1) DNase treatment

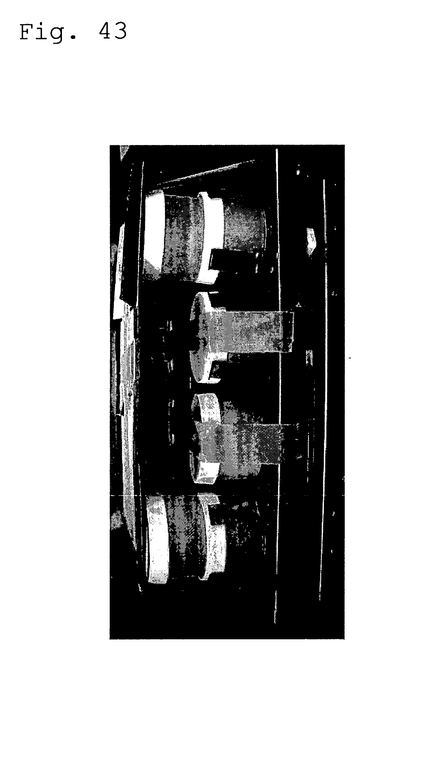

* The sample was soaked in treatment solution A (50ml), and rotation was performed at 37° C for 24 hours, at 150 revolutions/min in Bio-Shaker BR-300LF (TAITEC) (Figure 43).
* Treatment solution A was prepared by dissolving the following in PBS:

- DNase I 70U/ml(TaKaRa)
- 100U/ml penicillin 100$\mu$g/ml streptomycin (GIBCO)
- 1.25$\mu$g/ml amphotericin B (SIGMA)
- 5mM MgCl$_2$·6H$_2$O
- ·kanamycin 100mg/l (SIGMA).

(2) Gamma-ray irirradiation was performed using similar protocols as described abovein the Examples, and irradiation doses of 5kGy, 15kGy, 25kGy and 50kGy have been tested.

(3)PEG treatment: The sample was immersed in Treatment solution C (500ml) and rotation was conducted at 25° C for one week, at 200 revolutions/min in Bio-Shaker BR-300LF (TAITEC)(Figure 43).
Treatment Solution C was prepared by dissolving the following in PBS:

\* 80 %w/w Polyethylene Glycol(PEG): molecular weight 1000 (Nakalai Tesque)

- 100U/ml penicillin 100μg/ml and streptomycin (GIBCO)
- 1.25μg/ml amphotericin B (SIGMA)
- kanamycin 100mg/l (SIGMA).

(4) Wash

The sample was immersed in Treatment Solution D (500ml), and rotation was performed at 25° C for 24 or 5 hours, at 200 revolutions/min in Bio-Shaker BR-300LF (TAITEC) (Figure 43)

\* Treatment Solution D was prepared by dissolving the following in PBS:

- 100U/ml penicillin 100μg/ml and streptomycin (GIBCO)
- 1.25μg/ml amphotericin B (SIGMA)
- kanamycin 100mg/l (SIGMA).

B. Quantification of Remaining DNA

Apparatus used: biolumin960(Molecular Dynamics)

Reagent preparation:

(a)papain solution: ···55mM sodium citrate, 150mM sodium chloride, 5mM cystein chloride, 5mM EDTA were prepared in milliQ water for use.

↓

Just prior to use papain was added (0.56U/ml papain solution)

(b) TNE solution:···10mM Tris, 2M NaCl, 1mM EDTA were prepared and pH was adjusted to 7.4.

(c) Hoechst 33258 solution ··· prepared using TNF solution at 1μg/ml.

(d) human genome DNA solution ··· prepared using TNF solution at 1μg/ml.

Methods:

1. Frozen-dried sample was weighed for 10mg and placed into an Eppendorf tube.
2. Papain solution (1ml) was added thereto, and heat treated at 60 ° C for 24 hours.
3. Mixed by pipetting and centrifuged for five minutes at 1000rpm.
4. Diluted for 80 times using TNE solution.
5. Diluted solution (500μl) was mixed with Hoechst(1μg/ml; 500μl) and thereafter lightly centrifuged.
6. Applied the samples (300μl aliquot) to 96 plates for fluorescence.
7. Fluorescence was measure at 340-450nm (human genomic DNA solution was used for calibration curve to calculate theoretical values).

C. Quantification of remaining protein amount **APPARATUSES USED:**

Mixer Mill MM 300 multispecimen cell rupture apparatus (QIAGEN)

**REAGENTS:**

(a) protein extraction solution

Wako Tris(hydroxymethyl)aminomethane 204-07885 25mM

Wako glycin 077-00735 192mM

Wako sodium dodecyl sulfate 191-07145 0.1%

SIGMA protease inhibitor cocktail P2714.

**METHODS:**

1. The sample was minced lightly and freeze-dried.
2. The tissue was weighed for 20mg and placed in an Eppendorf tube.
3. Protein extraction solution (500μl) is placed in an Eppendorf tube, a stainless bead is placed therein and rapid oscillation (five minutes, 30/s) is conducted, followed by use of Mixer Mill MM 300 to disintegrate tissue.

4. Centrifuged lightly and let it stand for 72 hours at 4 °C.軽5. To a 96-well plate was added the extracted protein solution (20μl), and 200μl of Bio-RAD Protein assay (5 fold dilution) were added and absorbance (at 595nm) was measured (bovine serum albumin (SIGMA) was used to produce calibration line for calculating theoretical values).

D. Quantification of calcium

1. A sample (10 mg) was weighed.
2. 6M HCl was added to the sample for 50 μl per ml sample.
3. The sample was incubated at 85°C for 48 hours.
4. Equal volume of 6M NaOH (500 μl) was added thereto.
5. An atomic absorption photometer was used to measure calcium concentration.

E. Detection of a-Gal epitope
**REAGENTS:**

(a) Protein Block: Protein Block Serum-Free (DakoCytomation)
(b) aqueous mounting agent: GEL/MOUNT(biomeda)
(c) GSL1B4: used at x100 dilution
(product name)Griffonia simplicifolia Isolectin GS-IB4-Saporin
<B4 Lectin-Saporin><IB4-SAP>
(manufacturer) Advanced Targeting Systems.

**METHODS:**

1. A slice of 5μm width was produced and dried.
2. The slice was incubated with acetone at room temperature for ten minutes.
3. Drying was performed for five minutes.
4. The slice was incubated for ten minutes with Protein Block.
5. Protein Block was washed off and the slice was immersed in GSL1B4. The slice was incubated at 37° C for 60 minutes.
6. The slice was washed with PBS(-) for ten times X 3.
7. The slice was mounted with an aqueous mounting agent, and stored under dark.

(Study of gamma-ray treatment conditions)

Each of the following items was investigated as to the extent they have effects on the final product.

(i) gamma-ray irradiation (5, 15, 25 and 50kGy)
(ii) timing of gamma-ray irradiation (before or after the PEG treatment)

Evaluation indices are as follows:

(a) investigation of remaining cells
(b) investigation of remaining DNA
(c) investigation of remaining proteins
(d) investigation with TEM
(e) investigation by tension test
(f) investigation of immunological reaction after transplantation
(g) invenstigation of calcification after transplantation

(a) through (c) are for investigating decellularization, (d) is conducted by a microscope. (e) is for investigating strength, and (f) through (g) are directed to investigation of performance of the tissue. Accordingly, (a) through (e) are conducted *in vitro,* while (f) and (g) are conducted *in vivo.*

(2) Evaluation of alpha-Gal epitope removal capability by means of gamma-ray irradiation

It was investigated whether or not alpha-Gal epitope, a major heteroantigen for rejection response, can be disrupted and removed by means of gamma-ray irradiation (25kGy and 100kGy).

(Results)

(Investigation of gamma-ray treatment conditions)

(i) gamma-ray irradiation (5kGy, 15kGy, 25kGy and 50kGy) and (ii) the timing of gamma-ray irradiation (before and after the PEG treatment), were investigated.

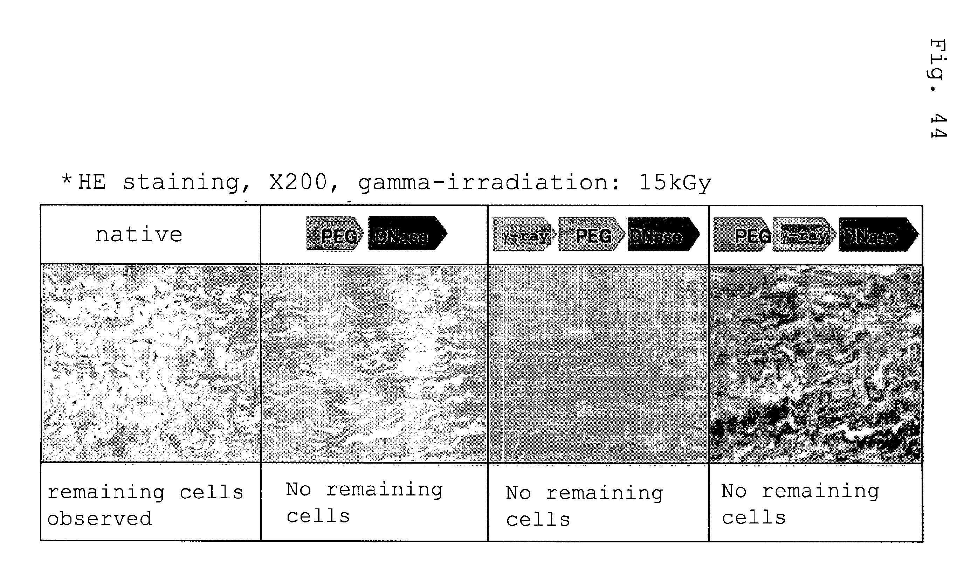

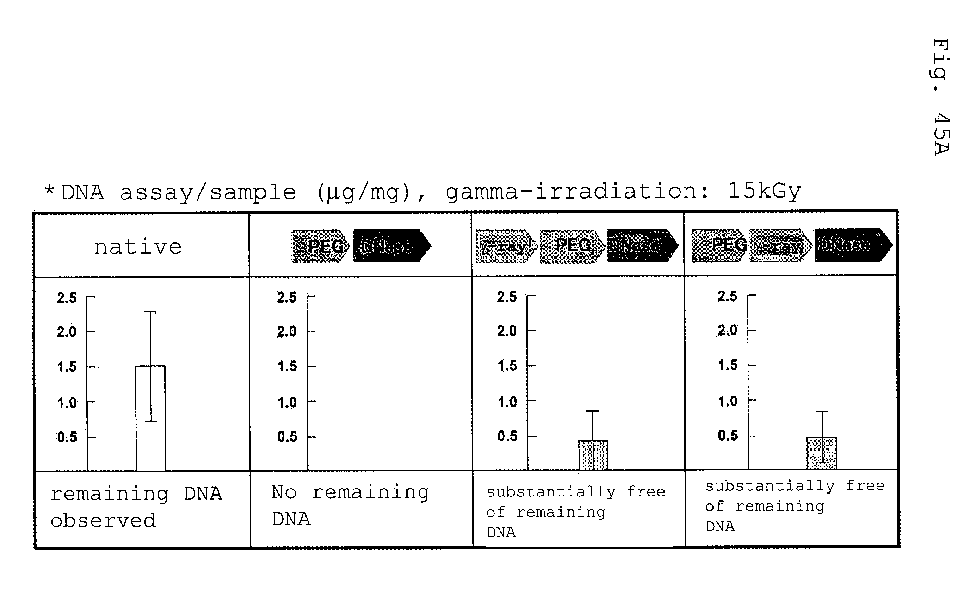

Figure **44** depicts results of investigation of remaining cells. Figure **44** depicts, from the left, the results of native (without treatment), DNase treatment after PEG treatment, an example of PEG and DNase treatments after exposure to gamma-ray irradiation, and an example of gamma-ray irradiation which was conducted after the PEG treatment but prior to the DNase treatment. As seen from the figure, no remaining cells have been observed in any of those with PEG+DNase treatments and PEG+DNase+gamma-ray irradiation treatments.

Figure **45A** depicts the results of investigation of remaining DNA. Figure **45A** depicts, from the left, the results of native (without treatment), DNase treatment after PEG treatment, an example of PEG and DNase treatments after exposure to gamma-ray irradiation, and an example of gamma-ray irradiation which was conducted after the PEG treatment but prior to the DNase treatment. As seen from the figure, no remaining cells have been observed in any of those with PEG+DNase treatments and PEG+DNase+gamma-ray irradiation treatments.

Figure **45B** depicts the results of investigation of remaining DNA of the decellularized tissue of the present invention used in Example 8. Figure **45B** depicts, from the left, the results of native (without treatment), DNase treatment after PEG treatment, an example of PEG and DNase treatments after exposure to gamma-ray irradiation, and an example of gamma-ray irradiation which was conducted after the PEG treatment but prior to the DNase treatment. It was observed that DNA remained in an example of gamma-ray irradiation + DNase treatment, but remaining DNA is significantly reduced in the example of PEG + DNase + gamma-ray irradiation treatment.

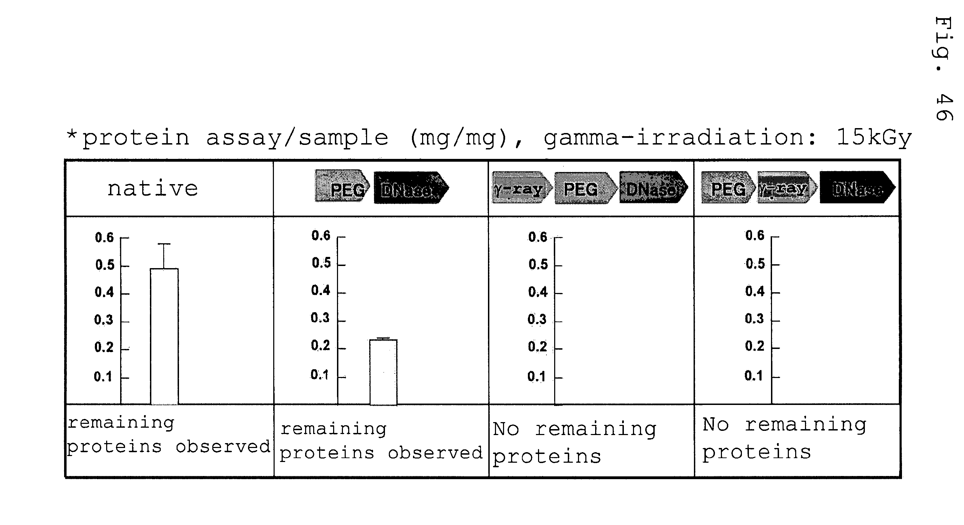

Figure **46** depicts the results of investigation of remaining protein. Figure **46** depicts, from the left, the results of native (without treatment), DNase treatment after PEG treatment, an example of PEG and DNase treatments after exposure to gamma-ray irradiation, and an example of gamma-ray irradiation which was conducted after the PEG treatment but prior to the DNase treatment. Remaining protein was obsrved for the sample with PEG+DNase treatment, while in the sample with gamma-ray irradiation in addition thereto, no remaining protein was observed.

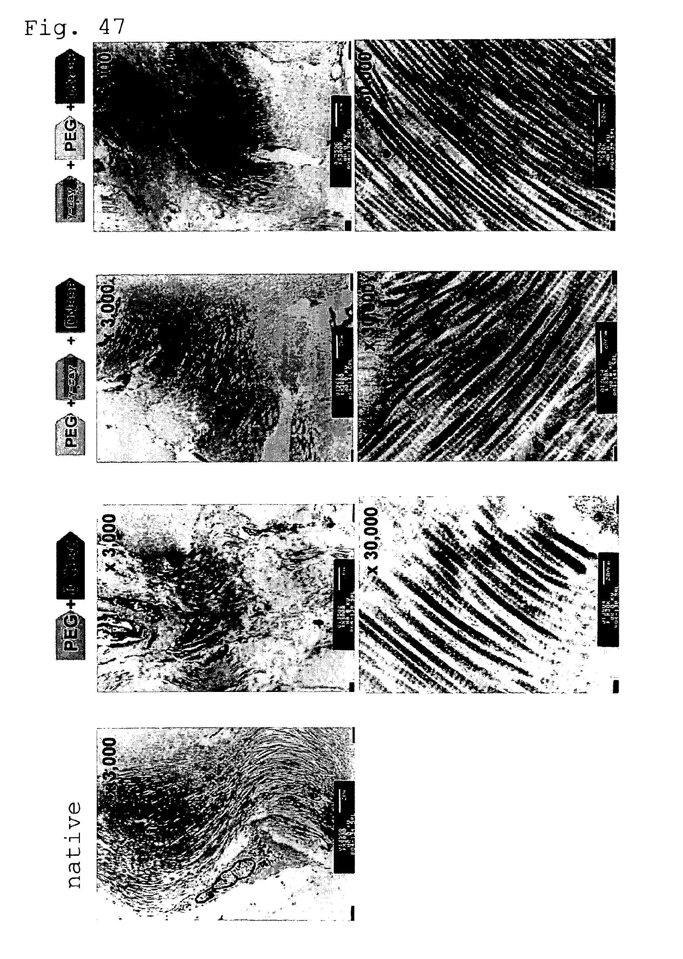

Figure **47** depicts the results of investigation of the decellularized tissue of the present invention with TEM (transmission electron microscope). Figure **47** depicts, from the left, the results of native (without treatment). DNase treatment after PEG treatment, an example of PEG and DNase treatments after exposure to gamma-ray irradiation, and an example of gamma-ray irradiation which was conducted after the PEG treatment but prior to the DNase treatment. Upper panel shows x3000 magnification, and the lower panel shows x 30,000 magnification. No fiber rupture or the like was observed for any samples with any particular treatment.

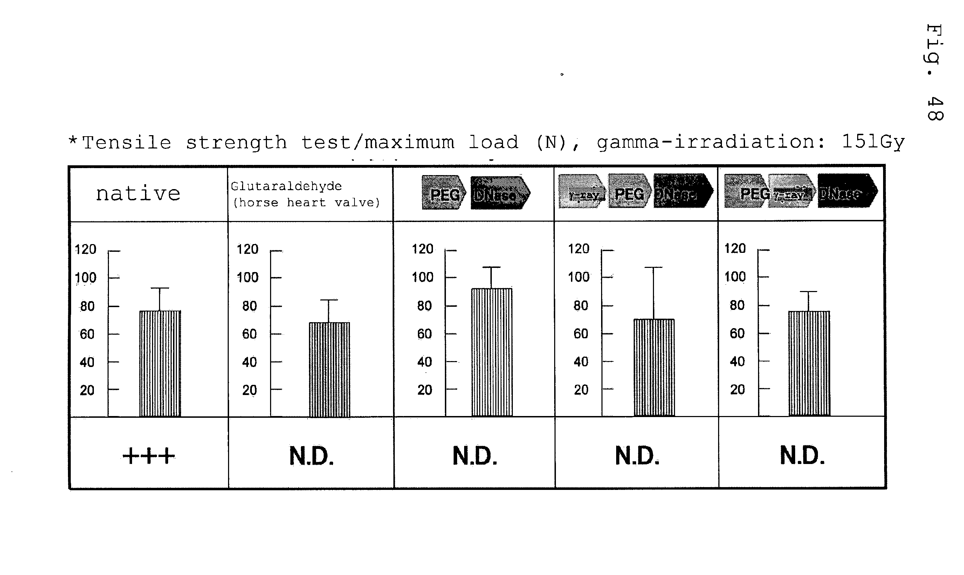

Figure **48** depicts the results of investigation of the tissue strength by measuring tensile strength thereof. Figure **48** depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment. No samples with any particular treatments were observed to reduce the strength thereof.

Figure **49** depicts the results of investigation of the immunological rejection reaction. Tissue with gamma-ray irradiation of 15 kGy was investigated by means of HE staining one week after the transplantation. Figure **49** depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment. At one week after the transplantation, no immunological rejection reaction was observed for either of those with PEG+DNase treatment and PEG+DNase+gamma-ray irradiation treatment.

Figure **50** depicts the results of investigation of the immunological rejection reaction for long transplantation. Tissue with gamma-ray irradiation of 15 kGy was investigated by means of HE staining two months after the transplantation. Figure **50** depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment. At two months after the transplantation, no immunological rejection reaction was observed for either of those with PEG+DNase treatment and PEG+DNase+gamma-ray irradiation treatment.

Figure **51** depicts the results of investigation of the calcification. Tissue with gamma ray irradiation of 15 kGy was investigated by means of von Kossa staining two months after the transplantation. Figure **51** depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment. At two months after the transplantation, no calcification rejection reaction was observed for either of those with PEG+DNase treatment and PEG+DNase+gamma-ray irradiation treatment.

Figure **52** depicts the results of investigation of the calcification by means of calcium content measurement. Tissue with gamma ray irradiation of 15 kGy was investigated (calcium/sample <mg/mg>) two months after the transplantation. Figure **52** depicts, from the left, the results of native (without treatment), glutaraldehyde treatment (horse heart valve), DNase treatment after PEG treatment, an example of PEG and DNase treatments after exposure to gamma-ray irradiation, and an example of gamma-ray irradiation which was conducted after the PEG treatment but prior to the DNase treatment. At two months after the transplantation, no calcification rejection reaction was observed for either of those with PEG+DNase treatment and PEG+DNase+gamma-ray irradiation treatment.

From the above-description, compared to the sample with PEG+DNase treatments, those with PEG+DNase+gamma-ray irradiation treatments have shown significantly superior effects. Timing of irirradiation of gamma-rays did not affect the effects thereof prior and after PEG treatment. In summary, the following table can be summerized as follows:

TABLE 4

|  | native | glutaral dehyde treatment horse pericardium | PEG DNa se | Method No.1 | Method No. 2 |
|---|---|---|---|---|---|
|  |  |  |  | gamma-ray irradiation→PEG→DNase | PEG→gamma-ray irradiatio n→DNase |
| remainin g cells | +++ | NA | - | - | - |
| remainin g DNA | +++ | NA | - | + | + |
| remainin g protein | +++ | NA | ++ | - | - |
| strength | +++ | NA | +++ | +++ | +++ |
| immunological reaction | +++ | + | - | - | - |
| calcification | + | +++ | + | + | + |

Next, dose of gamma-ray irradiation was varied and measurement was performed to obtain the following results:

TABLE 5

| TABLE 5 | native | glutar aldehy de treatment horse pericardium | PEG DNas e | gamma-ray irradiation→PEG →DNase | | | | PEG→gamma-ray irradiation→DNa se | | | |
|---|---|---|---|---|---|---|---|---|---|---|---|
|  |  |  |  | 5 kGy | 15 kGy | 25 kGy | 50 kGy | 5 kGy | 15 kGy | 25 kGy | 50 kGy |
| remaining cells | +++ |  | - | - | - | - | - | - | - | - | - |
| remaining DNA | +++ |  | - | + | + | - | - | ++ | + | + | + |
| remaining protein | +++ |  | ++ | + | - | + | + | + | - | + | + |
| streng th | +++ |  | +++ | +++ | +++ | +++ | ++ | +++ | +++ | +++ | ++ |
| immune logica 1 reaoti on | +++ | + | - | - | - | - | - | - | - | - | - |
| calcif icatio n | + | +++ | + | + | + | + | + | + | + | + | + |

In the samples in which PEG+DNase+gamma-ray irradiation treatments were performed, no significant effects were found due to the difference in gamma-ray irradiation dose. Accordingly, it is understood that any line dose used is useful in the practice of the present invention. Where a tissue in which strength is desired is used, preferable dose is said to be 5-25 kGy. Where DNA remnant is not desired, it may be preferable to provide dose of 15kGy or more.

(3) Evaluation of alpha-Gal epitope removal capability by means of gamma-ray irradiation

Most patients with failure in the heart, liver or kidney, which requires transplantation surgery, cannot undergo transplantation surgery due to shortage of donors who provide organs to be transplanted. Due to shortage of such organs in allograft, research directed to realization of pig xenograft which transplants a porcine organ to human. Recently, anti-alpha-Gal antibody, a native antibody present in a human, recognizes and crossreacts with alpha-gal antigen (alpha-gal epitope) largely expressed on the cellular surface of a porcine transplant, which greatly hinders zenograft transplantation adaptation in the body. This alpha-gal epitope is expressed in swine and other mammals, but not in a human. This is because alpha 1,3-galactose transferase gene, which biosynthesize this epitope, is inactivated in the evolution process of mammals (Trends in Glycoscience and Glycotechnology Vol.11 No.62 (Nov. 1999) pp.317-327).

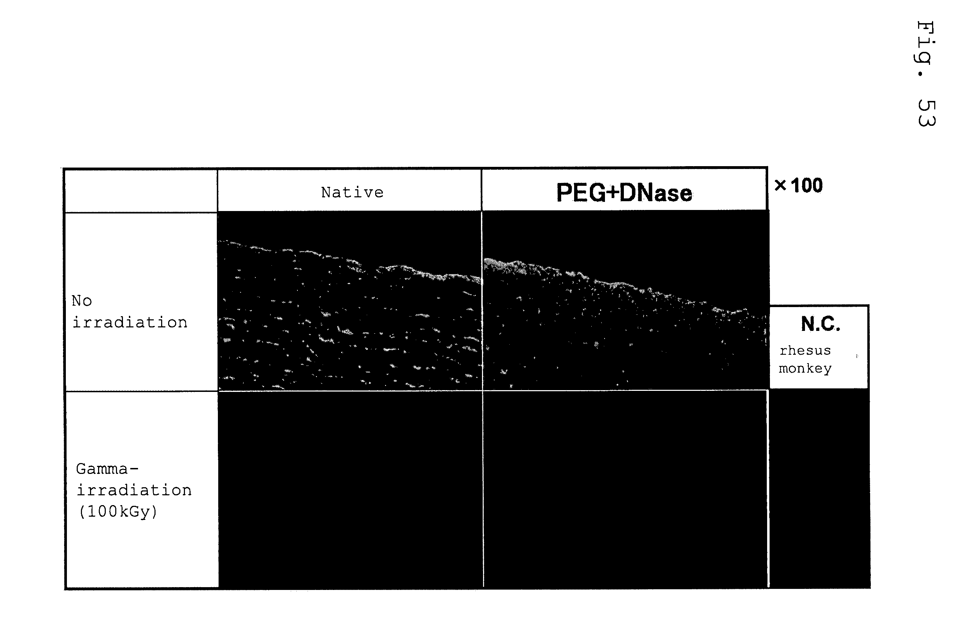

Figure 53 shows the results in which alpha-Gal epitope has been removed by means of gamma-ray irradiation. In addition to PEG+DNase treatments, gamma-ray irirradiation (100kGy) was performed, alpha-Gal epitope has been removed. As comparative control, example of macaque is shown. Detection was performed by detecting GS1B4 (al, 3Gal) epitope using a lectin.

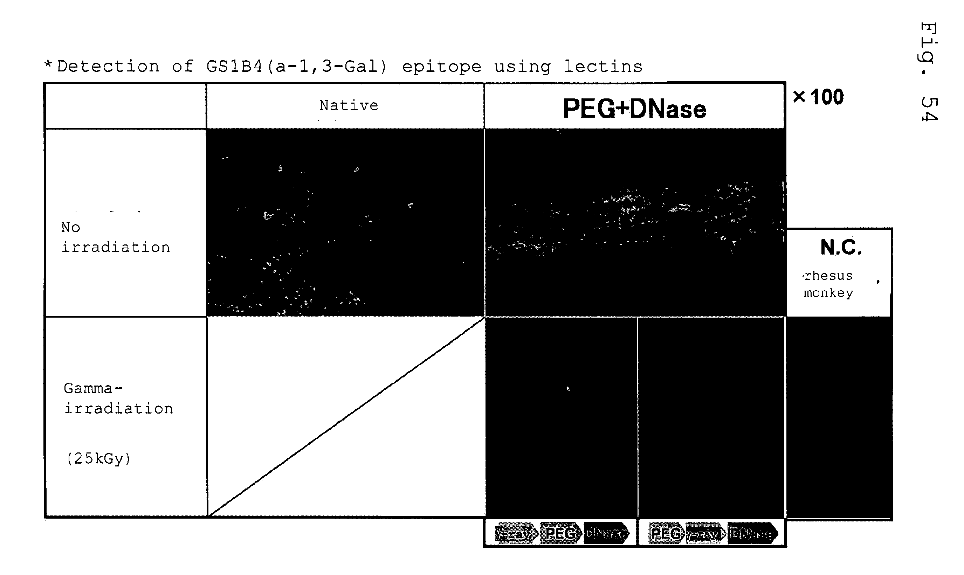

Furthermore, a lesser exposure dose by gamma-ray irradiation (25kGy) in addition to PEG+DNase treatments has also removed alpha-gamma epitope , as shown in Figure 54. In the present experiments, observation was performed by replacing the order of gamma-ray irradiation, in any case, alpha-Gal epitope has been significantly removed. As such, the removal effects of alpha-GAl epitope are suggsted for any irradiation conditions.

As described above, it was demonstrated that, in the present invention, PEG+DNase+gamma-ray irradiation treatments show advantageous significant effects over that of PEG+DNase treatments.

Moreover, small differential effects have been observed in the difference between the timing of gamma-ray irradiation (before and after the PEG treatment) in the sample with PEG+DNase+gamma-ray irradiation treatments.

In addition, small differential effects have been observed in the difference in exposure dose of gamma-ray irradiation in the sample with PEG+DNase+gamma-ray irradiation treatments.

PEG+DNase only treatments cannot remove alpha-Gal epitope, while gamma-ray irradiation in addition thereto (25kGy, 100kGy) has removed alpha-Gal epitope.

Although not wishing to be bound to any theories, it is believed that from the above-mentioned Examples, samples receive radical reactions such as gamma-ray irradiation and the like to result in crosslinkage, degradation and both of radiated polymers of a variety of types (for example, proteins, nucleic acids, sugar chains, PEG and the like), and decellularization is promoted, and alpha-Gal epitope has been removed to reinforce a biological tissue, and sterilization effects are promoted.

In the present invention, preferably, decellularized tissue having desired properties is provided by practicing an appropriate combination of all the decellularization such as PEG and the like, DNase treatment using deDNAation, and radical reactions such as gamma-ray irradiation as a promoting agent. In this case, extracellular matrices are not affected, and thus no problems will occur in biocompatibility, and rather as recellularization has ben confirmed, the effects attained thereby is recognized to be significant.,

(Example 9: Effects of PERV removal in porcine valves)

Porcine aoxtic valve basal portion was used to conduct decellularization treatment in combination of decellularization using polyethylene glycol (PEG), an amphipathic compound, and gamma-ray irradiation (100kGy). Transplantation method was conducted for HBD dog (20kg) under general anesthesia and the breast was opened and transplantation was performed to the descending aorta.

PERV detection has been performed by extracting DNA from each of (1) through (4), and conducting by RT-PCR.
(1) Naive porcine valve;
(2) Decellularized porcine valve;
(3) Decellularized porcine valve, which was removed thirty days after transplantation;
(4) Decellularized porcine valve, which was removed fifty-six days after transplantation;
A. DNA extraction method:

DNeasy Tissue Kit (QIAGEN) was used according to the manufacturer's protocol.

B. RT-PCR method:

1. PCR reaction mixture was prepared as shown in the following table:¥

| | | |
|---|---|---|
| 10×PCR buffer | | 2μl |
| NTPs | 2μl | |
| MgCl$_2$ | 1μl | |
| primer (10pM) forward | | 1μl |
| primer (10μM) reverse | | 1μl |
| recombinant Taq (TaKaRa) | 0.2μl | |
| dH$_2$O | 10.8μl | |
| emplate (cDNA or DNA) | | 2.0μl |

2. PCR was performed using GeneAmp9700 sequence detection system.

<*primer sets>

- for detection of PERV
  forward primer: act aca aca cgg ctg aag gta g (SEQ ID NO: 1)
  reverse primer: ttg tcg agt ggg gtc cta ttg(SEQ ID NO: 2)
- controls
- for detection of pigGAPDH
  forward primer: ctg cac cac caa atg ctt agc(SEQ ID NO: 3) reverse primer: gcc atg cca gtg agc ttc c(SEQ ID NO: 4)
- for detection of dog GAPDH
  forward primer: gtg atg ctg gtg ctg agt atg ttg(SEQ ID NO: 5)
  reverse primer: tgg cta gag gag cca agc agt t(SEQ ID NO: 6)

<thermocycle parameter>

Stage 1 95°C/5-10 minutes
Stage 2 95°C/15 second
Stage 3 58°C/15 second
Stage 4 72°C/1 minute

Repeats the above (40-45 cycles)
72°C/7minutes
4°C(end).

(Results)

It was demonstrated that Pig-derived virus (PERV) was removed in a porcine valve treated with the combination of PEG treatment and gamma-ray treatment (100kGy), which virus has a risk of infecting with a human.

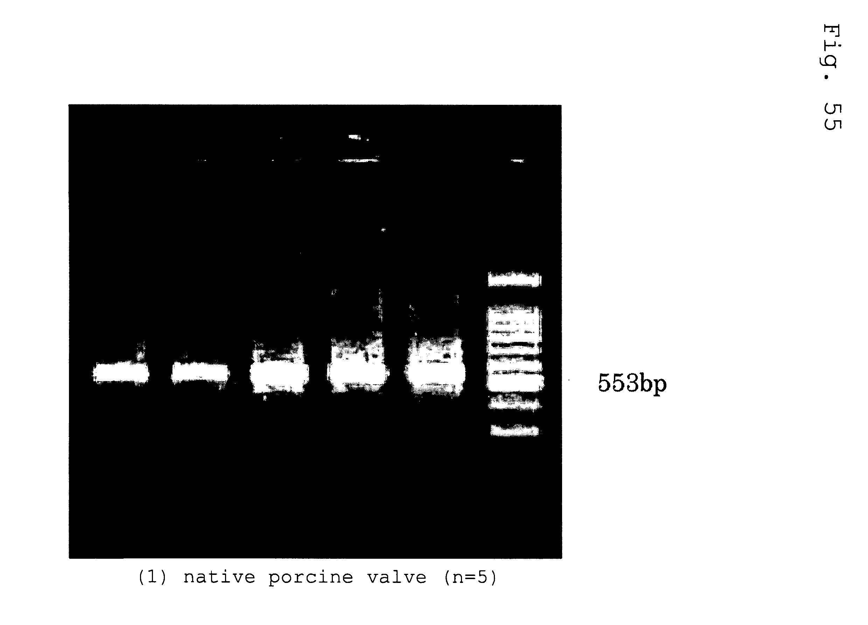

Figure 55 depicts the results of RT-PCR analysis relating to PERV for five examples of native porcine valves. As shown therein, PERV has been detected in the native porcine valves.

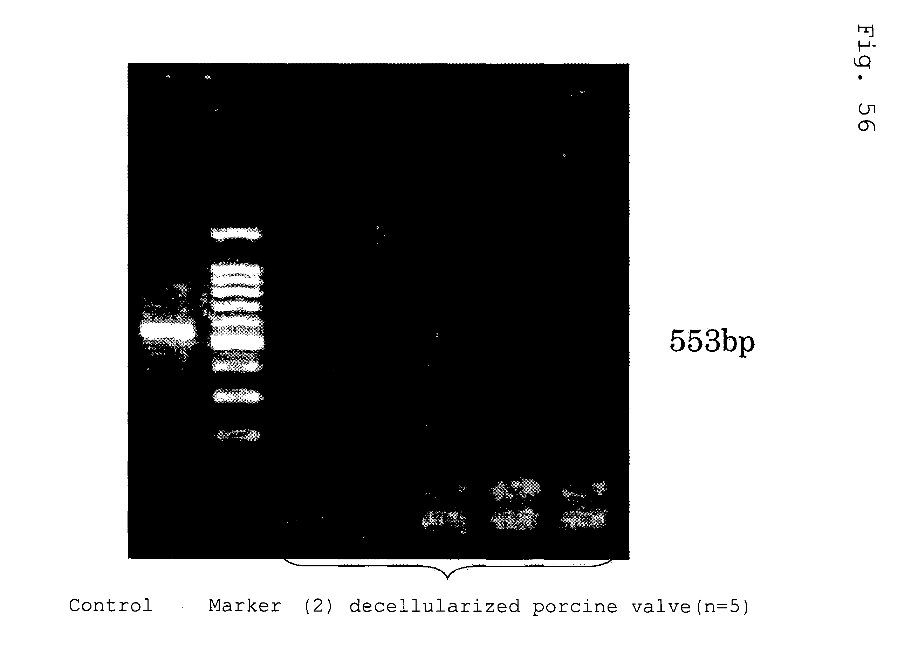

Figure 56 depicts results of RT-PCR analysis with respect to PERV for five cases of porcine valves treated with gamma-ray irradiation, and a positive control. As shown in the figure, no PERV has been detected in the decellularized porcine valve.

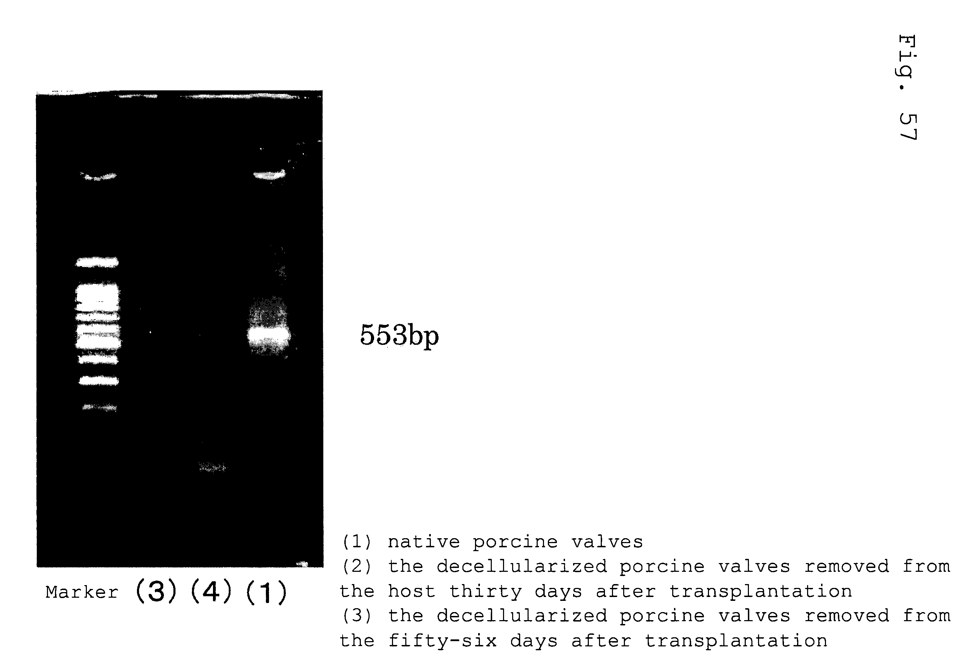

Figure 57 depicts the reults of RT-PCR analysis relating to PERV for thirty days (3) after transplantation and fifty-six days (4) for porcine valves treated with gamma-ray irradiation, and a positive control (1; native porcine valves). As shown therein, no PERV has been detected in the decellularized porcine valves removed from the host thirty and fifty-six days after the transplantation.

(Example 10)

The property of covalent linkage formed by the radical reaction in the above-mentioned Examples is confirmed. As a result, covalent linkages selected from the group consisting of covalent linkage formed between groups other than carboxyl group, hydroxyl group or amino group (for example, the groups comprise groups selected from the group consisting of sulfhydryl group, aldehyde group, carbonyl group, sulfo group and nitro group) and a linkage selected from the group consisting of carbon-carbon linkage, ether linkage and ester linkage, are observed.

As described above, the present invention is illustrated by way of preferred embodiments and examples, the present invention. It should be understood that the scope of the present invention is limited only by the claims. It is understood that those skilled in the art can carry out equivalents of the present invention based on the description of the present invention and the common general knowledge in the art from the specific preferable embodiments of the present invention. All patents, patent applications, and publications cited in this specification are herein incorporated by reference in their entireties to the same extent as if each were specifically herein described.

INDUSTRIAL APPLICABILITY

The present invention established an improved decellularization technology in which tissue damage rate is suppressed to a clinically applicable level, while cell survival ratio is reduced. This technology reinforces decellularized tissue and grafts prepared thereby. Therefore, such tissue is industrially applicable.

**EP 1 698 356 A1**

**Claims**

1. A decellularized tissue which has been subjected to a radical reaction.

2. A decellularized tissue according to Claim 1, **characterized in that** said decellularized tissue is substantially free of solubilized protein.

3. A decellularized tissue according to Claim 1, **characterized in that** a extracellular matrix component is at least partially crosslinked by means of covalent bonding.

4. A decellularized tissue according to Claim 3, wherein the extracellular matrix component is selected from the group consisting of collagen, elastin, laminin, fibronectin, glycosaminoglycan and proteoglycan.

5. A decellularized tissue according to Claim 3, wherein the crosslinking is formed between two or more amino acids having no free amino group or carboxy group at the side chain thereof.

6. A decellularized tissue according to Claim 3, wherein the crosslinking is formed on the groups other than carboxyl, hydroxyl, and amino groups.

7. A decellularized tissue according to Claim 6, wherein the group comprises a group selected from the group consisting of sulfhydryl group, aldehyde group, carbonyl group, sulfo group and nitro group, and the crosslinking is formed by means of a bonding selected from the group consisting of carbon-carbon bonding, ether bondin and ester bonding.

8. A decellularized tissue according to Claim 1, wherein

   A) a cell residual rate of the tissue is less than a level at which an immune reaction is elicited in an organism; and
   B) the tissue is not damaged to such an extent that hinders the tissue from exhibiting a function which was possessed by the tissue when the tissue was not decellularized.

9. A decellularized tissue according to Claim 1, wherein the radical reaction comprises exposure to a free radical.

10. A decellularized tissue according to Claim 1, wherein the tissue strength thereof is substantially the same as that of a decellularized tissue without exposure to a radical reaction, whereas decellularization thereof is significantly progressed.

11. A decellularized tissue according to Claim 1, wherein the radical reaction comprises a treatment selected from the group consisting of gamma-ray irradiation, ultraviolet irradiation, exposure to a free radical source, exposure to ultrasonication, electron beam irradiation, and x-ray irradiation.

12. A decellularized tissue according to Claim 1, wherein the radical reaction is gamma-ray irradiation, and the dose of the gamma-ray irradiation is between 10 and 250 kGy.

13. A decellularized tissue according to Claim 1, wherein the radical reaction is gamma-ray irradiation conducted under an atmosphere selected from the group consisting of in vacuum, in oxygen, in nitrogen, in the atmosphere, in water, in an amphipathic molecule solution and a combination thereof.

14. A decellularized tissue according to Claim 1, wherein the decellularized tissue is subjected to DNase treatment.

15. Decellularized tissue according to Claim 1, wherein the tissue is treated in a solution containing a non-micellar amphipathic molecule,

16. Decellularized tissue according to Claim 15, wherein the solution containing the non-micellaz amphipathic molecule comprises a 1,2-epoxide polymer.

17. Decellularized tissue according to Claim 15, wherein the solution containing the non-micellar amphipathic molecule comprises polyethylene glycol (PEG).

18. Decellularized tissue according to Claim 1'7, wherein an average molecular weight of the PEG is between 200 to 6000.

19. Decellularized tissue according to Claim 17, wherein an average molecular weight of the PEG is about 1000.

20. Decellularized tissue according to Claim 1, wherein the cell residual rate of the tissue is about 5 % or less.

21. Decellularized tissue according to Claim 1, wherein the cell residual rate of the tissue is about 4 % or less.

22. Decellularized tissue according to Claim 1, wherein the cell residual rate of the tissue is about 1 % or less.

23. Decellularized tissue according to Claim 1, wherein the tissue has substantially no cells remaining.

24. Decellularized tissue according to claim 1, wherein the tissue damage rate of the tissue is about 30% or less.

25. Decellularized tissue according to claim 1, wherein the tissue damage rate of the tissue is about 15% or less.

26. Decellularized tissue according to claim 1, wherein the tissue damage rate of the tissue is about 5% or less.

27. Decellularized tissue according to claim 1, wherein the tissue has a tissue strength which permits a clinical application.

28. Decellularized tissue according to claim 1, wherein the tissue has a tissue strength which is 80% or more of the tissue strength which was possessed by the tissue when the tissue was not decellularized.

29. Decellularized tissue according to claim 1, wherein the tissue has a tissue strength having a β value which is 80% or more of the β value which was possessed by the tissue when the tissue was not decellularized.

30. Decellularized tissue according to claim 1, wherein the tissue has a tissue strength having a β value of 20 or more.

31. Decellularized tissue according to claim 1, wherein the tissue is luminal tissue.

32. Decellularized tissue according to claim 1, wherein the tissue is tissue selected from blood vessels, blood vessel-like tissue, cardiac valves, pericardia, dura mater, corneas, and bones.

33. Decellularized tissue according to claim 1, wherein a survival rate of naive extracellular matrix in the decellularized tissue is at least about 50%.

34. Decellularized tissue according to claim 33, wherein the survival rate of the extracellular matrix is substantially a hundred percent.

35. Decellularized tissue according to claim 1, wherein the tissue is derived from a mammal.

36. Decellularized tissue according to claim 1, wherein the tissue is derived from a human.

37. Decellularized tissue according to claim 1, wherein the tissue is derived from a swine.

38. Decellularized tissue according to claim 1, wherein viruses have been substantially removed from the tissue.

39. Decellularized tissue according to claim 38, wherein the viruses are selected from the group consisting of retroviruses and herpes viruses.

40. Decellularized tissue according to claim 38, wherein the viruses comprises porcine endogenous retrovirus (PERV).

41. Decellularized tissue according to claim 40, wherein the PERV is removed such that no significant infection to a human cell is observed.

42. A tissue graft comprising decellularized tissue according to Claim 1.

43. A tissue graft according to Claim 42 further comprising a cell.

44. A tissue graft according to Claim 42, wherein the tissue graft has a form selected from the group consisting of membrane-form, luminal form and valvular form.

45. A method of producing decellularized tissue, comprising the steps of:

   1) providing tissue;
   2) immersing the tissue in a solution containing a non-micellar amphipathic molecule; and
   3) subjecting the tissue to a radical reaction.

46. A method according to Claim 45, wherein the radical reaction comprises exposure to a free radical.

47. A method according to Claim 45, wherein the tissue strength thereof is substantially the same as that of a decellularized tissue without exposure to a radical reaction, whereas decellularization thereof is significantly progressed.

48. A method according to Claim 45, wherein the radical reaction comprises a treatment selected from the group consisting of gamma-ray irradiation, ultraviolet irradiation, exposure to a free radical source, exposure to ultrasonication, electron beam irradiation, and x-ray irradiation.

49. A method according to Claim 45, wherein the radical reaction is gamma-ray irradiation, and the dose of the gamma-ray irradiation is between 10 and 250 kGy.

50. A method according to Claim 45, wherein the radical reaction is gamma-ray irradiation, and the dose of the gamma-ray irradiation is between 40 and 100 kGy.

51. A method according to Claim 45, wherein the radical reaction is gamma-ray irradiation conducted under an atmosphere selected from the group consisting of in vacuo, in oxygen, in nitrogen, in the atmosphere, in water, in an amphipathic molecule solution and a combination thereof.

52. A method according to Claim 45, wherein the radical reaction is conducted in the atmosphere.

53. A method according to Claim 45, wherein the tissue is treated in a solution containing a non-micellar amphipathic molecule.

54. A method according to Claim 53, wherein the solution containing the non-micellar amphipathic molecule comprises a 1,2-epoxide polymer.

55. A method according to Claim 53, wherein the solution containing the non-micellar amphipathic molecule comprises polyethylene glycol (PEG).

56. A method according to Claim 55, wherein the average molecular weight of the PEG is between about 200 to about 2000.

57. A method according to Claim 55, wherein the average molecular weight of the PEG is between about 1000 to about 2000.

58. A method according to Claim 55, wherein the PEG solution is a solution of about 60% to about 100%.

59. A method according to Claim 45, wherein the tissue is a luminal tissue, a valvular tissue or amembrenous tissue.

60. A method according to Claim 45, wherein the tissue is tissue selected from blood vessels, blood vessel-like tissue, cardiac valves, pericardia, dura mater, corneas, and bones.

61. A method according to Claim 45, wherein the tissue is derived from a mammal.

62. A method according to Claim 45, wherein the tissue is derived from a human or a swine.

63. A method according to Claim 45, wherein the step of immersing is conducted for 30 minutes to ten days.

64. A method according to Claim 45, wherein the step of immersing further comprises a step of physical treatment.

65. A method according to Claim 45, further comprising the step of D) washing the tissue.

66. A method according to Claim 65, wherein the step of washing is conducted for half a day to five days.

67. A method according to Claim 45, wherein the amphipathic molecule is biocompatible.

68. A method according to Claim 45, wherein the step of immersing comprises a step of agitation.

69. A method according to Claim 45, wherein the step of immersing comprises agitation for one week or longer.

70. A method according to Claim 45, wherein the decellularized tissue is subjected to a DNase treatment.

71. A method according to claim 45, further comprising:

    4) performing chemical treatment.

72. A method according to claim 68, wherein the chemical treatment is performed with DNase.

73. A method according to claim 68, wherein the chemical treatment is performed with DNaseI. A

**74.** A method according to Claim 72, wherein the treatment by the DNase is conducted for 24 hours or longer.

**75.** A method according to Claim 45, wherein the radical reaction is gamma-ray irradiation and the period of irirradiation of the gamma-ray irradiation is a period so as to total a dose of about 10-300kGy.

**76.** A method according to claim 45, further comprising:

disseminating a cell.

**77.** Decellularized tissue obtainable by a method according to Claim 45.

**78.** Amethod for tissue regeneration, comprising the steps of:

a) providing decellularized tissue subjected to a radical reaction, to an organism; and
b) incubating the tissue within the organism for a time sufficient for the tissue to regenerate.

**79.** A method according to Claim 78, wherein the radical reaction comprises exposure to a free radical.

**80.** A method according to Claim 78 further comprising the step of providing a cell to the decellularized tissue.

**81.** A method according to Claim 80, wherein the cell is derived from the organism.

**82.** A method according to Claim 80, wherein the cell is present within the organism.

**83.** A method according to Claim 80, wherein the cell is derived from a host homologous to the organism.

**84.** A method according to Claim 80, wherein the cell is derived from a host heterologous to the organism.

**85.** A method according to Claim 80, wherein the cell is previously isolated from the organism.

**86.** A method according to Claim 80, the cell is a blood vessel cell or a blood vessel-like cell.

**87.** A method according to Claim 78, further comprising providing a physiologically active substance capable of inducing cell differentiation to the organism.

**88.** A method according to Claim 87, wherein the physiologically active substance is derived from or foreign to the organism.

**89.** A method according to Claim 8'7, wherein the physiologically active substance is provided in a form of a nucleic acid or a polypeptide.

**90.** A method according to Claim 87, wherein the physiologically active substance is selected from the group consisting of HGF, VEGF, FGF, IGF, PDGF, and EGF.

**91.** A method according to Claim 78, wherein the tissue is tissue selected from the group consisting of bloodvessels, blood vessel-like tissue, cardiac valves, pericardia, dura mater, corneas, and bones.

**92.** A method of producing a tissue graft, comprising the steps of:

A) providing decellularized tissue subjected to a radical reaction, to an organism;
B) allowing a self cell in the organism to infiltrate the decellularized tissue; and
C) incubating the tissue within the organism for a time sufficient for the cell to differentiate.

**93.** A method according to Claim 92, wherein the radical reaction comprises exposure to a free radical.

**94.** A method according to Claim 92, the cell is a blood vessel cell or a blood vessel-like cell.

**95.** A method according to Claim 92, wherein the tissue is tissue selected from the group consisting of bloodvessels, blood vessel-like tissue, cardiac valves, pericardia, dura mater, corneas, and bones.

**96.** A method according to Claim 92, wherein the decellularized tissue further comprises a cell.

**97.** A method according to Claim 92, further comprising the step of D) providing a physiologically active substance capable of inducing differentiation of the cell.

**98.** A method according to Claim 97, wherein the physiologically active substance is a cytokine having hematopoiesis activity.

**99.** A tissue graft, produced by a method according to claim 92.

**100.** A method for treating a subject requiring implantation of tissue or an organ or treating a subject at a risk of implantation of tissue or an organ for prophylaxis , the method comprising the steps of:

A) providing decellularized tissue subjected to a radical reaction, or a tissue graft comprising the decellularized tissue; and
B) implanting the decellularized tissue or tissue graft to the subject.

**101.** A method according to Claim 100, wherein the radical reaction comprises exposure to a free radical.

**102.** A method according to Claim 100, the tissue further comprises a cell.

**103.** A method according to Claim 100, wherein the tissue has no cells.

**104.** A method according to Claim 100, wherein the tissue is derived from the subject.

**105.** A method according to Claim 100, wherein the tissue is tissue selected from blood vessels, blood vessel-like tissue, cardiac valves, pericardia, dura mater, corneas, and bones.

**106.** A method according to Claim 100, wherein the tissue is derived from a mammal.

**107.** A method according to Claim 100, wherein the tissue is derived from a human.

**108.** A pharmaceutical for organ transplantation comprising:

A) decellularized tissue subjected to a radical reaction, or a tissue graft comprising the decellularized tissue.

**109.** A pharmaceutical according to Claim 108, wherein the radical reaction comprises exposure to a free radical.

**110.** A pharmaceutical according to Claim 108, wherein the tissue is tissue selected from blood vessels, blood vessel-like tissue, cardiac valves, pericardia, dura mater, corneas, and bones.

**111.** A pharmaceutical according to Claim 108, wherein the tissue is derived from a mammal.

**112.** A pharmaceutical according to Claim 108, wherein the tissue is derived from a human or a swine.

**113.** A pharmaceutical according to Claim 108, wherein the tissue is derived from a subject in need of the transplantation.

**114.** Use of decellularized tissue subjected to a radical reaction, or a tissue graft comprising the decellularized tissue for manufacture of a pharmaceutical for organ transplantation.

**115.** Use according to Claim 114, wherein the radical reaction comprises exposure to a free radical.

Fig. 1

Fig. 2

PEG1000 — Without agitation ×200; With agitation ×200

Fig. 3

×40

Fig. 4

Fig. 5

Fig. 6

Fig. 7

Fig. 8

Fig. 9

Fig. 10

Fig. 11

PEG600 control

X40

X200

X100

Fig. 12

Fig. 13

Fig. 14

Fig. 15

100

Fig. 16

Fig. 17

Fig. 18

Fig. 19

Fig. 20

Fig. 21

Fig. 22

Fig. 23

Fig. 24

Fig. 25

Fig. 26

**50KGy**

Fig. 27

**100KGy**

Fig. 28

γ-ray 130KGy + DNAase

HE staining x200

Fig. 29

PEG-γ valve

x40

X200

cusp

Fig. 30A

Treatment : PEG + γ-ray 100kGy
Object : valve
Magnification : X40

Treatment : PEG + γ-ray 100kGy
Object : valve
Magnification : X100

Treatment : PEG + γ-ray 100kGy
Object : valve
Magnification : X40

Treatment : PEG + γ-ray 100kGy
Object : valve
Magnification : X100

Valve    PEG + γ :decellularization effects

Fig. 30B

valve cusp    PEG + γ :decellularization effects

Treatment : PEG + γ-ray 100kGy
Object : valve cusp
Magnification : X40

Treatment : PEG + γ-ray 100kGy
Object : valve cusp
Magnification : X100

## Fig. 31

**PEG**　　**Results of tensile strength tests**

| Testing machine | RTC series | | | Type of testing | Extension | | |
|---|---|---|---|---|---|---|---|
| Load in full-scale | **5 kgf** | | | Rated capacity of load cell | **100 N** | | |
| Range of load | **40 %RO** | | | Rated capacity of the extensometer | **20 cm** | | |
| Range of the extensometer | unapplied | | | Test Speed | **10.0 mm/min** | | |
| Recording speed off | | | | Rigidity of the testing machine | **0 mm/kgf** | | |
| Midpoint (load) **N** | **0** | **0** | **0** | Midpoint (extension) **cm** | **0** | **50** | **60** |
| | **0** | **0** | **0** | | **0** | **0** | **0** |
| Analysis of Elastic moduli | Interval | **1** | **50** | Initial length  Distance between chunks | **10 mm** | | |
| | Pitch | **1** | **%max** | origin in extension  initial load point | **0.03 N** | | |
| slack correction | applied | | | Determination of rupture point | **0.5 N** | | |
| Storing SS curve | ON | | | | | | |

| **TestID=120** | Maximum load | Maximum load | Rupture load | Rupture load | Maximum Extension | Elastic Modulus |
|---|---|---|---|---|---|---|
| Test No. | **kgf** | **N** | **kgf** | **N** | **mm** | **MPa** |
| 1 | 1.3522 | 13.260 | 1.0889 | 10.678 | 16.227 | 2.3930 |
| 2 | 1.3049 | 12.797 | 1.0265 | 10.066 | 20.987 | 1.8916 |
| 3 | 0.9980 | 9.7870 | 0.9281 | 9.1012 | 14.327 | 1.8013 |
| 4 | 1.0020 | 9.8261 | 0.7277 | 7.1365 | 16.367 | 1.5761 |
| 5 | 0.7636 | 7.4879 | 0.7340 | 7.1981 | 4.2267 | 4.7653 |
| Average | 1.0841 | 10.832 | 0.9010 | 8.8360 | 14.427 | 2.4855 |
| JIS weighted avg | 1.2675 | 12.430 | 1.0186 | 9.9888 | 18.459 | 3.4698 |
| Median | 1.0020 | 9.8261 | 0.9281 | 9.1012 | 16.227 | 1.8916 |
| Maximum | 1.3522 | 13.260 | 1.0889 | 10.678 | 20.987 | 4.7653 |
| SD(n-1) | 0.2437 | 2.3897 | 0.1656 | 1.6239 | 6.2066 | 1.3090 |
| SD(n) | 0.2180 | 2.1374 | 0.1481 | 1.4525 | 5.5513 | 1.1708 |

LOAD vs Extension (mm)

90

# Fig. 32

**Results of tensile test of native canine aorta.**

| Testing machine | RTC series | | | Type of testing | Extension | | |
|---|---|---|---|---|---|---|---|
| Load in full-scale | 5 kgf | | | Rated capacity of load cell | 100 N | | |
| Range of load | 40 %RO | | | Rated capacity of the extensometer | 20 6m | | |
| Range of the extensometer | unapplied | | | Test Speed | 10.0 mm/min | | |
| Recording speed off | | | | Rigidity of the testing machine | 0 mm/kgf | | |
| Midpoint (load) N | 0 | 0 | 0 | Midpoint (extension) cm | 0 | 50 | 60 |
| | 0 | 0 | 0 | | 0 | 0 | 0 |
| Analysis of Elastic moduli | Interval | 1 | 50 | Initial length Distance between chunks | 10 mm | | |
| | Pitch | 1 %max | | origin in extension initial load point | 0.03 N | | |
| slack correction | applied | | | Determination of rupture point | 0.5 N | | |
| Storing SS curve | ON | | | | | | |

| TestID=37 Test No. | Maximum load kgf | Maximum load N | Rupture load kgf | Rupture load N | Maximum Extension mm | Elastic Modulus MPa |
|---|---|---|---|---|---|---|
| 1 | 0.7591 | 7.4445 | 0.5038 | 4.9404 | 27.887 | 1.0918 |
| Average | 0.7591 | 7.4445 | 0.5038 | 4.9404 | 27.887 | 1.0918 |
| JIS weighted avg | 0.7591 | 7.4445 | 0.5038 | 4.9404 | 27.887 | 1.0918 |
| Median | 0.7591 | 7.4445 | 0.5038 | 4.9404 | 27.887 | 1.0918 |
| Maximum | 0.7591 | 7.4445 | 0.5038 | 4.9404 | 27.887 | 1.0918 |

LOAD vs Extension (mm)

# Fig. 33

**results of tensile test of conventional artificial valves by means of decellularization cell method by SDS**

| Testing machine | RTC series | | | Type of testing | Extension | | |
|---|---|---|---|---|---|---|---|
| Load in full-scale | 5 kgf | | | Rated capacity of load cell | 100 N | | |
| Range of load | 40 %RO | | | Rated capacity of the extensometer | 20 cm | | |
| Range of the extensometer | unapplied | | | Test Speed | 10.0 mm/min | | |
| Recording speed off | | | | Rigidity of the testing machine | 0 mm/kgf | | |
| Midpoint (load) N | 0 | 0 | 0 | Midpoint (extension) cm | 0 | 50 | 60 |
| | 0 | 0 | 0 | | 0 | 0 | 0 |
| Analysis of Elastic moduli | Interval | 1 | 50 | Initial length   Distance between chunks | 10 mm | | |
| | Pitch | 1 %max | | origin in extension  initial load point | 0.03 N | | |
| slack correction | applied | | | Determination of rupture point | 0.5 N | | |
| Storing SS curve | ON | | | | | | |

| TestID=17 Test No. | Maximum load kgf | Maximum load N | Rupture load kgf | Rupture load N | Elastic Modulus MPa |
|---|---|---|---|---|---|
| 1 | 1.0401 | 10.200 | 1.0284 | 10.085 | 2.5168 |
| 2 | 0.7095 | 6.9574 | 0.6856 | 6.7231 | 1.4561 |
| 3 | 0.7142 | 7.0038 | 0.6339 | 6.2164 | 1.4976 |
| 4 | 0.8572 | 8.4063 | 0.8503 | 8.3387 | 1.6830 |
| 5 | 0.6693 | 6.5639 | 0.6613 | 6.4847 | 1.1928 |
| Average | 0.7981 | 7.8263 | 0.7719 | 7.5696 | 1.6653 |
| JIS weighted avg | 0.9196 | 9.0180 | 0.9040 | 8.8649 | 2.0527 |
| Median | 0.7142 | 7.0038 | 0.6856 | 6.7231 | 1.4976 |
| Maximum | 1.0401 | 10.200 | 1.0284 | 10.085 | 2.5168 |
| SD(n-1) | 0.1529 | 1.4996 | 0.1663 | 1.6313 | 0.5050 |
| SD(n) | 0.1368 | 1.3413 | 0.1488 | 1.4591 | 0.4517 |

LOAD

Extention   mm

Fig. 34

Fig. 35

×200

×100

Fig. 36

×100

×40

Fig. 37

Fig. 38

×100

×100

×40

Fig. 39

Fig. 40

×100

×40

Fig. 41

×100

Fig. 42

×100

×40

Fig. 43

Fig. 44

*HE staining, X200, gamma-irradiation: 15kGy

| native | PEG DNase | PEG γ-ray DNase | PEG γ-ray DNase |
|---|---|---|---|
| remaining cells observed | No remaining cells | No remaining cells | No remaining cells |

Fig. 45A

*DNA assay/sample (µg/mg), gamma-irradiation: 15kGy

Fig. 45B

EP 1 698 356 A1

＊DNA assay/sample (μg/mg), gamma-irradiation: 15kGy

| native | γ-ray ▶ DNase ▶ | γ-ray ▶ PEG ▶ DNase ▶ | PEG ▶ γ-ray ▶ DNase ▶ |
|---|---|---|---|
| remaining DNA observed | remaining DNA observed | substantially free of remaining DNA | substantially free of remaining DNA |

Fig. 46

EP 1 698 356 A1

*protein assay/sample (mg/mg), gamma-irradiation: 15kGy

| native | PEG DNase | γ-ray PEG DNase | PEG γ-ray DNase |
|---|---|---|---|
| remaining proteins observed | remaining proteins observed | No remaining proteins | No remaining proteins |

Fig. 47

Fig. 48

EP 1 698 356 A1

*Tensile strength test/maximum load (N), gamma-irradiation: 151Gy

| native | Glutaraldehyde (horse heart valve) | PEG DNase | γ-ray PEG DNase | PEG γ-ray DNase |
|---|---|---|---|---|
| +++ | N.D. | N.D. | N.D. | N.D. |

Fig. 49

*HE staining, one week after transplantation, gamma-irradiation:15kGy

Fig. 50

*HE staining, two months after transplantation, gamma-irradiation:15kGy

Fig. 51

*von Kossa staining , two months after transplantation, gamma-irradiation:15kGy

| native | Glutaraldehyde (horse heart valve) | PEG DN100 | PEG X-ray / PEG DN100 | PEG X-ray / PEG DN100 |
|---|---|---|---|---|
| No calcification reaction | Calcification reaction observed | No calcification reaction | No calcification reaction | No calcification reaction |

Fig. 52

*Investigation of the calcification/sample (mg/mg), two months after transplantation, gamma-irradiation:15kGy

| native | Glutaraldehyde (horse heart valve) | PEG DNase | γ-ray PEG DNase | PEG γ-ray DNase |
|---|---|---|---|---|
| 2.5 2.0 1.5 1.0 0.5 | 2.5 2.0 1.5 1.0 0.5 | 2.5 2.0 1.5 1.0 0.5 | 2.5 2.0 1.5 1.0 0.5 | 2.5 2.0 1.5 1.0 0.5 |
| No calcification reaction | Calcification reaction observed | No calcification reaction | No calcification reaction | No calcification reaction |

Fig. 53

EP 1 698 356 A1

| | Native | PEG+DNase | ×100 |
|---|---|---|---|
| No irradiation | | | **N.C.** rhesus monkey |
| Gamma-irradiation (100kGy) | | | |

Fig. 54

EP 1 698 356 A1

*Detection of GS1B4(a-1,3-Gal) epitope using lectins

| | Native | PEG+DNase | ×100 |
|---|---|---|---|
| No irradiation | | | N.C. rhesus monkey |
| Gamma-irradiation (25kGy) | | | |

γ-ray PEG DNase   PEG γ-ray DNase

Fig. 55

553bp

(1) native porcine valve (n=5)

Fig. 56

Fig. 57

553bp

Marker (3) (4) (1)

(1) native porcine valves
(2) the decellularized porcine valves removed from the host thirty days after transplantation
(3) the decellularized porcine valves removed from the fifty-six days after transplantation

## INTERNATIONAL SEARCH REPORT

| | International application No. |
|---|---|
| | PCT/JP2004/019441 |

**A. CLASSIFICATION OF SUBJECT MATTER**
$Int.Cl^7$  A61L27/00, A61K35/12

According to International Patent Classification (IPC) or to both national classification and IPC

**B. FIELDS SEARCHED**

Minimum documentation searched (classification system followed by classification symbols)
$Int.Cl^7$  A61L27/00, A61K35/12

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2003/002165 A (COOK BIOTEC INC.), 09 January, 2003 (09.01.03), Full text & US 2003/0014126 A & EP 1404388 A & AU 2002320189 A & JP 2004-532714 A | 1-13,15-31, 33-59,61-69, 75-77,92-94, 96-99, 108-109, 111-115 |
| Y | | 14,32,60, 70-74,95,110 |
| Y | WO 2001/054619 A (CRYOLIFE, INC.), 02 August, 2001 (02.08.01), Full text & AU 200133072 A & EP 1250109 A & JP 2004-500188 A & US 6866686 B | 14,70-74 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 29 March, 2005 (29.03.05) | 19 April, 2005 (19.04.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

## INTERNATIONAL SEARCH REPORT

International application No.

PCT/JP2004/019441

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 6-261933 A (LIFECELL CORP.),<br>20 September, 1994 (20.09.94),<br>Full text<br>& EP 564786 A    & CA 2089336 A<br>& US 5336616 A    & AU 668703 B | 32,60,95,110 |
| A | WO 2003/020107 A (REGENERATION TECHNOLOGIES, INC.),<br>13 March, 2003 (13.03.03),<br>Full text<br>& EP 1427322 A    & JP 2005-503850 A<br>& AU 2002323425 A | 1-77,92-99,<br>108-115 |

Form PCT/ISA/210 (continuation of second sheet) (January 2004)

## INTERNATIONAL SEARCH REPORT

International application No.

PCT/JP2004/019441

| Box No. II | Observations where certain claims were found unsearchable (Continuation of item 2 of first sheet) |

This international search report has not been established in respect of certain claims under Article 17(2)(a) for the following reasons:

1. ☒ Claims Nos.: 78-91, 100-107
    because they relate to subject matter not required to be searched by this Authority, namely:
The inventions as set forth in claims 78 to 91 and 100 to 107 pertain to methods for treatment of the human body by therapy and thus relate to a subject matter which this International Searching Authority is not required, under the provisions of        (continued to extra sheet)

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

| Box No. III | Observations where unity of invention is lacking (Continuation of item 3 of first sheet) |

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**   ☐   The additional search fees were accompanied by the applicant's protest.

☐   No protest accompanied the payment of additional search fees.

Form PCT/ISA/210 (continuation of first sheet (2)) (January 2004)

120

**INTERNATIONAL SEARCH REPORT**

International application No.

PCT/JP2004/019441

Continuation of Box No.II-1 of continuation of first sheet(2)

Article 17(2)(a)(i) of the PCT and Rule 39.1(iv) of the Regulations under the PCT, to search.

Form PCT/ISA/210 (extra sheet) (January 2004)

SEQUENCE LISTING

<110> CARDIO INCORPORATED
NATIONAL INSTITUTE OF ADVANCED INDUSTRIAL SCIENCE AND TECHNOLOGY

<120> Decellularized tissue and methods for making the same

<130> CD012EP

<160> 6

<170> PatentIn version 3.2

<210> 1
<211> 22
<212> DNA
<213> artificial

<220>
<223> primer

<400> 1
actacaacac ggctgaaggt ag          22

<210> 2
<211> 21
<212> DNA
<213> artificial

<220>
<223> primer

<400> 2
ttgtcgagtg gggtcctatt g          21

<210> 3
<211> 21
<212> DNA
<213> artificial

<220>
<223> primer

<400> 3
ctgcaccacc aaatgcttag c          21

<210> 4
<211> 19
<212> DNA
<213> artificial

<220>
<223> primer

```
<400>   4
gccatgccag tgagcttcc                                    19


<210>   5
<211>   24
<212>   DNA
<213>   artificial

<220>
<223>   primer

<400>   5
gtgatgctgg tgctgagtat gttg                              24


<210>   6
<211>   22
<212>   DNA
<213>   artificial

<220>
<223>   primer

<400>   6
tggctagagg agccaagcag tt                                22
```